(19) Europäisches Patentamt
European Patent Office
Office européen des brevets

(11) EP 0 677 524 B1

(12) **EUROPEAN PATENT SPECIFICATION**

(45) Date of publication and mention of the grant of the patent:
**13.02.2002 Bulletin 2002/07**

(21) Application number: **95111206.9**

(22) Date of filing: **26.10.1989**

(51) Int Cl.[7]: **C07D 495/22**, C07D 495/14, A61K 31/55 // (C07D495/22, 333:00, 249:00, 243:00, 221:00), (C07D495/22, 333:00, 249:00, 243:00, 209:00), (C07D495/14, 333:00, 249:00, 243:00)

# (54) Thieno[3,2-f](1,2,4-triazolo)[4,3-a]1,4-diazepine derivatives as PAF antagonists

Thieno[3,2-f] (1,2,4- triazolo)[4,3-a] 1,4- diazepinderivate als PAF-Antagonisten

Dérivés de thieno [3,2-f](1,2,4-triazolo) [4,3-a] 1,4-diazepine comme antagonistes du PAF

(84) Designated Contracting States:
**AT BE CH DE FR GB IT LI LU NL SE**

(30) Priority: **31.10.1988 JP 27546088**
**24.11.1988 JP 29706888**
**16.12.1988 JP 31801688**
**28.12.1988 JP 33162288**

(43) Date of publication of application:
**18.10.1995 Bulletin 1995/42**

(62) Document number(s) of the earlier application(s) in accordance with Art. 76 EPC:
**89119910.1 / 0 367 110**

(73) Proprietor: **Eisai Co., Ltd.**
**Tokyo (JP)**

(72) Inventors:
- **Okano, Kazuo**
  **Tsukuba-gun, Ibaraki (JP)**
- **Miyazawa, Shuhei**
  **Kitasoma-gun Ibaraki 302-01 (JP)**
- **Clark, Richard Stephen J.**
  **Tsukuba-shi, Ibaraki (JP)**
- **Abe, Shinya**
  **Ushiku-shi, Ibaraki (JP)**
- **Kawahara, Tetsuya**
  **Tsukuba-shi, Ibaraki (JP)**
- **Shimomura, Naoyuki**
  **Tsukuba-shi, Ibaraki 305 (JP)**
- **Asano, Osamu**
  **Tsukuba-shi, Ibaraki (JP)**
- **Yoshimura, Hiroyuki**
  **Tsukuba-shi, Ibaraki (JP)**
- **Miyamoto, Mitsuaki**
  **Tsuchiura-shi, Ibaraki (US)**
- **Sakuma, Yoshinori**
  **Tsuchiura-shi, Ibaraki (JP)**
- **Muramoto, Kenzo**
  **Tsukuba-shi, Ibaraki (JP)**
- **Obaishi, Hiroshi**
  **Tsukuba-shi, Ibaraki (JP)**
- **Harada, Koukichi**
  **Tsukuba-shi, Ibaraki (JP)**
- **Tsunoda, Hajime**
  **Tsukuba-shi, Ibaraki (JP)**
- **Katayama, Satoshi**
  **Tsukuba-shi, Ibaraki (JP)**
- **Yamada, Kouji**
  **Ushiku-shi, Ibaraki (JP)**
- **Souda, Shigeru**
  **Ushiku-shi, Ibaraki 300-12 (JP)**
- **Machida, Yoshimasa**
  **Tsukuba-shi, Ibaraki (JP)**
- **Katayama, Kouichi**
  **Tsukuba-shi, Ibaraki (JP)**
- **Yamatsu, Isao**
  **Ushiku-shi, Ibaraki (JP)**

(74) Representative:
**Hansen, Bernd, Dr. Dipl.-Chem. et al**
**Hoffmann Eitle, Patent- und Rechtsanwälte,**
**Arabellastrasse 4**
**81925 München (DE)**

(56) References cited:
**EP-A- 0 194 416**
**EP-A- 0 230 942**
**EP-A- 0 268 242**
**EP-A- 0 368 175**
**DE-A- 3 724 031**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Remarks:

The file contains technical information submitted after the application was filed and not included in this specification

## Description

The invention provides new 1,4-diazepine derivatives and a pharmacologically acceptable salts thereof, a process for preparing them and the pharmaceutical use. The compounds and their salts have an excellent medical activity.

(Prior Arts)

In recent years, a platelet activating factor (hereinafter referred to simply as PAF) has attracted much attention and its relationship with various diseases is now being clarified. Now, it has been assumed that PAF takes part in not only inflammation, but also DIC, endotoxin shock, asthma, ulcers in alimentary canal, hepatisis and the rejection at the time of organ transplantation. In addition, attention has been drawn to as a mediator which is one of allergic reactions.

Under these circumstances, there have been made investigations on compounds having the anti-PAF activity. Among these compounds, a 1,4-diazepine compound having the anti-PAF action has been proposed, for example, in Japanese Laid-open Patent Application No. 63-33382. However, a satisfactory anti-PAF agent which is adapted, particularly, for allergies such as asthma has not been developed yet.
DE-A-37 24 031 reveals tetracine compounds showing the following formulae Ia and Ib

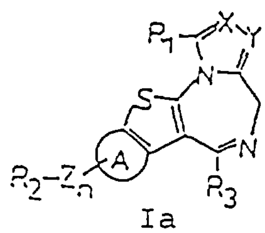

Ia

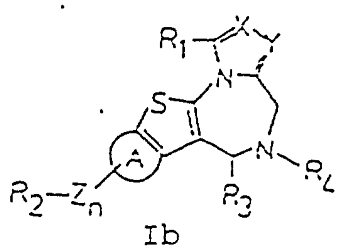

Ib

These compounds are said to have PAF antagonist activity. Specific embodiments thereof involve 3-thioacetyl-6-(2-chlorophenyl)-11-methyl-2,3,4,5,-tetrahydro-8H-pyrido-[4',3':4,5]thieno[3,2-f][1,2,4]-triazolo[4,3-a][1,4]diazepine (example 25), 6-(2 chlorophenyl)-11-methyl-2,3,4,5-tetrahydro-8-H-pyrido-[4',3':4,5]thieno[3,2-f][1,2,4]triazolo[4,3-a][1,4]-diazepine, (example 26), and 3-(carboethoxymethyl)-6-(2-chlorophenyl)-11-methyl-2,3,4,5-tetrahydro-8-H-pyrido [4',3':4,5]thieno[3,2-f] [1,2,4]-triazolo[4,3-a][1,4]-diazepine (example 27). Example 25 has an anti-PAF activity of $10^{-6}$ mol ($IC_{50}$).

EP-O 194 416 A1 discloses thieno-triazolo-1-4-diazepino-2-amides with the following formula I

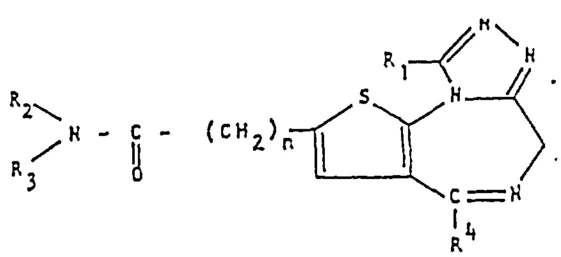

(I)

EP-O 230 942 discloses thieno-1,4-diazepine compounds of the general formula

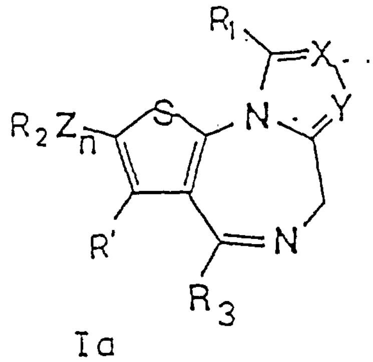

Ia

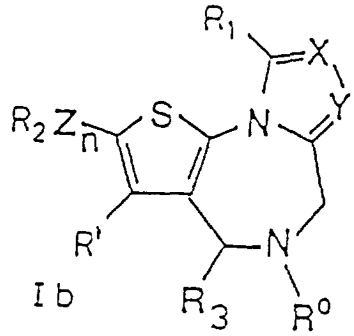

I b

The above compounds are also used for the treatment of diseases connected with PAF. EP-O 268 242 A1 discloses a thienotriazolodiazepine compound of the general formula:

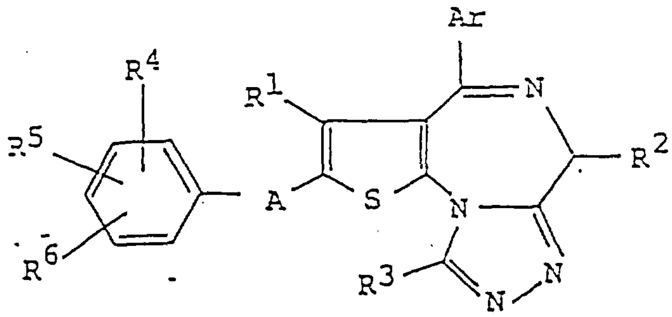

(I)

Said compounds exhibit PAF-antagonistic activity and are useful for the prevention or treatment of various PAF-induced diseases.

(Summary of the Invention)

The present invention. provides novel 1,4-diazepine derivatives defined below or pharmacologically acceptable salts thereof having excellent and long lasting PAF-inhibiting activity,

Triazolo-1,4-di-azepine compounds have the following formula (I)

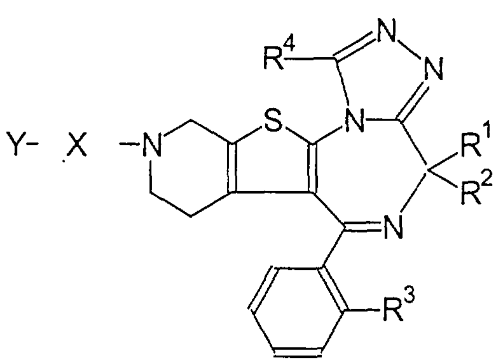

(I)

wherein $R^1$ and $R^2$ independently represent a hydrogen atom or a $C_1$-$C_6$ alkyl group, under the proviso that compounds wherein $R^1$ is hydrogen and $R^2$ is methyl are excluded, $R^3$ represents a hydrogen atom or a halogen atom, $R^4$ represents a hydrogen atom or a $C_1$-$C_6$ alkyl group, X represents

(a) a group of the formula,

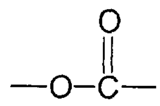

(b) a group of the formula,

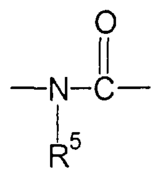

wherein $R^5$ represents a hydrogen atom or a $C_{1-6}$ alkyl group,

(c) a group of the formula,

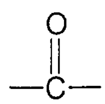

or

(d) a group of the formula,

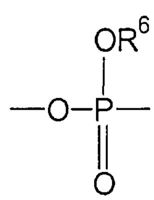

(wherein $R^6$ represents a $C_1$-$C_6$ alkyl group) and Y represents

(1) a $C_3$-$C_7$ cycloalkyl group which may have a methyl group as a substituent(s),

(2) a $C_3$-$C_7$ cycloalkyl-$C_1$-$C_6$ alkyl,

(3) an alkynyl group having up to 6 carbon atoms,

(4) a group of the formula

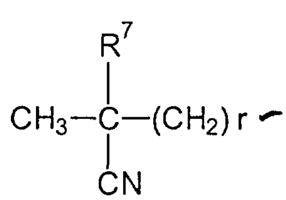

in which $R^7$ is hydrogen or methyl and r is zero, 1 or 2,

(5) a group of the formula $NC-(CH_2)_p-$ (wherein p is an integer of from 1 to 6),

(6) a group of the formula $A-(CH_2)_q-$ (wherein A represents a group selected from a pyridyl group, a pyranyl group and a morpholino group and q is an integer of from 0 to 6),

(7) an alkynyl group having up to 6 carbon atoms
wherein a phenyl group or a $C_3$-$C_7$ cycloalkyl group is joined to any carbon atom,

(8) a group of the formula

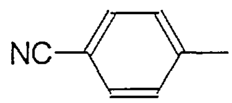

(9) a group of the formula

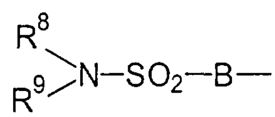

wherein $R^8$ and $R^9$ are the same or different and represent a hydrogen atom, a $C_1$-$C_6$ alkyl group, a pyridylmethyl group or a $C_3$-$C_7$ cycloalkyl group or $R^8$ and $R^9$ may be joined along with a nitrogen atom to form a ring, and B represents a phenylene group or a $C_1$-$C_3$ alkylene group,

(10) a group of the formula

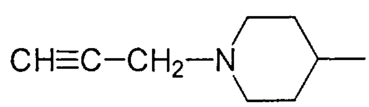

(11) a group of the formula

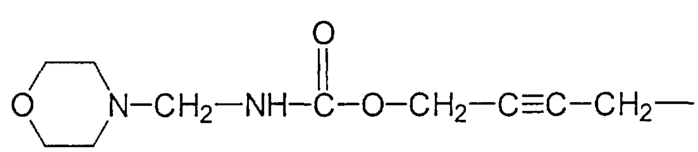

(12) a group of the formula

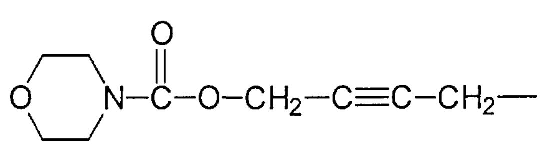

(13) a group of the formula

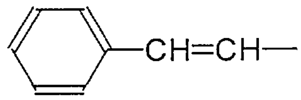

(14) a group of the formula

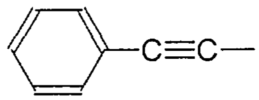

(15) a $C_1$-$C_6$ alkyl group, or

(16) a $C_3$-$C_7$ cycloalkyl-alkenyl group, whereby the alkenyl group has upto 6 C-atoms with the following proviso
when X is

(a)

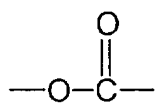
,

(b)

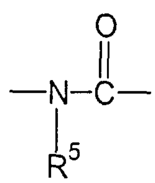
or

(c)

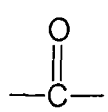
,

Y is selected from (1) to (14) or (16), and
when X is

(d)

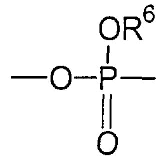

Y is selected from (15).

The invention further involves the related 1,4-diazepine derivatives of claims 2 to 4.

In the formula of the invention it is preferable that X is (a), (b) or (c). It is more preferable that X is (c) -CO-.

It is preferable that Y is one of (1) to (16), more preferably (4), (3), (16) and (2). Most preferable examples for Y include a $C_{3-7}$ cycloalkyl, such as cyclopropyl, and $HC{=}C{-}C(CH_3)_2$-.

Preferable compounds have the formula in which R3 is chlorine, R1 and $R^2$ are hydrogen, R4 is methyl, and Y-X-is defined as follows:

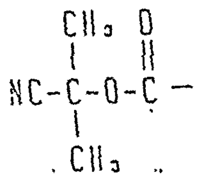

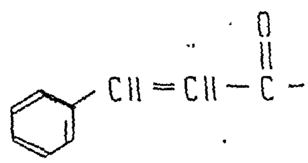

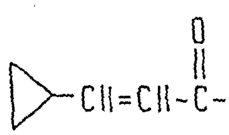

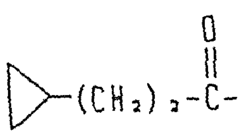

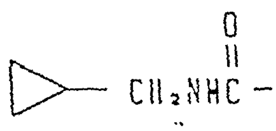

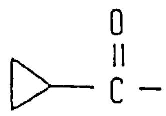

H

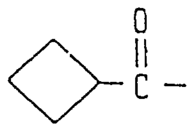

H

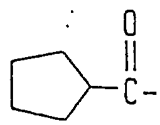

H

Furthermore the invention also provides triazolo-1,4-di-azepine compounds of the formula (II) and a pharmacologically acceptable salt thereof

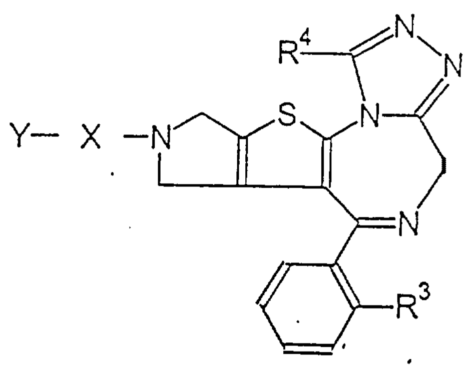

(II)

wherein $R^3$ represents a halogen atom, $R^4$ represents a $C_1$- alkyl group, X represents

(a) a group of the formula

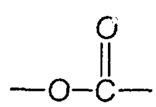

and Y represent

(2) a $C_3$-$C_7$ cycloalkyl-$C_1$-$C_6$ alkyl,

(3) an alkynyl group having up to 6 carbon atoms,

(4) a group of the formula

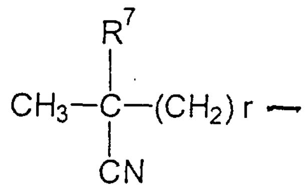

in which $R^7$ is hydrogen or methyl and r is zero, 1 or 2,

(5) a group of the formula NC-$(CH_2)_p$- (wherein p is an integer of from 1 to 6), or

(6) a group of the formula A-$(CH_2)_q$- (wherein A represents a group selected from a pyridyl group, a pyranyl group and a morpholino group and q is an integer of from 0 to 6).

The invention further involves the related triazolo-1,4-diazepine compounds of claims 20-25.

In the invention a pharmaceutical composition comprises a pharmacologically effective amount of a compound or the salt thereof as defined above, and a pharmacologically acceptable carrier. A method for treating a disease against which anti-PAF activity is effective, which comprises administering a pharmacologically effective amount of the compound or the salt thereof as defined above. The disease is an allergic disease such as athma.

The 1,4-diazepine derivatives of the general formula (I) or (II) have good PAF-inhibiting efficacy and good persistency with high safety.

In the compounds (I) of the invention, the $C_{1-6}$ alkyl group for R1, R2, R4, R5, R6, R8, R9 is a linear or branched alkyl group having from 1 to 6 carbon atoms and includes, for example, a methyl group, an ethyl group, a propyl group, an isopropyl group, a butyl group, an isobutyl group, a sec-butyl group, a tert-butyl group, a pentyl group (amyl group), an isopentyl group, a neopentyl group, a tert-pentyl group, a 1-methylbutyl group, a 2-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group or the like. Of these, preferable groups include a methyl group, an ethyl group, a propyl group and an isopropyl group, of which the methyl group is most preferred.

The $C_{3-7}$ cycloalkyl group mentioned in the definition of Y is for example, cyclopropyl, cyclobutyl, cyclopentyl, cyclohexyl or cycloheptyl. Of these, cyclopropyl, cyclobutyl and cyclopentyl are most preferred. The cycloalkyl may have a substituent such as methyl.

The cycloalkylakyl group is a group which is derived from the above-indicated cycloalkyl group. Typical examples include cyclopentylmethyl, cyclopropylmethyl, cyclohexylmethyl, and cyclohexylethyl groups.

The cycloalkylalkenyl group is a group which is derived from the above-indicated cycloalkylalkyl group. Typical and preferable groups include, for example, those of the following formulae

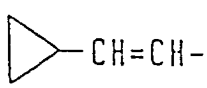 , 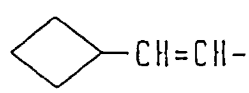 ,

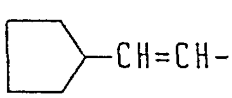 , 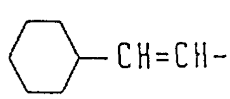

The alkynyl group is a group which has upto 6 carbon atoms and a triple bond at any portion thereof. Typical alkynyl groups include, for example, $CH\equiv C-CH_2-$, $CH\equiv C-CH_2-CH_2-$, $CH\equiv C-CH_2-CH_2-CH_2-$, $CH\equiv C-CH_2-CH_2-CH_2-CH_2-$,

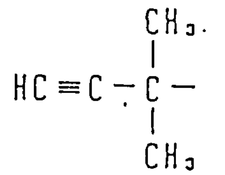

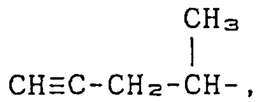

$CH_3$-C≡C-$CH_2$-$CH_2$-, and $CH_3$-C≡C-$CH_2$-. Of these, CH≡C-$CH_2$-, CH≡C-$CH_2$-$CH_2$-, CH≡C-$CH_2$-$CH_2$-$CH_2$- and CH≡C-$CH_2$-$CH_2$-$CH_2$-$CH_2$- are most preferred.

In the formula (4),

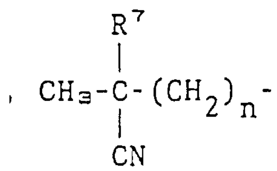

for Y, $R^7$ is hydrogen, or preferably a methyl group.

In the formula (5), p is from 1 to 6m preferably from 1 to 4.

In the formula (6), q is from 0 to 6.

In the formula (7), an alkynyl group having from upto 6 carbon atoms wherein a phenyl group or a cycloalkyl group is joined to any carbon atom of the group includes, for example, those groups of the following formulae

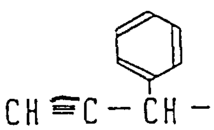

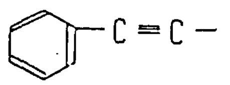

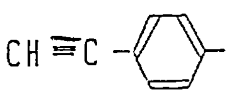

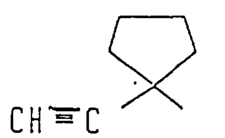

In (9), $R^8$ and $R^9$ may form a ring which has the following formula :

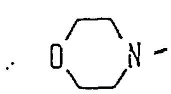

,

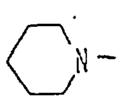

,

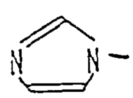

,

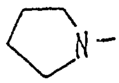 , 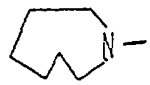 , 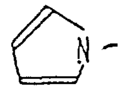 .

As described before, when X represents a group of the formula,

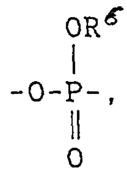

Y is a $C_{1-6}$ alkyl group, preferably a methyl or ethyl group.

In the practice of the invention, the most preferable group represented by X is a group of the formula (a)

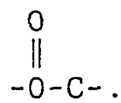

A more preferable group is of the formula (b),

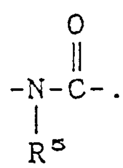

Preferably, groups of the formula (c),

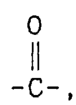

or the formula (d),

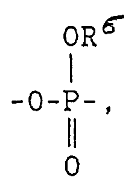

are used.

In the present invention, a first preferable group of compounds are those of the following chemical, structural formula

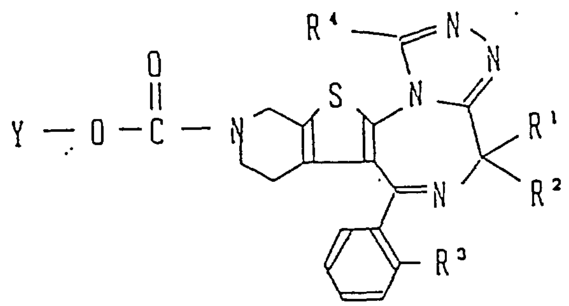

(A)

wherein $R^1$, $R^2$, $R^3$, $R^4$ and Y have, respectively, the same meanings as defined before where the most preferable group represented by Y is a group of the formula,

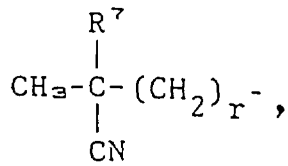

in which R7 is hydrogen or methyl, r is zero, 1 or 2, a group of the formula, NC-$(CH_2)_p$- wherein p is an integer of from 1 to 6, or an alkynyl group having upto 6 C-atoms. Most preferably, $R^3$ is a halogen atom such as a chlorine atom and $R^4$ is a methyl group.

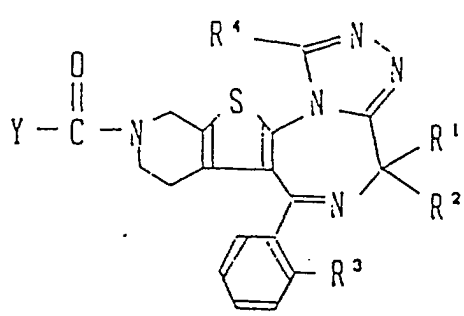

(B)

wherein $R^1$, $R^2$, $R^3$, $R^4$ and Y have, respectively, the same meanings as defined before where the most preferable group for Y is a $C_{3-7}$ cycloalkyl such as cyclopropyl, an alkynyl having upto 6 c-atoms, a $C_{3-7}$ cycloalkyl-$C_{1-6}$-alkyl group, a $C_{3-7}$cycloalkyl- wherein the alkenyl group has upto 6 C-atoms alkenyl group, a group of the formula,

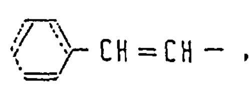

or a group represented by the formula, NC-$(CH_2)_p$- wherein p is an integer of from 1 to 6.

A second preferable group of compounds are those of the chemical, structural formula (C)

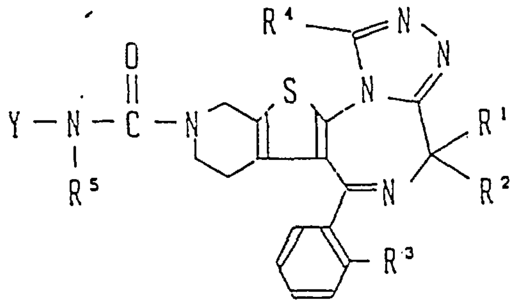

(C)

wherein $R^1$, $R^2$, $R^3$, $R^4$, $R^5$ and Y have, respectively, the same meanings as defined before where the most preferable group

represented by Y is a group of the formula,

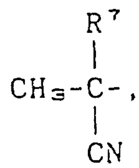

wherein $R^7$ represents a hydrogen atom or a methyl group, a group of the formula, $NC-(CH_2)_p-$ wherein p is an integer of from 1 to 6, or an alkynyl group having up to 6 C-atoms.

The pharmacologically acceptable salts used in the present invention are ordinarily employed innoxious salts such as, for example, inorganic salts such as hydrochlorides, hydrobromides, .sulfates, phosphates and the like, organic salts such as acetates, maleats, succinates and methanesulfonates , and salts of amino acids such as alginine, aspartic acid and glutamic acid.

The compounds of the invention have asymmetric carbon in the molecule and may take various steric isomers. In the practice of the invention, the individual isomers and mixtures thereof are all within the scope of the invention.

The isomers can be obtained according to an usual process for preparation.

Moreover, some compounds may form hydrates, which are also within the scope of the invention.

The compounds of the invention are prepared by usual procedures, among which typical processes are described below.

Preparation Process 1

For the preparation of compounds of the formula (I) wherein X is of the formula (a),

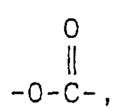

or of the formula (b),

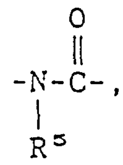

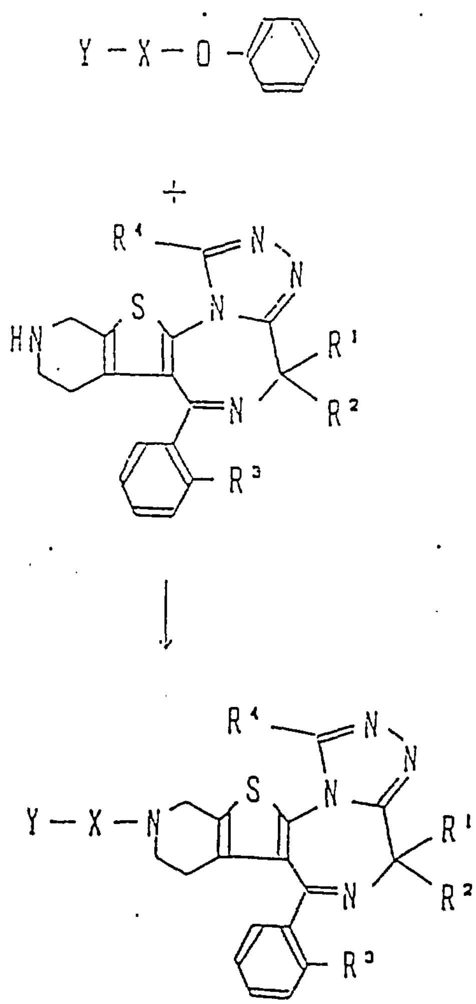

(XX)

(III)

(I')

wherein X, Y, $R^1$, $R^2$, $R^3$ and $R^4$ have, respectively, the same meaning as defined before.

The compound of the formula (XX) and the compound of the formula (III) are subjected to condensation reaction to obtain the compound of the general formula (I') which is one of intended substances.

This reaction is performed by usual manner in a solvent-free conditions or in a solvent inert to the reaction and selected from chloroform, tetrahydrofuran, diethyl ether, acetone, benzene, toluene and dimethylformamide. The reaction temperature is generally from room temperature to approximately 150°C and most preferably from 100 to 130°C.

In the above reaction, the compound of the general formula (XX) which is used as the starting material is prepared, for example, according to the following process.

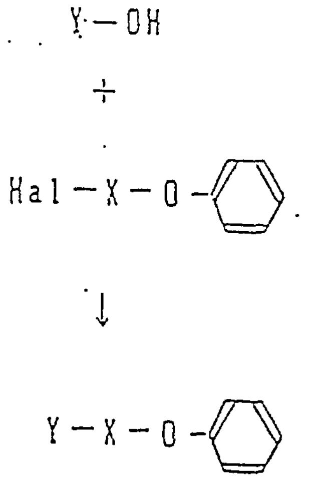

(IV)

(V)

(XX)

wherein Y and X have, respectively, the same meanings as defined before, and Hal represents a halogen atom.

In the above reaction, the compound of the general formula (IV) is subjected to condensation reaction with the halide of the general formula (V) to obtain the compound of the general formula (XX).

The reaction should preferably be effected in the presence of bases including amines such as triethylamine and pyridine , alkali hydrides such as sodium hydride and potassium hydride, and alkali hydroxides such as sodium hydroxide, and potassium hydroxide.

This reaction may be performed in the absence of solvent or in a solvent. Examples of the solvent include ethers such as tetrahydrofuran and dioxane, halogen-based compounds such as methylene chloride and, chloroform benzene compounds such as benzene, toluene and xylene, and compounds such as dimethylformamide and dimethylsulfoxide

Preparation Process 2

For the preparation of compounds wherein X is of the formula,

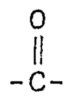

,

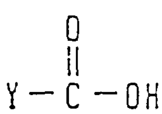

(VI)

or its reactive acid derivative

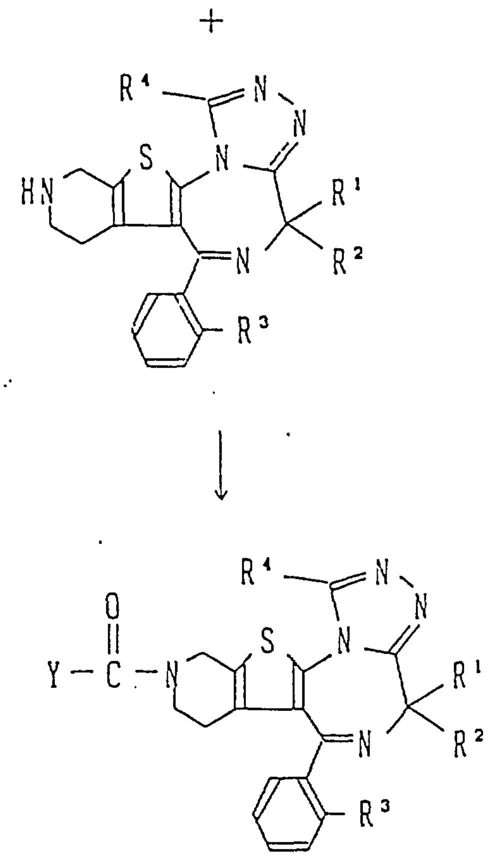

(III)

(I")

More particularly, the carboxylic acid of the general formula (VI) or its reactive derivative and the compound of the general formula (III) are subjected to condensation reaction to obtain the compound of the general formula (I") which is one of intended substances.

This condensation reaction is carried out by usual manner. The reactive derivatives include: acid halides such as acid chlorides and acid bromides; acid azides; N-hydroxybenzotriazole; active esters such as N-hydroxysuccinimide; symmetric acid anhydrides; mixed acid anhydrides with alkali carbonates and p-toluenesulfonic acid.

This reaction is carried out by heating in a solvent-free condition or in a solvent not taking part in the reaction, e.g. benzene, toluene, xylene, tetrahydrofuran, chloroform, carbon tetrachloride or dimethylformamide thereby causing, for example, dehalogenation reaction. Better results are obtained when the reaction is effected in the presence of inorganic salts such as sodium hydrogencarbonate, potassium carbonate, sodium carbonate and caustic soda or organic bases such as triethylamine, pyridine, pyrimidine and diethylaniline.

When free carboxylic acids are used, better results are obtained for the reaction in the presence of a condensing agent such as dicyclohexylcarbodiimide, 1,1'-carbonyldiimidazole or the like.

Preparation Process 3

For the preparation of compounds wherein X is of the formula,

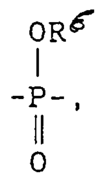

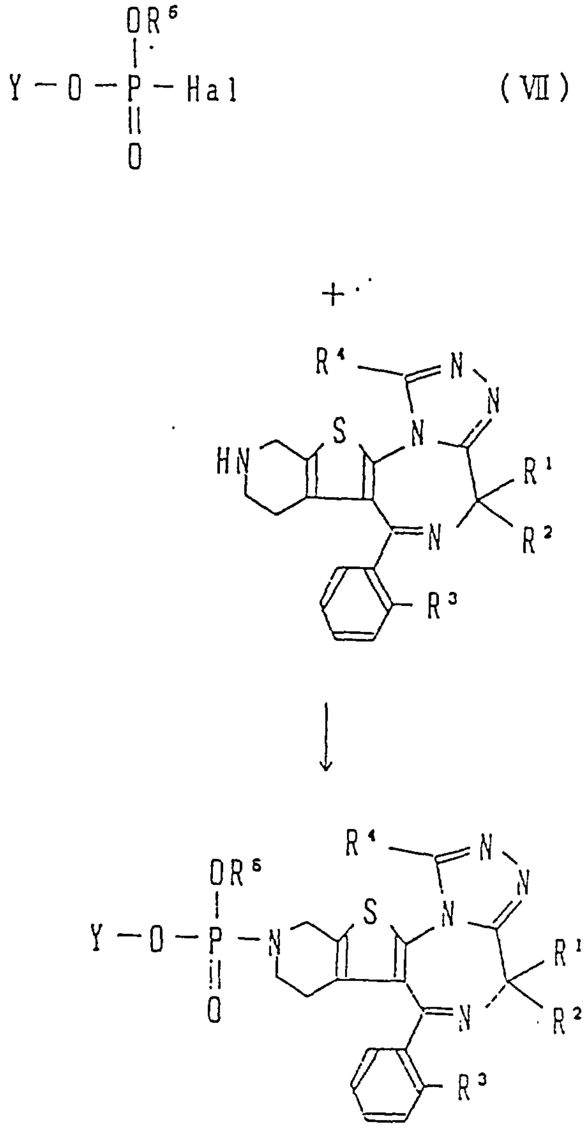

(VII)

(III)

(I''')

wherein Y, $R^1$, $R^2$, $R^3$, $R^4$ and $R^6$ have, respectively, the same meanings as defined before, and Hal represents a halogen atom.

The halide compound of the general formula (VII) and the compound of the general formula (III) are reacted to obtain compound (I''') which is an intended substance.

The reaction is a dehydrohalogenation reaction which is effected by usual manner under heating conditions in a solvent-free condition or in a solvent not taking part in the reaction and selected, for example, from benzene, toluene, xylene, tetrahydrofuran, chloroform, carbon tetrachloride and dimethylformamide. Better results are obtained when the reaction is carried out in the presence of inorganic salts such as sodium hydrogencarbonate, potassium carbonate, sodium carbonate and caustic soda or organic bases such as triethylamine, pyridine, pyrimidine and diethylaniline.

The starting compound (III) used in the above Preparation Processes 1 to 3 can be prepared, for example, according to the following procedure:

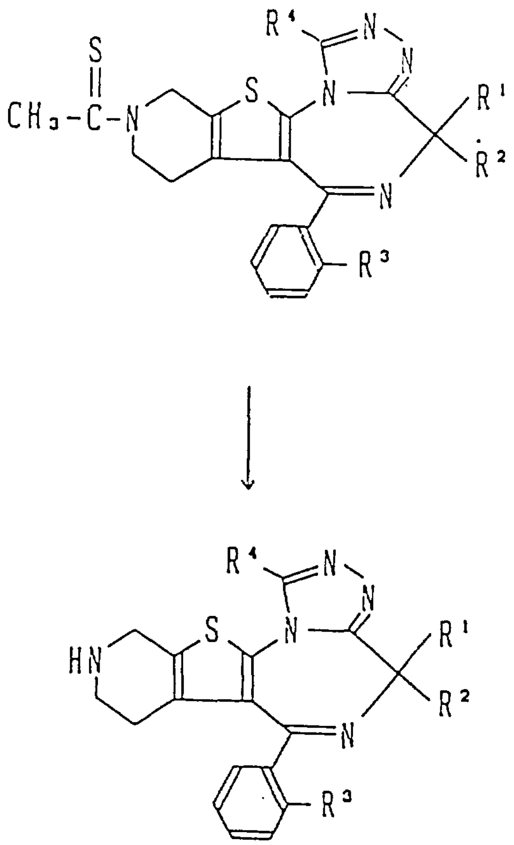

(IX)

(III)

wherein $R^1$, $R^2$, $R^3$, and $R^4$ have, respectively, the same meanings as defined before.

In the above reaction, the thioamide compound of the general formula (IX) is subjected to hydrolysis reaction to obtain the compound of the general formula (III).

The present reaction is carried out by usual manner wherein the compound of the general formula (III) can be obtained by heating in the presence, for example, of sodium hydroxide, potassium hydroxide, sodium ethoxide, sodium methoxide, potassium ethoxide or, potassium methoxide. For the reaction, there may be used a solvent such as, for example, an alcohol solvent such as methyl alcohol or, ethyl alcohol, tetrahydrofuran, dimethoxyethane, or a hydrous solvent.

With the above starting compound (III) wherein $R^1$ is a hydrogen atom, $R^2$ is a methyl group (not covered by the claims) and $R^4$ is a methyl group, the preparation process can be more particularly described as follows.

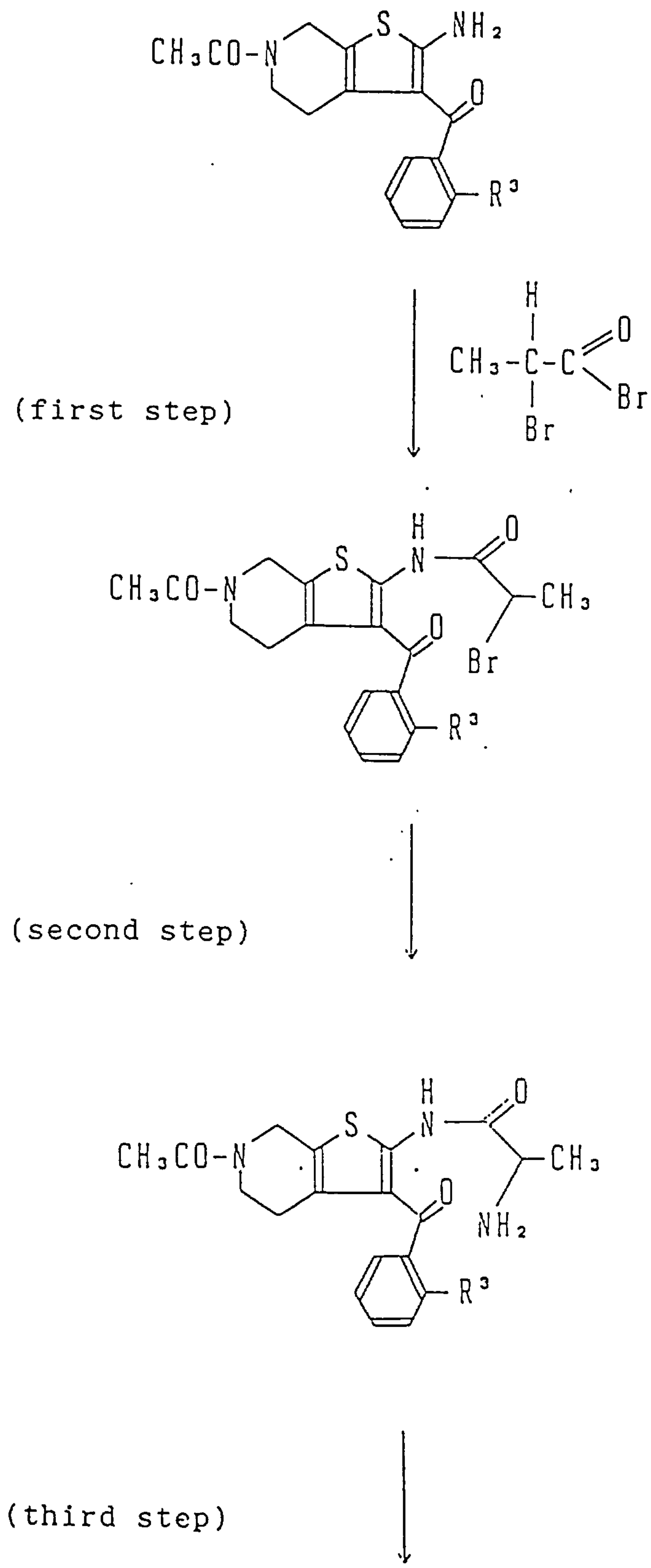


(X)

(XI)

(XII)

(XIII)

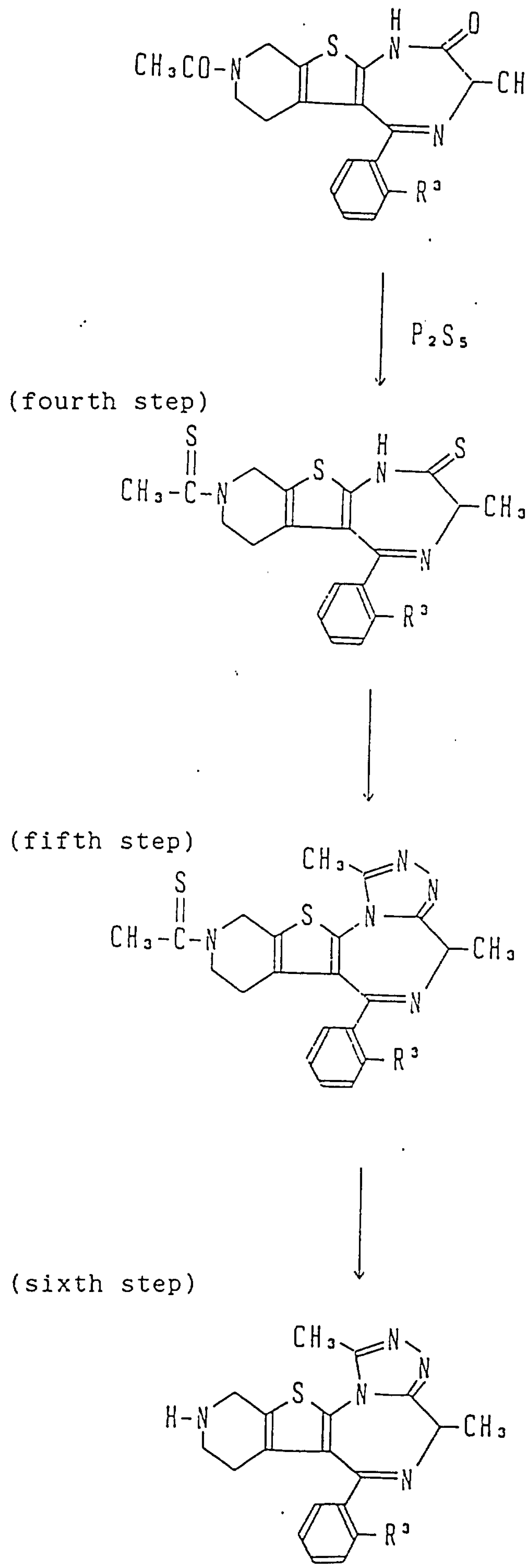


(XIV)

(XV)

(XVI)

(XVII)

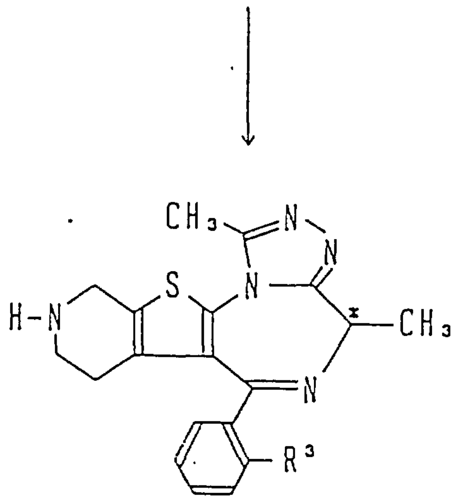

(XVIII)

wherein the mark "*" represents asymmetric carbon and (XVIII) represents the respective enanethiomer.

The respective steps indicated above are briefly illustrated in the following.

(First Step)

2-Bromopropionyl bromide of the formula (XI) is subjected to condensation reaction with the compound of the general formula (X) by usual manner to obtain the compound of the general formula (XII).

This reaction is carried out in a two-phase system (under Schotten-Bauimann conditions) of an organic solvent such as, for example, toluene, benzene or xylene in the presence of either an alkali hydroxide such as sodium hydroxide or potassium hydroxide or a base such as sodium hydrogencarbonate or, potassium hydrogencarbonate.

Alternatively, the reaction may be performed in the presence of a base including an amine such as triethylamine or pyridine, an alkali hydroxide such as sodium hydroxide or potassium hydroxide, or an alkali hydride such as sodium hydride or potassium hydride in a solvent not taking part in the reaction such as, for example, dichloromethane, dichloroethane, tetrahydrofuran, toluene, benzene, xylene or, dimethylformamide.

(Second Step)

In this step, ammonia gas is introduced into the compound of the general formula (XII) by usual manner to obtain the compound (XIII).

This reaction should preferably be effected at low temperatures ranging, for example, from 30°C to 100°C.

The reaction is carried out in a solvent-free condition or by the use of an appropriate solvent which does not take part in the reaction and is selected from ethers such as tetrahydrofuran and, dioxane, ethyl acetate, chloroform, methanol, ethanol, pyridine and dichloroethane.

(Third Step)

In this step, the compound of the general formula (XII) is subjected to dehydration reaction by usual manner thereby causing cyclization to obtain the compound of the general formula (XIV).

One of the procedures is particularly described. The compound is dissolved in an appropriate solvent not taking part in the reaction such as, for example, benzene, toluene, xylene or pyridine, to which one equivalent of an acid catalyst such as acetic acid, or silica gel. While removing the water produced as the reaction proceeds by the use of a dehydrator or by means of the Dean Stark apparatus, the reaction system is heated.

(Fourth Step)

This step is one wherein phosphorus pentasulfide is added to the compound of the general formula (XIV) for reaction to obtain the compound of the general formula (XV).

This reaction is carried out in a solvent such as pyridine, dimethoxyethane, diglymes, tetrahydrofuran, toluene, benzene or xylene. The reagent may be, aside from phosphorus pentasulfide, the Lauson reagent, (2,4-bis(4-methoxyphenyl9-1,3-dithia-2,4-diphosphetan-2,4-disulfide). In some case, the reaction is carried out in the presence of a base such as sodium hydrogencarbonate.

(Fifth Step)

This step involves the reaction wherein acetohydrazide is reacted with the compound of the general formula (XV) to cause the cyclization reaction, thereby obtaining compound of the general formula (XVI).
This reaction is effected by heating acetohydrazide in a solvent which does not take part in the reaction such as, for example, dioxane, dimethoxyethane or diglymes or in a solvent-free condition. Alternatively, hydrazide hydrate is reacted in a solvent such as methanol or ethanol and the resultant hydrazide is reacted to ethyl ortho-acetate to obtain an intended product. Still alternatively, hydrazide may be reacted with acetyl chloride or acetic anhydride and the resultant product is dehydrated to obtain compound (XVI).

(Sixth Step)

This step is one wherein the compound of the general formula (XVI) is hydrolyzed by usual manner to obtain the compound of the general formula (XVII).
This reaction proceeds according to known procedures. For instance, heating in the presence of potassium hydroxide, sodium hydroxide, sodium ethoxide, sodium methoxide, potassium ethoxide or potassium methoxide results in the compound of the general formula (XVII).
For the reaction, solvents may be used including alcohol solvents such as methyl alcohol or ethyl alcohol, tetrahydrofuran, dimethoxyethane or hydrous solvents.
The compound of the general formula (XVII) is an important intermediate for obtaining final compounds having good anti-PAF activity.
The compounds of the general formula (III), of which those of the formula wherein $R^1$ is a hydrogen atom and $R^2$ is a $C_1$-$C_6$ alkyl group are very valuable as intermediates.
The intermediates have asymmetric carbon and thus optical isomers exist. In the practice of the invention, $d\ell$ products may be resolved into optically active products, if desired.
The resolution may be performed at the stage of the compound of the general formula (XIII) wherein an optical resolving agent such as (+)-tartaric acid, (+)-camphoric acid, (+)-dibenzoyltartaric acid, (+)-10-camphorsulfonic acid or (+)-mandelic acid may be used for the resolution. Alternatively, at the stage of the compound of the general formula (III) or (XVII), the resolution may be possible using an optical resolving agent such as dibenzoyl-D-tartaric acid or dibenzoyl-L-tartaric acid. Still alternatively, when using a column for optical isomer resolution such as, for example, a chiral polyamide silica gel HPLC (elute: tetrahydrofuran-hexane), the resolution at the stage of the compound of the general formula (XIII), (XVII) or (III) is possible.
The other compounds can be obtained in the same way, except for changing starting materials.
The effects of the invention are more particularly described by way of experimental example.

Experimental Example

PAF Recepteor Binding Assay to the Human Platelet

(Method)

Platelets are obtained from healthy men according to usual method and suspended at a concentration of $10^8$ platelets/460 µl in a binding buffer (10 mM phosphate-buffers saline (pH 7.0), with 0.1% (w/V) BSA and 0.9 mM $CaCl_2$). Platelets ($10^8$) in 460 µl of the buffer were added to polypropylene tubes and preincubated with test compounds (20 µl), after vortexing, for 6 min at 37 °C. Subsequently, 20 µl of a binding buffer solution of $^3$H-PAF (final $^3$H-PAF concentration 0.6 - 1 nM) was added to the tubes, which were incubated for 6 minutes. The binding reaction was stopped by adding of 3 ml of an ice-cold washing solution (saline containing 0.1% (w/v) BSA). Platelets were isolated by vaccum filtration on glass filters (Whatman GF/C). After drying the glass filter, radioactivity on the glass filter was measured in scintilator with a liquid scintillation counter.
The inhibition percent is caluculated according to the following equation and the value of IC is determined by interpolation from the figure.

$$\text{Inhibition \%} = \frac{\text{(total binding) - (total binding with compound)}}{\text{(total binding ) - (non-specific binding total)}}$$

binding : radioactivity of binding in the absence of cold PAF or test compounds
non-specific binding : radioactivity of binding in the presence of $10^{-5}$ M PAF

These results are shown in Table I.

non-specific binding : radioactivity (dpm) after the incubation with $10^{-5}$ M of cold PAF.

The results are shown in Table 1.

The mark "*" indicates asymmetric carbon and the marks "(+)" and "(-)" indicate specific rotation.

As shown in Table 1, it is obvious that these compounds have anti-PAF activity. Moreover, it has been found that the compounds possess more potent and long-acting anti-PAF activity, and show better safety properties than known compounds. Thus, the present invention has a great merit.

Accordingly, the compounds will be effective for the therapy and prophylaxis of all diseases mediated by PAF.

Typical diseases for which the compounds are useful as a therapeutic and prophylactic agent include allergic diseases, asthma, thrombosis, cerebral apoplexy (cerebral hemorrhage, cerebral thrombosis), myocardial infarction, (angina pectoris), human disseminated intravascular coagulation syndrome (DIC), thrombophlebitis, glomerular hepatitis, anaphylactic shock, hemorrhagic shock and the like. The invented compounds will be particularly useful as an anti-allergic agent and an anti-asthmatic agent.

When these compounds are administered as an anti-PAF agent, they may be useful to orally dose in the form of a tablet, powder, granule, capsule, syrup or the like.
Alternatively, they may be parenterally dosed as a suppositoty, injection, external remedy or drip. In the case of the invention, the compounds should preferably be used as an oral agent.

The dosage may depend on the type of disease, the degree of symptom and the age. When these compounds are orally administered, the doses of 0.001 - 10 mg/kg, preferably 0.01 - 0.5 mg/kg, will be benefitial.

For the preparations to use as peroral and parenteral dose, they are made using ordinary, pharmaceutically acceptable additives. For the preparation of injections or drips, pH modifiers, buffer solutions, stabilizers and solubilizers are added to the principal ingredient, if necessary. The mixture can be freeze-dried, if necessary, to made injections for subcutaneous, intramuscular or intervenous or drip administrations.

Table 1

| Test compound | PAF receptor binding assay IC50 ($\mu$M) |
|---|---|
| 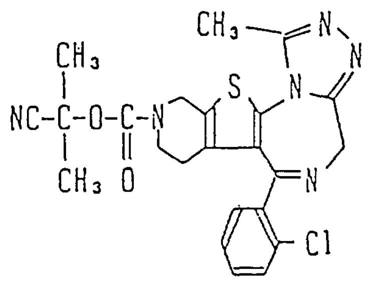 | 0.0033 |
| 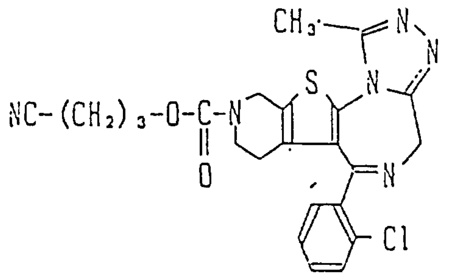 | 0.0027 |
| 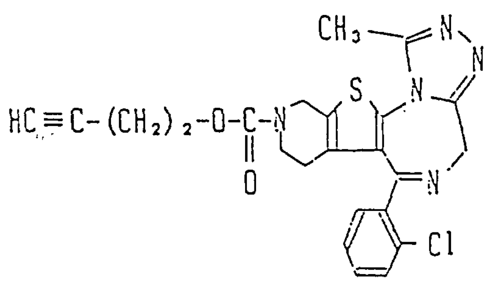 | 0.0035 |
| 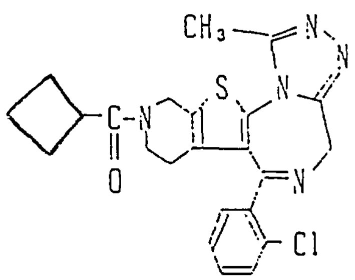 | 0.0018 |

Table 1 (Cont'd)

| | |
|---|---|
| 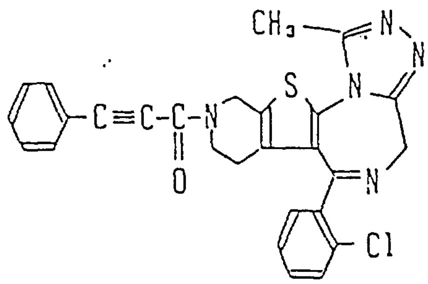 | 0.00056 |
| 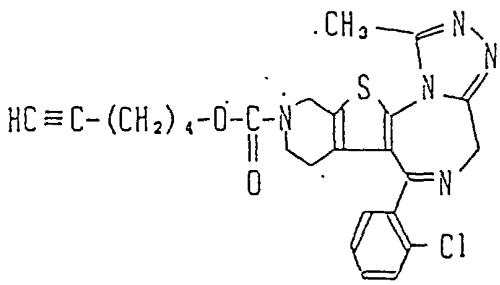 | 0.00022 |
| 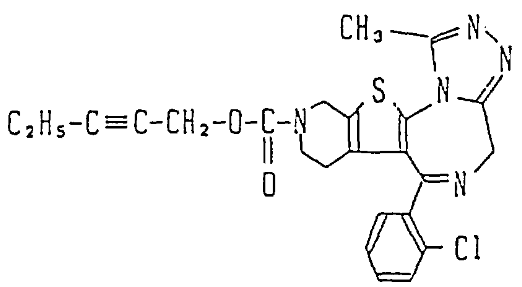 | 0.00074 |

[Examples]

Typical examples of the invention are described as follows:

(A) Examples 1 to 69
(B) Examples 70 to 92 and Preparation Examples 1 to 5,
(C) Examples 93 to 106 and Preparation Examples 6 to 14, and

are disclosed hereinafter.

It will be noted that the preparation of starting compounds or substances will be described as Preparatory Examples.

Example 1

6-(2-Chlorophenyl)-3-(1-cyano-1-methylethoxycarbonyl)-11-m ethyl-2,3,4,5-tetrahydro-8H-pyrido[4',3':4,5]thieno [3,2-f][1,2, 4]triazolo[4,3-a][1,4]diazepine

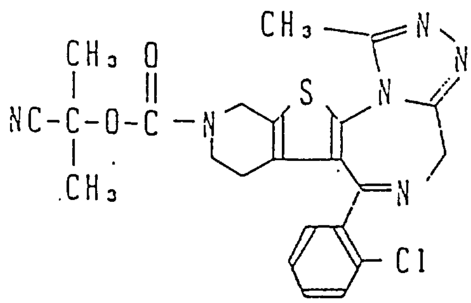

(1) Synthesis of 1-cyano-1-methylethyl phenyl carbonate

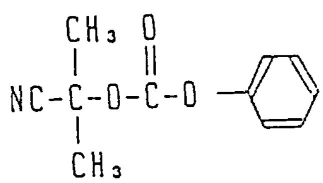

1.40 g (9 mmols) of phenyl chloroformate was dropped into a pyridine solution (20 ml) of 0.85 g (10 mmols) of acetone cyanohydrin under ice-cooling conditions, followed by agitation for 30 minutes. After completion of the reaction, the solvent was distilled off to obtain a residue, which was dissolved in chloroform, followed by washing with N hydrochloric acid and a saturated sodium hydrogencarbonate aqueous solution and drying with magnesium sulfate. The resultant product was purified by silica gel column chromatography (elution solvent: ethyl acetate: n-hexane = 1: 49), thereby quantitatively obtaining the intended compound in the form of a colorless solid matter.

(2) Synthesis of 6-(2-chlorophenyl)-3-(1-cyano-1-methylethoxycarbonyl)-11-methyl-2,3,4,5-tetrahydro-8H-pyrido[4', 3':4,5]thieno[3,2-f][1,2,4]triazolo[4,3-a][1,4]diazepine

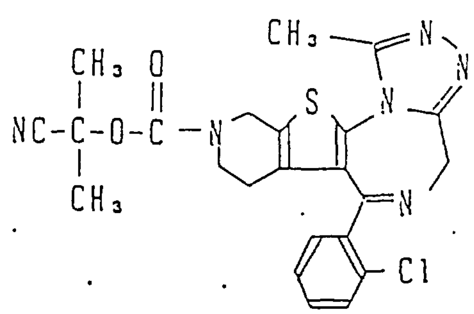

0.15 g of 1-cyano-1-methylethyl phenyl carbonate and 0.15 g of 6-(2-chlorophenyl)-11-methyl-2,3,4,5-tetrahydro-8H-pyrido[4',3':4,5]thieno[3,2-f][1,2,4]triazolo[4,3-a][1,4]diazepine were dissolved in chloroform and made uniform, after which the solvent was distilled off. The resultant mixture was agitated at a bath temperature of 120°C for 1 hour. After cooling, purification by silica gel column chromatography (elution solvent: chloroform:methanol = 99:1) could yield 0.18 g of the intended product as amorphous.

· $^1$H-NMR (90MHz, $CDCl_3$) δ

1.77(6H,s), 1.80 - 2.20(2H,m), 2.68(3H,s), 3.10-3.60(2H,m), 4.22(1H,m), 4.50 - 4.88(2H,m), 5.60(1H,m), 7.35 (4H,m)

· FABMS ($M+H^+$) m/z:481

Example 2

6-(2-Chlorophenyl)-3-(3-cyanopropoxycarbonyl)-11-methyl-2, 3,4,5-tetrahydro-8H-pyrido[4',3':4,5]thieno[3,2-f] [1,2,4] triazolo[4,3-a][1,4]diazepine

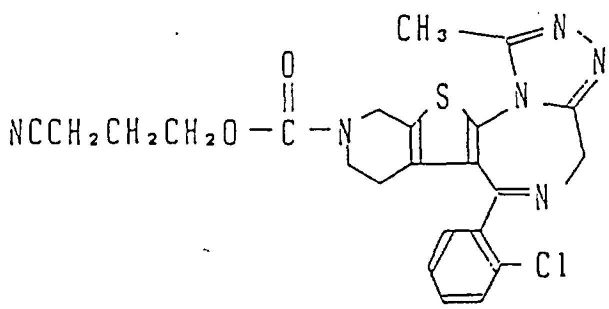

(1) Synthesis of 3-cyanopropyl phenyl carbonate

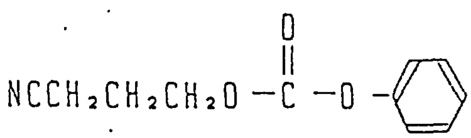

1.50 g of phenyl chloroformate was dropped into a chloroform solution (20 ml) of 0.85 g of 4-hydroxybutyronitrile and 1.50 g of pyridine under ice-cooling conditions, followed by agitation for 30 minutes. After completion of the reaction, the reaction mixture was washed with a saturated sodium hydrogencarbonate aqueous solution and dried with magnesium sulfate, after which the solvent was distilled off, followed by purification by silica gel column chromatography (elution solvent: ethyl acetate: n-hexane = 3: 17), thereby obtaining 1.20 g of the intended compound.

(2) Synthesis of 6-(2-chlorophenyl)-3-(3-cyanopropoxycarbonyl)-11-methyl-2,3,4,5-tetrahydro-8H-pyrido[4',3':4, 5] thieno[3,2-f][1,2,4]triazolo[4,3-a][1,4]diazepine

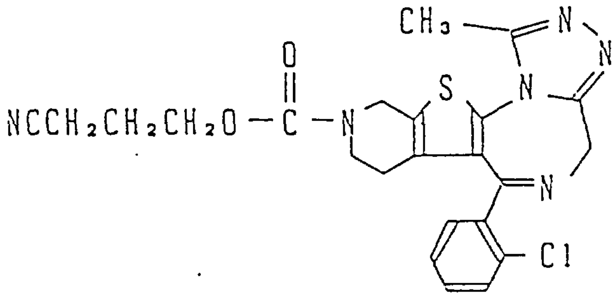

0.11 g of 1-cyanopropyl phenyl carbonate and 0.13 g of 6-(2-chlorophenyl)-11-methyl-2,3,4,5-tetrahydro-8H-pyrido[4',3':4,5]thieno[3,2-f][1,2,4]triazolo[4,3-a][1,4]diazepine were dissolved in chloroform and made uniform, after which the solvent was distilled off. The resultant mixture was agitated at a bath temperature of 110°C for 1 hour. After cooling, purification by silica gel column chromatography (elution solvent: chloroform:methanol = 49:1) could yield 0.10 g of the intended product.

· $^1$H-NMR (90MHz, $CDCl_3$) δ

1.41 - 1.80(m, 2H), 1.80 - 2.17(m, 2H), 2.22 - 2.52(m,2H), 2.60(s, 3H), 2.80 - 5.76(m, 6H), 4.20(t, J=7Hz, 2H), 7.30(m, 4H)

· FABMS ($M+H^+$) m/z:481

Example 3

3-(3-Butynyloxycarbonyl)-6-(2-chlorophenyl)-11-methyl-2,3, 4,5-tetrahydro-8H-pyrido[4',3':4,5]thieno[3,2-f] [1,2,4] triazolo[4,3-a][1,4]diazepine

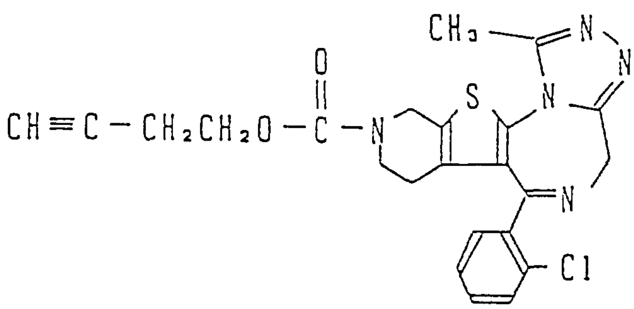

(1) Synthesis of 3-butynyl phenyl carbonate

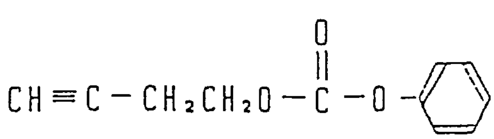

1.70 g of phenyl chloroformate was dropped into a dichloromethane solution (20 ml) of 0.70 g of 3-butyn-1-ol

and 1.50 g of pyridine under ice-cooling conditions, followed by agitation for 30 minutes. After completion of the reaction, the reaction mixture was washed with a saturated sodium hydrogencarbonate aqueous solution and dried with magnesium sulfate, after which the solvent was distilled off, followed by purification by silica gel column chromatography (elution solvent: ethyl acetate: n-hexane = 1: 49), thereby obtaining the intended compound as a colorless oil at a quantitative yield.

(2) Synthesis of 3-(3-butynyloxycarbonyl)-6-(2-chlorophenyl-propoxycarbonyl)-11-methyl-2,3,4,5-tetrahydro-8H-pyrido[4',3':4,5]thieno[3,2-f][1,2,4]triazolo[4,3-a][1,4]diazepine

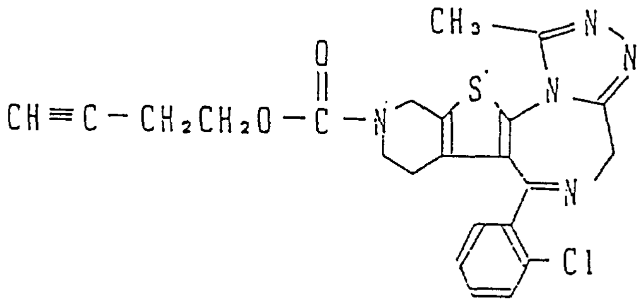

0.10 g of 3butynyl phenyl carbonate and 0.18 g of 6-(2-chlorophenyl)-11-methyl-2,3,4,5-tetrahydro-8H-pyrido [4',3':4,5]thieno[3,2-f][1,2,4]triazolo[4,3-a][1,4]diazepine were dissolved in chloroform and made uniform, after which the solvent was distilled off. The resultant mixture was agitated at a temperature of 110°C for 1 hour. After cooling, purification by silica gel column chromatography (elution solvent: chloroform:methanol = 99:1) could yield 0.17 g of the intended product.

· $^1$H-NMR (90MHz, $CDCl_3$) δ

1.60 - 2.16(m, 2H), 1.94 (s,3H), 2.50(dt, J=2Hz, 7Hz, 2H), 2.66(s, 3H), 2.86 - 5.74(m, 6H), 4.17(t, J=7Hz, 2H), 7.29(m, 4H)

· MS m/z(Pos. Gab):466$(M+H)^+$

Example 4

6-(2-Chlorophenyl)-3-(2-cyanoethylaminocarbonyl)-11-methyl-2,3,4,5-tetrahydro-8H-pyrido[4',3':4,5]thieno[3,2-f][1,2,4]triazolo[4,3-a][1,4]diazepine

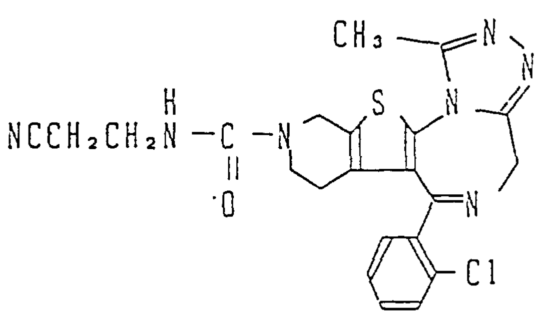

(1) Synthesis of N-(2-cyanoethyl)carbamate

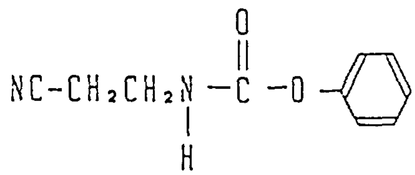

1.40 g of phenyl chloroformate was dropped into a dichloroethane solution (20 ml) of 0.70 g of 3-aminopropionitrile and 1.20 g of triethylamine under ice-cooling conditions, followed by agitation for 30 minutes.

After completion of the reaction, the reaction mixture was washed with saturated sodium hydrogencarbonate and dried with magnesium sulfate, after which the solvent was distilled off, followed by purification by silica gel column chromatography (elution solvent: ethyl acetate: n-hexane = 1: 9), thereby obtaining 1.30 g of the intended compound.

(2) Synthesis of 6-(2-chlorophenyl)-3-(2-cyanoethylaminocarbonyl)-11-methyl-2,3,4,5-tetrahydro-8H-pyrido[4',3' :4,5] thieno[3,2-f][1,2,4]triazolo[4,3-a][1,4]diazepine

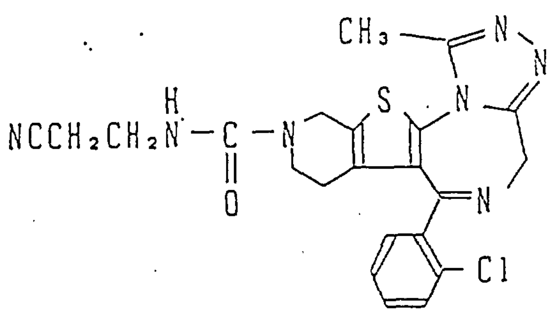

0.09 g of N-(2-cyanoethyl)carbamate and 0.18 g of 6-(2-chlorophenyl)-11-methyl-2,3,4,5-tetrahydro-8H-pyrido[4',3':4,5]thieno[3,2-f][1,2,4]triazolo[4,3-a][1,4]diazepine were dissolved in chloroform and made uniform, after which the solvent was distilled off. The resultant mixture was agitated at 140°C for 1 hour. After cooling, purification by silica gel column chromatography (elution solvent: chloroform:methanol = 19:1) could yield 0.12 g of the intended product.

· $^{1}$H-NMR (90MHz, $CDCl_3$) δ ;

1.45 - 2.23(m, 2H), 2.60(t, J=7Hz, 2H), 2.64(s,3H), 2.80-5.69(m, 9H), 7.29(m, 4H)

· MS m/z(Pos, Fab):466 $(M+H)^+$

Example 5

6-(2-Chlorophenyl)-11-methyl-3-(2-propynyl)-2,3,4,5-tetrahydro-8H-pyrido[4',3':4,5]thieno[3,2-f][1,2,4]triazolo[4,3-a][1,4]diazepine

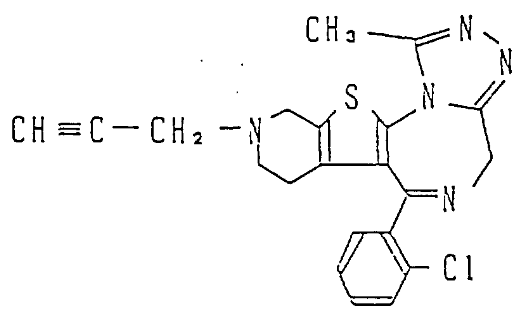

30 mg of sodium hydride (60%) was added to a dimethylformamide (20 ml) solution of 0.12 g of 6-(2-chlorophenyl)-11-methyl-2,3,4,5-tetrahydro-8H-pyrido[4',3':4,5]thieno[3,2-f][1,2,4]triazolo[4,3-a][1,4]diazepine at room temperature, followed by agitation at 60°C for 1 hour. Again, 60 mg of 3-bromopropyne was added at room temperature and agitated at 60°C for 1 hour. After cooling, water was added to the reaction mixture, which was extracted with ethyl acetate and dried with magnesium sulfate, followed by removal of the solvent and purification by silica gel column chromatography (elution solvent:chloroform:methanol = 98.5:1.5), thereby obtaining 20 mg of the intended product.

· $^1$H-NMR (90MHz, $CDCl_3$) δ

1.52 - 2.12(m, 2H), 2.25(t, J=2Hz, 1H), 2.16 - 2.84(m,2H), 2.66(s, 3H), 3.45(d, J=2Hz,2H), 3.74(m, 2H), 3.90 - 4.40, 5.20-5.76(2m, 2H), 7.27(m, 2H)

· MS m/z:407

Example 6

6-(2-Chlorophenyl)-11-methyl-3-cyclopropanecarbonyl-11-methyl-2,3,4,5-tetrahydro-8H-pyrido[4',3':4,5]thieno[3,2-f][ 1,2,4]triazolo[4,3-a][1,4]diazepine

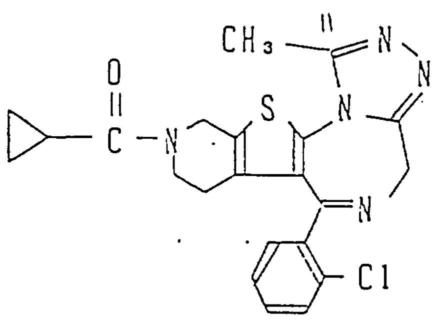

90 mg of cyclopropanecaronyl chloride was dissolved in 4 ml of N-dimethylformamide, into which an N,N-dimethylformamide solution (6 ml) of 150 mg of 6-(2-chlorophenyl)-11-methyl-2,3,4,5-tetrahydro-8H-pyrido[4',3':4,5]thieno[3,2-f]-[1,2,4]triazolo[4,3-a][1,4]diazepine and 210 mg of triethylamine was dropped at -60°C and agitated as it is for 30 minutes. After removal of the solvent by distillation,. a saturated sodium hydrogencarbonate aqueous solution was added, followed by extraction with chloroform and drying with anhydrous magnesium sulfate. This was filtered off and, after removal of the solvent by distillation, the resultant residue was subjected to silica gel column chromatography (developing solvent: MeOH:$CH_2Cl_2$ = 1:99) to obtain 140 mg of the captioned compound (yield 79%).

· $^1$H-NMR (90MHz, $CDCl_3$) δ :

0.4 - 1.3 (m,4H), 1.4 - 2.7(m, 3H), 2.67(s,3H), 2.8-5.8(m, 6H), 71. - 7.6(m, 4H),

· MS m/z (Pos, Fab): 438(M+H)+

Example 7 (reference example)

6-(2-Chlorophenyl)-3-cyclopropanecarbonyl-8, 11-dimethyl-2,3,4,5-tetrahydro-8H-pyrido[4',3':4,5]thieno[3,2-f] [1,2,4] triazolo[4,3-a][1,4]diazepine (not claimed)

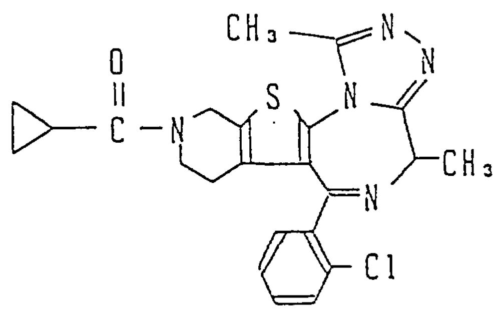

100 mg of 6-(2-chlorophenyl)-3-cyclopropanecarbonyl-11-methyl-2,3,4,5-tetrahydro-8H-pyrido[4',3':4,5] thieno[3,2-f][ 1,2,4]triazolo[4,3-a][1,4]diazepine was dissolved in 4 ml of N,N-dimethylformamide, to which 54 mg of sodium hydride (55%) and 0.5 ml of methyl bromide, followed by agitation for 1 hour at room temperature. The reaction was stopped by addition of water and the solution was neutralized with acetic acid. Subsequently, the solvent was distilled off under reduced pressure and the resultant residue was extracted with 20 ml of dichloromethane. The solution was dried with anhydrous magnesium sulfate, after which the solvent was removed, followed by purification with silica gel column chromatography (400 mesh, 10 g) to obtain the captioned product.

· $^1$H-NMR (90MHz, $CDCl_3$) δ :

0.55 - 1.15 (m, 4H), 1.45 - 2.5(m, 3H), 2.10(d, J=6.8 Hz,3H), 2.66(s, 3H), 2.8 - 4.8(m,3H), 4.26(q, J=6.8Hz, 1H), 4.8-5.2(m,1H), 7.05 - 7.65(m,4H)

· MS m/z(Pos. Fab): 452(M+H)+

Example 8

6-(2-Chlorophenyl)-3-cinnamoyl-11-methyl-2,3,4,5-tetrahydro-8H-pyrido[4',3':4,5]thieno[3,2-f][1,2,4]triazolo[4,3-a] [1,4]diazepine

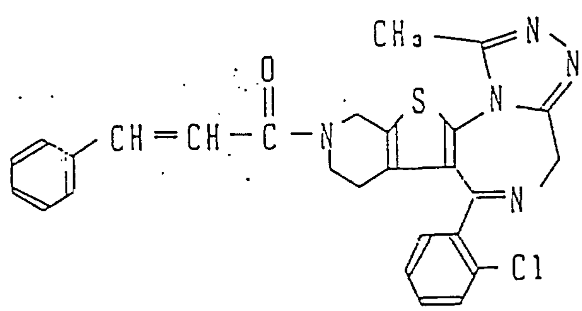

80 mg of cinnamoyl chloride was dissolved in 8 ml of N,N-dimethylformamide, into which 4 ml of an N,N-dimethylformamide solution of 120 mg of 6-(2-chlorophenyl)-11-methyl-2,3,4,5-tetrahydro-8H-pyrido[4',3': 4,5]thieno [3,2-f][1,2,4]triazolo[4,3-a][1,4]diazepine and 160 mg of triethylamine was dropped, followed by agitation as it is. After removal of the solvent by distillation, a saturated sodium hydrogencarbonate aqueous solution was added to, followed by extraction with chloroform and drying with anhydrous magnesium sulfate. The solution was filtered off and, after removal of the solvent by distillation, the resultant residue was subjected to silica gel column chromatography (devel-

oping solvent: MeOH:$CH_2Cl_2$ = 1:99) to obtain 11 mg of the captioned compound (yield 68%).

MS m/z(Pos. Fab): 500(M+H)$^+$

Example 9

6-(2-Chlorophenyl)-3-cyclobutanecarbonyl-11-methyl-2,3,4,5-tetrahydro-8H-pyrido[4',3':4,5]thieno[3,2-f][1,2,4]triazolo[4,3-a][1,4]diazepine

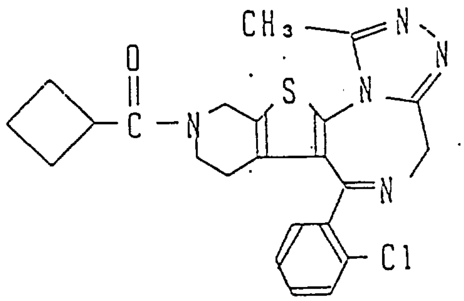

50 mg of cyclobutanecarboxylic acid, 70 mg of 1-hydroxybenzotriazole monohydrate and 150 mg of 6-(2-chlorophenyl)-11-methyl-2,3,4,5-tetrahydro-8H-pyrido[4',3': 4,5]thieno[3,2-f][1,2,4]triazolo[4,3-a][1,4]diazepine was dissolved in 8 ml of N,N-dimethylformamide, to which 100 mg of N,N'-dicyclohexylcarbodiimide was added under ice-cooling conditions, followed by agitation for about 10 minutes and agitation at 4°C overnight. Thereafter, after further agitation at room temperature for about 1 hour, the insoluble matter was removed by filtration and the solvent was distilled off. A saturated sodium hydrogencarbonate aqueous solution was added, followed by extraction with chloroform and drying with anhydrous magnesium sulfate. The resultant solution was subjected to filtration and the solvent was distilled off, and the residue was subjected to silica gel column chromatography (developing solvent: MeOH:$CH_2Cl_2$ = 1:99) to obtain 180 mg of the intended compound (yield 98%).

· $^1$H-NMR (90MHz, $CDCl_3$) δ :

1.4 - 2.5(m, 9H), 2.67(s, 3H), 2.8 - 5.9(m,6H), 7.1-7.6(m,4H)

· MS m/z(Pos. Fab): 452(M+H)$^+$

Example 10

6-(2-Chlorophenyl)-3-diethylphosphoro-11-methyl-2,3,4,5-tetrahydro-8H-pyrido[4',3':4,5]thieno[3,2-f][1,2,4]triazolo[4,3-a][1,4]diazepine

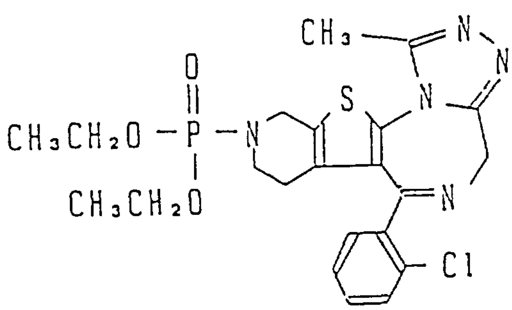

100 mg of 6-(2-chlorophenyl)-11-methyl-2,3,4,5-tetrahydro-8H-pyrido[4',3':4,5]thieno[3,2-f][1,2,4]triazolo[4,3-a][1,4]diazepine was dissolved in 5 ml of tetrahydrofuran and 1 ml of triethylamine, to which 100 mg of diethylchlorophosphate, followed by agitation at room temperature for 1 hour. The reaction solution was added to a saturated

sodium bicarbonate aqueous solution, followed by extraction with ethyl acetate and drying with anhydrous magnesium sulfate. The solvent was removed for concentration under reduced pressure and the resultant residue was purified by silica gel column chromatography (developing solvent: $CH_3OH:CH_2Cl_2$ = 5:95) to obtain 100 mg of the intended compound (yield 72%).

· MS(FAB)$(M+H)^+$ = 506

· NMR (90MHz):

1.28(t, 6H, J=8.0), 1.44 - 2.20(m, 2H), 2.69(s, 3H), 3.70-4.60(m,9H), 5.33 - 5.72(m,1H), 7.12 - 7.48(m,5H)

Example 11 (Reference Example)

6-(2-Chlorophenyl)-8,11-dimethyl-3-(3-cyanopropoxycarbonyl)-2,3,4,5-tetrahydro-8H-pyrido[4',3':4,5]thieno[3, 2-f][1,2,4]tri-azolo[4,3-a][1,4]diazepine (not claimed)

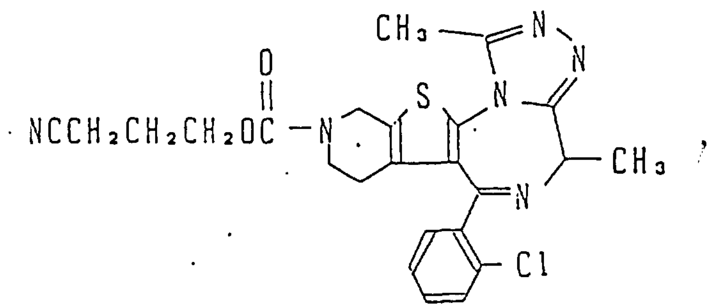

To a solution of 62 mg of 6-(2-chlorophenyl)-3-(3-cyanopropoxycarbonyl)-11-methyl-2,3,4,5-tetrahydro-8H-pyrido[4',3':4,5]thieno[3,2-f][1,2,4]triazolo-[4,3-a][1,4]diazepine dissolved in 2 ml of dimethylformamide at room temperature were added 34 mg of sodium hydride (55%) and 0.2 ml of methyl bromide, followed by agitation at at room temperature for 1 hour. Water was added to the solution in order to stop the reaction and the solution was neutralized with acetic acid. The solvent was distilled off under reduced pressure and the resultant residue was extracted with 10 ml of dichloromethylene and then with 20 ml of dichloromethylene. The solution was dried with magnesium sulfate and the solvent was removed, followed by purification with silica gel column chromatography (400 mesh, 10 g, elution solvent: methanol:dichloromethane = 1: 99), thereby obtaining 21 mg of the intended compound.

· NMR (90MHz, $CDCl_3$):

7.4(5H,Ar), 4.9(1H,d,J=18Hz, N-$CH_2$[C-2]), 4.5(1H,d,J=18Hz,N-$CH_2$[C-2]), 4.2(1H,m,$C_8$-H), 4.1(2H,t,J=8Hz, O-$CH_2$), 2.7(3H,s), 2.4(3H,d,J=7Hz), 2.1(3H,d,J=7Hz CH$C\underline{H_3}$), 3.0 - 2.0(6H,m)

Example 12

6-(2-Chlorophenyl)-8,8-diethyl-3-(3-cyanopropoxycarbonyl)-11-methyl-2,3,4,5-tetrahydro-8H-pyrido[4',3':4,5]thieno [3,2-f][ 1,2,4]tri-azolo[4,3-a][1,4]diazepine

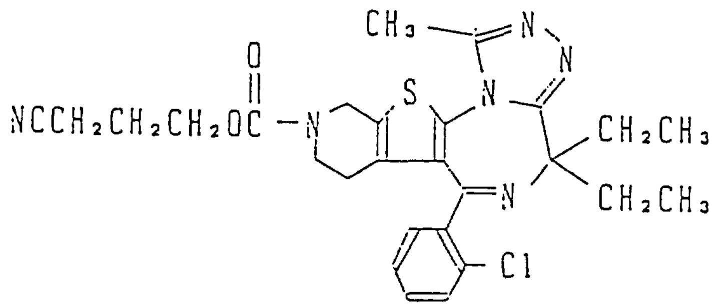

To a solution of 69 mg of 6-(2-chlorophenyl)-3-(3-cyanopropoxycarbonyl)-11-methyl-2,3,4,5-tetrahydro-8H-pyrido[4',3':4,5]thieno[3,2-f][1,2,4]triazolo-[4,3-a][1,4]diazepine dissolved in 2 ml of dimethylformamide at room temperature were added 10 mg of sodium hydride (55%) and 0.03 ml of methyl bromide, followed by agitation at at room temperature for 2 hours.

The thin layer chromatography revealed the presence of the starting compound and two novel products including a monoethyl product and a diethyl product. Accordingly, 10 mg of sodium hydride and 0.03 ml of ethyl bromide were further added and agitated for 2 hours, after which water was added to the solution in order to stop the reaction and the solution was neutralized with acetic acid. The solvent was distilled off under reduced pressure and the resultant residue was extracted with 10 ml of dichloromethylene and then with 20 ml of dichloromethylene. The solution was dried with magnesium sulfate and the solvent was removed, followed by purification with silica gel column chromatography (400 mesh, 13 g, elution solvent: methanol:dichloromethane = 1: 99), thereby obtaining 41 mg of the intended compound.

· NMR (90MHz, $CDCl_3$):

7.4(5H,Ar), 4.9(1H,d,J=18Hz, N-$CH_2$[C-2]), 4.5(1H,d,J=18Hz,N-$CH_2$[C-2]), 4.1(2H,t,J=8$H_2$,O-$CH_2$), 2.7(3H,s, $CH_3$), 2.0 - 2.7(10H,n), 1.3(6H,t,J=7Hz,$CH_2C\underline{H}_3$)

Example 13

3-(3-Butynyloxycarbonyl)-6-(2-chlorophenyl)-8,8-diethyl-11-methyl-2,3,4,5-tetrahydro-8H-pyrido[4',3':4,5]thieno[3,2-f] [1,2, 4]tri azolo[4,3-a][1,4]diazepine

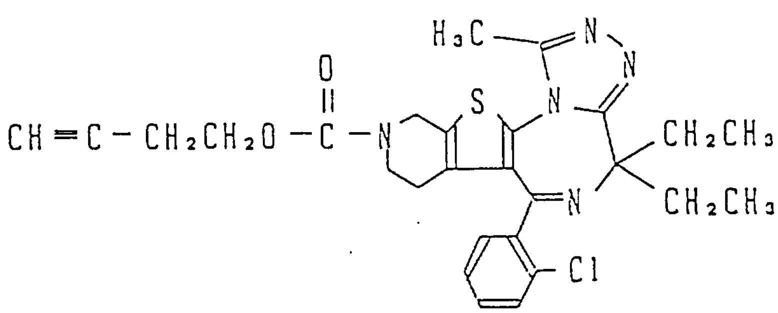

The general procedure of Example 11 was repeated using 65 mg of 3-(3-butynyloxycarbonyl)-6-(2-chlorophenyl)-11-methyl-2,3,4,5-tetrahydro-8H-pyrido[4',3':4,5]thieno[3,2-f][ 1,2,4]triazolo[4,3-a][1,4]diazepine, thereby obtaining 23 mg of the intended product.

· NMR (90MHz, $CDCl_3$):

7.4(5H,Ar), 4.9(1H,d,J=18Hz, N-$CH_2$[C-2]), 4.5(1H,d,J=18Hz,N-$CH_2$[C-2]), 4.1(2H,t,J=8$H_2$,O-$CH_2$), 2.7(3H,s, $CH_3$), 2.0 - 2.7(8H,n,$CH_2CH_3$ and C$\underline{H_2}$$CH_3$), 1.3(6H,t,J=7Hz,$CH_2$C$\underline{H_3}$)

Example 14

6-(2-Chlorophenyl)-3-(1-cyano-1-methylethoxycarbonyl)-8,8, 11-trimethyl-2,3,4,5-tetrahydro-8H-pyrido[4',3':4,5] thieno[3,2-f][1,2,4]triazolo[4,3-a][1,4]diazepine

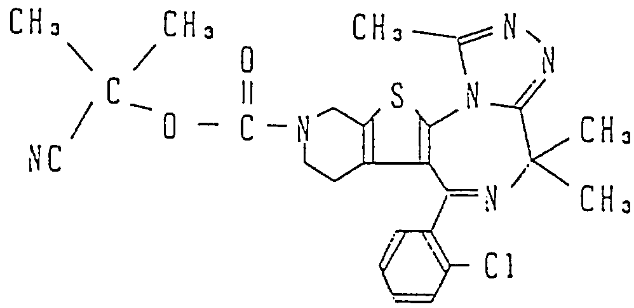

· $^1$H-NMR (90MHz, $CDCl_3$) δ :

1.8(s,6H), 2.8(s,3H), 3.1(s,3H), 3.0 - 3.9(m,4H), 3.8(s,3H), 4.4 - 4.9(m,2H), 7.4(m,4H)

Example 15

6-(2-Chlorophenyl)-3-cyclopropylmethylaminocarbonyl-11-methyl-2,3,4,5-tetrahydro-8H-pyrido[4',3':4,5]thieno[3,2-f] [1,2,4]triazolo[4,3-a][1,4]diazepine

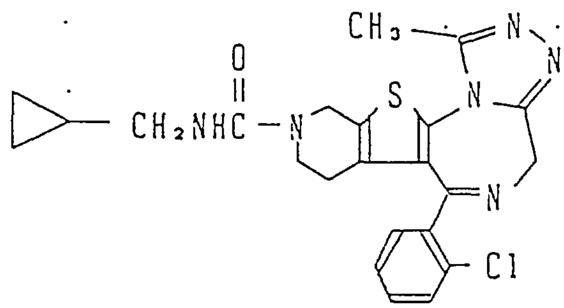

· $^1$H-NMR (90MHz, $CDCl_3$) δ :

0.04 - 0.32(m,2H), 0.36 - 0.60(m,2H), 0.70 - 1.16(m,1H), 1.52 - 2.17(m,2H), 2.66(s,3H), 2.84 - 5.85(m,7H), 3.04 (dd,J=6Hz,7Hz,2H), 7.32(m,4H)

· FABMS($M+H^+$) m/z:467

Example 16

6-(2-Chlorophenyl)-3-(1-ethynylcyclohexylaminocarbonyl)-11-methyl-2,3,4,5-tetrahydro-8H-pyrido[4',3':4,5]thieno[3,2-f] [1,2,4]triazolo[4,3-a][1,4]diazepine

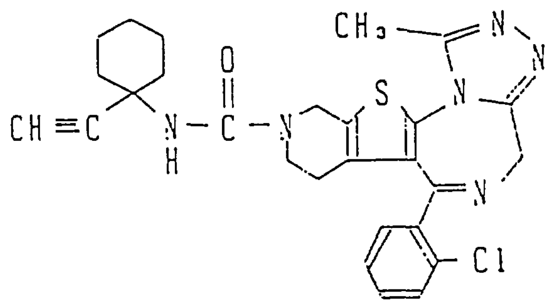

· $^{1}$H-NMR (90MHz, $CDCl_3$) δ :
1.12 - 2.24(m,12H), 2.37(s,1H), 2.80 - 5.76(m,7H), 7.29(m,4H)
· FABMS(M+H$^{+}$) m/z:519

Example 17

6-(2-Chlorophenyl)-3-(5-cyanopentylaminocarbonyl)-11-methyl-2,3,4,5-tetrahydro-8H-pyrido[4',3':4,5]thieno[3,2-f] [1,2,4]triazolo[4,3-a][1,4]diazepine

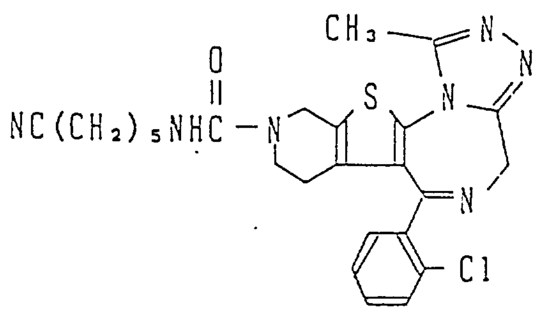

· $^{1}$H-NMR (90MHz, $CDCl_3$) δ :
1.28 - 2.16(m,8H), 2.32(t,J=7Hz,2H), 2.81 - 5.68(m,9H), 7.29(m,4H)
· FABMS(M+H$^{+}$) m/z: 508

Example 18

6-(2-Chlorophenyl)-3-(4-cyanophenylaminocarbonyl)-11-methyl-2,3,4,5-tetrahydro-8H-pyrido[4',3':4,5]thieno[3,2-f][1,2,4]triazolo[4,3-a][1,4]diazepine

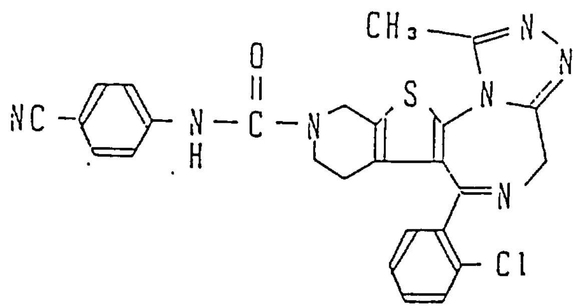

$^1$H-NMR (90MHz, $CDCl_3$) δ :
1.55 - 2.18(m,2H), 2.67(s,3H), 3.80 - 5.70(m,6H), 7.32(m,4H), 7.47(m,4H), 8.40(br,s,1H)
· FABMS(M+H$^+$) m/z: 514

Example 19

6-(2-Chlorophenyl)-3-(3-cyanopentylaminocarbonyl)-11-methyl-2,3,4,5-tetrahydro-8H-pyrido[4',3':4,5]thieno[3,2-f][1,2,4]triazolo[4,3-a][1,4]diazepine

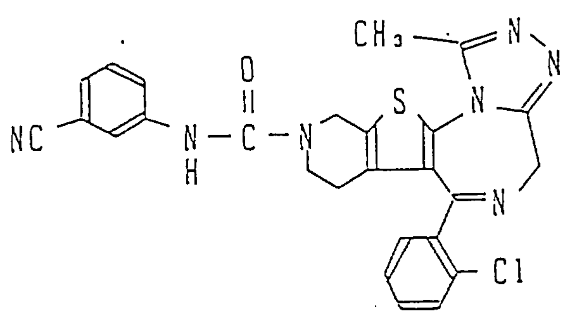

· $^1$H-NMR (90MHz, $CDCl_3$) δ :
1.43 - 2.17(m,2H), 2.63(s,3H), 3.04 - 5.68(m,6H), 7.05-7.72(m,8H)
· FABMS(M+H$^+$) m/z: 514

Example 20

6-(2-Chlorophenyl)-3-(3-ethynylphenylaminocarbonyl)-11-methyl-2,3,4,5-tetrahydro-8H-pyrido[4',3':4,5]thieno[3,2-f] [1,2,4]triazolo[4,3-a][1,4]diazepine

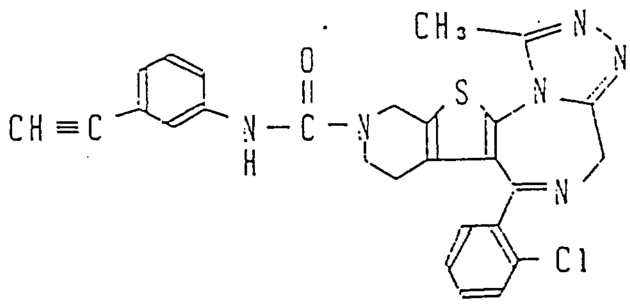

· $^1$H-NMR (90MHz, $CDCl_3$) δ :
1.40 - 2.40(m,2H), 2.66(s,3H), 3.01(s,1H), 3.05-5.08(m,6H), 6.64(br.s,1H), 6.88 - 7.48(m,8H)
· FABMS($M+H^+$) m/z: 513

Example 21

6-(2-chlorophenyl)-11-methyl-3-(morpholin-4-yl)carbonyl-2,3,4,5-tetrahydro-8H-pyrido[4',3':4,5]thieno[3,2-f] [1,2,4]triazolo[4,3-a][1,4]diazepine

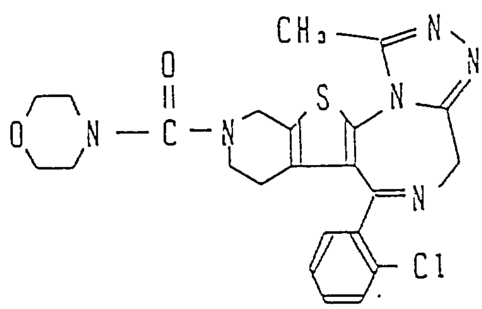

· $^1$H-NMR (90MHz, $CDCl_3$) δ :
1.32 - 2.44(m,2H), 2.66(s,3H), 2.92 - 5.80(m,6H), 3.21(t,J=6Hz,4H), 3.63(t,J=6Hz,4H), 7.29(m,4H)
· FABMS($M+H^+$) m/z: 483

Example 22

6-(2-Chlorophenyl)-3-(1-ethynylcyclopentylaminocarbonyl)-11-methyl-2,3,4,5-tetrahydro-8H-pyrido[4',3':4,5]thieno[3, 2-f][1,2,4]triazolo[4,3-a][1,4]diazepine

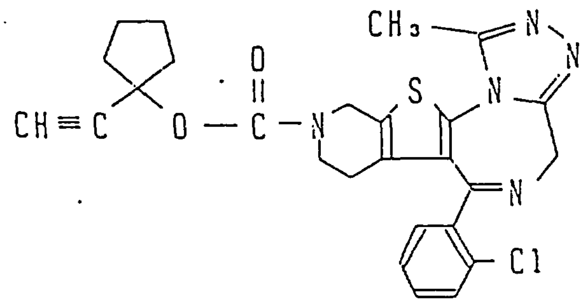

· $^1$H-NMR (90MHz, $CDCl_3$) δ :
1.40 - 2.44(m,10H), 2.56(s,1H), 2.67(s,3H), 2.90-5.80(m,6H), 7.29(m,4H)
· FABMS(M+H$^+$) m/z: 506

Example 23

6-(2-Chlorophenyl)-11-methyl-3-(phenylethylcarbonyl)-2,3,4, 5-tetrahydro-8H-pyrido[4',3':4,5]thieno[3, 2-f][1,2,4] triazolo[4,3-a][1,4]diazepine

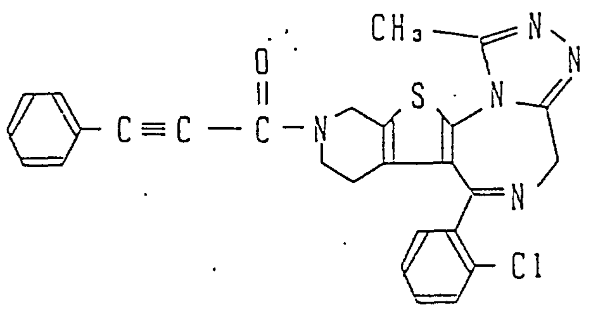

· $^1$H-NMR (90MHz, $CDCl_3$) δ :
1.67 - 2.40(m,2H), 2.68(s,3H), 3.04 - 5.80(m,6H), 7.12-7.60(m,9H)
· FABMS(M+H$^+$) m/z: 498

Example 24

6-(2-Chlorophenyl)-3-(1,1-dimethyl-2-propynyloxycarbonyl)-11-methyl-2,3,4,5-tetrahydro-8H-pyrido[4',3':4,5]thieno [3,2-f][1,2,4]triazolo[4,3-a] [1,4]diazepine

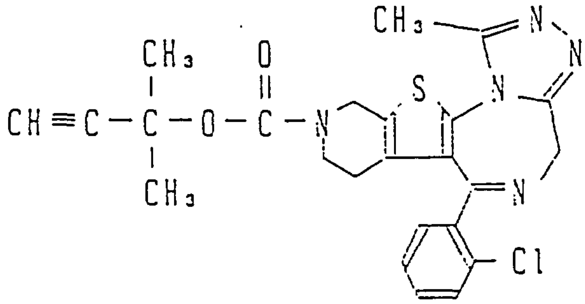

· $^1$H-NMR(90 MHz, $CDCl_3$) $\delta$ :
1.40 - 2.18(m,2H), 1.68(s,6H), 2.53(s,1H), 2.67(s,3H), 2.90 - 5.76(m,6H), 7.30(m,4H)
· FABMS ($M+H^+$) m/z:480

Example 25

6-(2-Chlorophenyl)-11-methyl-3-(1-methyl-2-propynyloxycarbonyl)-2,3,4,5-tetrahydro-8H-pyrido[4',3':4,5]thieno[3,2-f] [1,2,4]triazolo[4,3-a] [1,4]diazepine

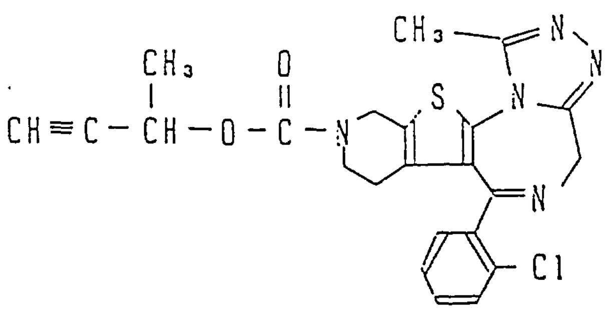

· $^1$H-NMR(90 MHz, $CDCl_3$) $\delta$ :
1.38 - 2.32(m,2H), 1.50(d,J=7Hz,3H), 2.45(d,J=2Hz,1H), 2.66(s,3H), 2.88 - 5.70(m,6H), 5.34(dq,J=2Hz,7Hz,1H), 7.29(m,4H)
· FABMS ($M+H^+$) m/z:466

Example 26

6-(2-Chlorophenyl)-11-methyl-3-(1-methyl-3-butynyloxycarbonyl)-2,3,4,5-tetrahydro-8H-pyrido[4',3':4,5]thieno[3,2-f][1,2,4]triazolo[4,3-a] [1,4]diazepine

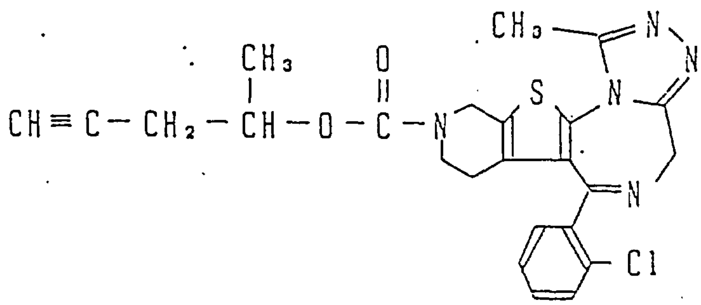

· $^1$H-NMR(90 MHz, $CDCl_3$) δ :

1.30(d,J=7Hz,3H), 1.50 - 2.60(m,5H), 2.66(s,3H), 2.88-5.72(m,7H), 7.29(m,4H)

· FABMS ($M+H^+$) m/z:480

Example 27

6-(2-Chlorophenyl)-11-methyl-3-(2-pentyloxycarbonyl)-2,3,4,5-tetrahydro-8H-pyrido[4',3':4,5]thieno[3,2-f][1,2,4]triazolo[4,3-a] [1,4]diazepine

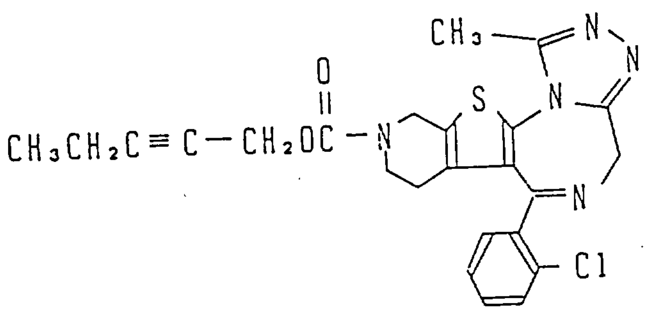

· $^1$H-NMR(90 MHz, $CDCl_3$) δ :

1.13(d,J=7Hz,3H), 1.54 - 2.36(m,2H), 2.25(tq,J=2Hz, 7Hz, 2H), 2.67(s,3H), 2.84 - 5.76 (m,6H), 4.66(t,J=2Hz, 2H), 7.30(m,4H)

· FABMS ($M+H^+$) m/z:480

Example 28

6-(2-Chlorophenyl)-11-methyl-3-(3-pentynyloxycarbonyl)-2,3,4,5-tetrahydro-8H-pyrido[4',3':4,5]thieno[3,2-f][1,2,4]triazolo[4,3-a] [1,4]diazepine

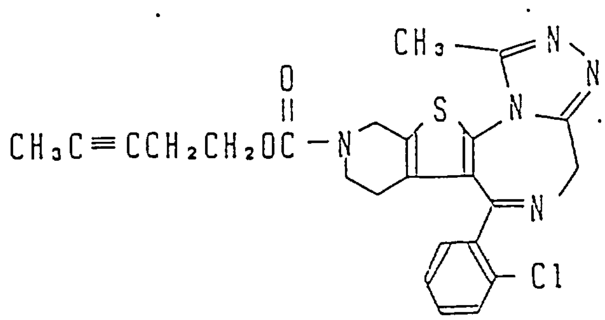

· $^1$H-NMR(90 MHz, $CDCl_3$) δ :
1.50 - 2.20(m,2H), 1.74(t,J=2Hz,3H), 2.42(m,2H), 2.66(s,3H), 2.90 - 5.70(m,6H), 4.11(t,J=7Hz,2H), 7.30(m,4H)
· FABMS ($M+H^+$) m/z:480

Example 29

6-(2-Chlorophenyl)-3-[2-(imidazol-1-yl)ethoxycarbonyl]-11-methyl-2,3,4,5-tetrahydro-8H-pyrido[4',3':4,5]thieno [3,2-f] [1,2,4]triazolo[4,3-a][1,4]diazepine

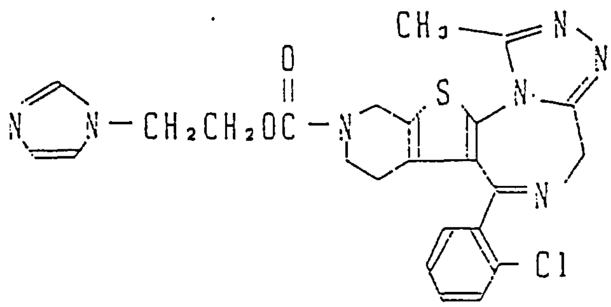

· $^1$H-NMR(90 MHz, $CDCl_3$) δ :
1.50 - 2.19(m,2H), 2.66(s,3H), 2.80 - 5.80(m,10H), 6.70-6.92(m,1H), 6.92 - 7.06(m,1H), 7.12 - 7.54(m, 1H), 7.30 (m,4H)
· FABMS ($M+H^+$) m/z:508

Example 30

6-(2-Chlorophenyl)-11-methyl-3-(2,3,5,6-tetrahydropyran-4-yl)oxycarbonyl-2,3,4,5-tetrahydro-8H-pyrido[4',3':4,5] thieno[3,2-f][1,2,4]triazolo[4,3-a] [1,4]diazepine

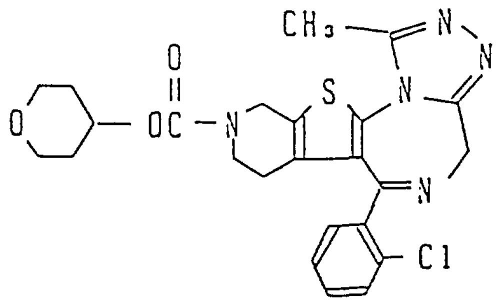

· $^1$H-NMR(90 MHz, $CDCl_3$) δ :
1.40 - 2.12(m,6H), 2.67(s,3H), 2.84 - 5.68(m,11H), 7.29(m,4H)
· FABMS ($M+H^+$) m/z:498

Example 31

6-(2-Chlorophenyl)-3-(5-hexynyloxycarbonyl)-11-methyl-2,3,4,5-tetrahydro-8H-pyrido[4',3':4,5] thieno[3,2-f][1,2,4] triazolo[4,3-a][1,4]diazepine

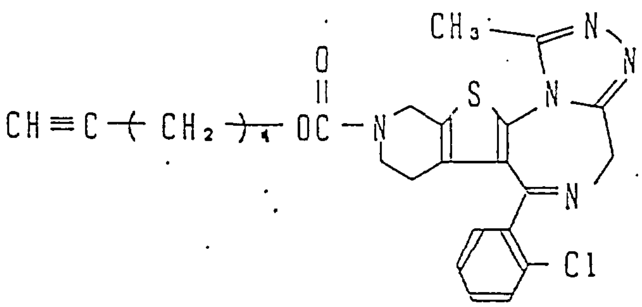

· $^1$H-NMR(90 MHz, $CDCl_3$) δ :
1.20 - 2.32(m,6H), 1.94(t,J=2Hz,1H), 2.21(dt,J=2Hz,7Hz,2H), 2.66(s,3H), 2.84 - 5.76(m,6H), 7.28(m,4H)
· FABMS ($M+H^+$) m/z:494

Example 32

6-(2-Chlorophenyl)-3-(3-cyano-1-oxopropyl)-11-methyl-2,3,4,5-tetrahydro-8H-pyrido[4',3':4,5] thieno[3,2-f][1,2,4] triazolo[4,3-a][1,4]diazepine

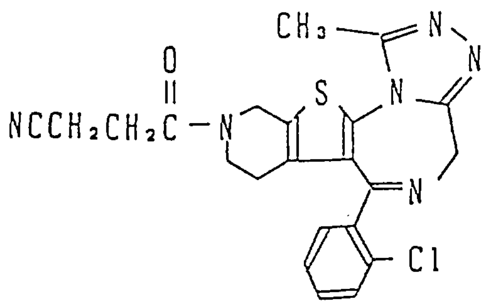

· $^1$H-NMR(90 MHz, $CDCl_3$) $\delta$ :
1.16 - 2.38(m,2H), 2.42 -2.79(m,4H), 2.67(s,3H), 3.20-5.72(m,6H), 7.31(m,4H)
· FABMS ($M+H^+$) m/z:451

Example 33

6-(2-Chlorophenyl)-3-(2-cyano-1-oxoethyl)-11-methyl-2,3,4,5-tetrahydro-8H-pyrido[4',3':4,5] thieno[3,2-f][1,2,4] triazolo[4,3-a][1,4]diazepine

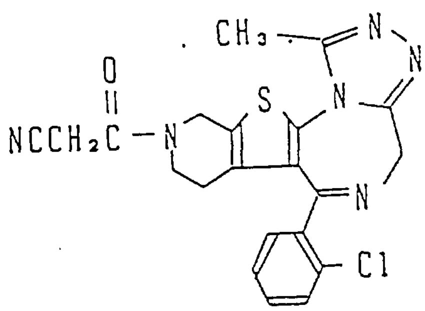

· $^1$H-NMR(90 MHz, $CDCl_3$) $\delta$ :
1.60 - 2.35(m,2H), 2.66(s,3H), 3.20 - 5.76(m,8H), 7.30(m,4H)
· FABMS ($M+H^+$) m/z:437

Example 34

6-(2-Chlorophenyl)-3-(3-cyanopropoxycarbonyl)-11-methyl-2,3,4,5-tetrahydro-8H-pyrido[4',3':4,5] thieno[3,2-f][1,2,4] triazolo[4,3-a][1,4]diazepine

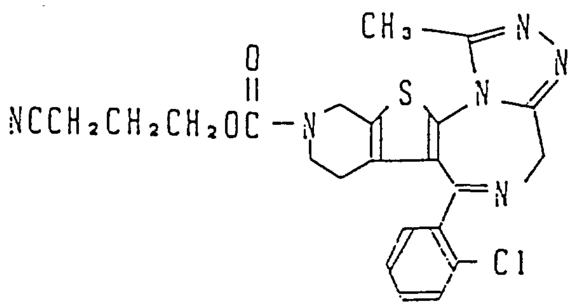

· $^1$H-NMR(90 MHz, $CDCl_3$) δ :

1.41 - 1.80(m,2H), 1.80 - 2.17(m,2H), 2.22 - 2.52(m,2H), 2.66(s,3H), 2.86 - 5.76(m,6H), 4.20(t,t=7Hz,2H), 7.30 (m,4H)

· FABMS (M+H$^+$) m/z:481

Example 35

6-(2-Chlorophenyl)-11-methyl-3-(1-oxo-4-pentyl)-2,3,4,5-tetrahydro-8H-pyrido[4',3':4,5] thieno[3,2-f][1,2,4]triazolo [4,3-a][1,4]diazepine

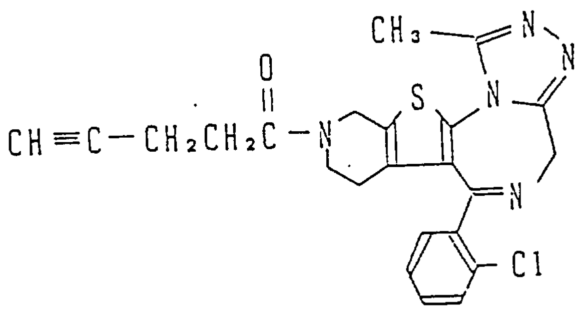

· $^1$H-NMR(90 MHz, $CDCl_3$) δ :

1.54 - 2.18(m,2H), 1.96(m,1H), 2.44 - 2.65(m,4H), 2.71(s,3H), 2.88 - 5.76(m,6H), 7.42(m,4H)

· FABMS (M+H$^+$) m/z:450

Example 36

6-(2-Chlorophenyl)-11-methyl-3-(1-propargylpiperidin-4-yl)oxycarbonyl-2,3,4,5-tetrahydro-8H-pyrido[4',3':4,5]thieno [3,2-f][1,2,4]triazolo[4,3-a][1, 4]diazepine

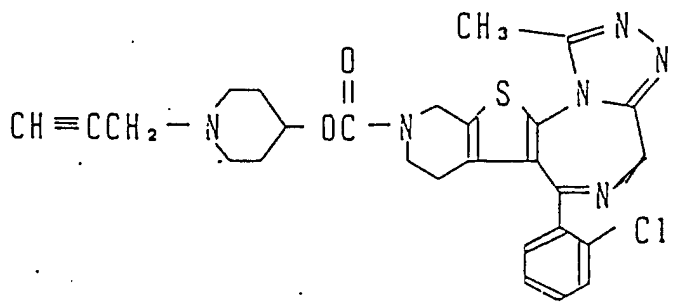

· $^1$H-NMR(90 MHz, $CDCl_3$) $\delta$ :
1.4 - 2.9(m,11H), 2.25(t,1H), 2.7(s,3H), 3.3(d,2H), 3.0-3.9(m,2H), 4.0 - 4.9(m,1+2H), 5.4 - 5.8(m,1H), 7.4(m,4H)
· MS m/z(Pos. FD):535

Example 37

6-(2-Chlorophenyl)-3-cyclohexyloxycarbonyl-11-methyl-2,3,4,5-tetrahydro-8H-pyrido[4',3':4,5] thieno[3,2-f][1,2,4] triazolo[4,3-a][1,4]diazepine

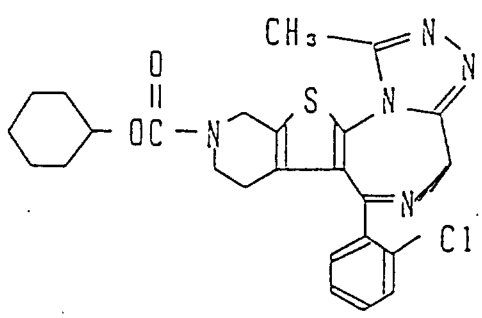

· $^1$H-NMR(90 MHz, $CDCl_3$) $\delta$ :
1.2 - 2.3(m,12H), 2.7(s,3H), 3.0 - 4.0(m,2H), 4.0-4.8(m,1+1+2H), 5.4 - 5.8(m,1H), 7.4(m,4H)
· MS m/z(Pos. FAB):496

Example 38

6-(2-Chlorophenyl)-3-cyclohexylethoxycarbonyl-11-methyl-2,3,4,5-tetrahydro-8H-pyrido[4',3':4,5] thieno[3,2-f][1,2,4] triazolo[4,3-a][1,4]diazepine

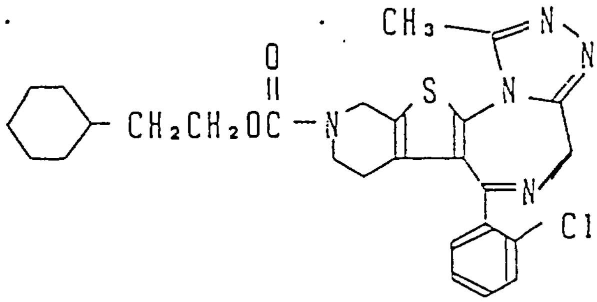

· $^1$H-NMR(90 MHz, $CDCl_3$) δ :
0.6 - 2.7(m,17H), 2.7(s,3H), 3.0 - 4.0(m,2H), 4.0-4.4(m,1+2H), 4.4 - 4.8(m,2H), 5.4 - 5.8(m,1H), 7.4(m,4H)
· MS m/z (Pos. FAB):538

Example 39

6-(2-Chlorophenyl)-3-cyclopropylmethoxycarbonyl-11-methyl-2,3,4,5-tetrahydro-8H-pyrido[4',3':4,5] thieno[3,2-f] [1,2,4]triazolo[4,3-a][1,4]diazepine

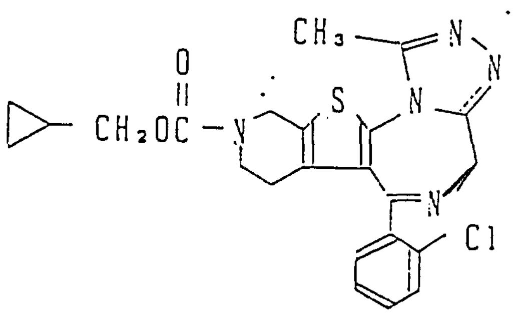

· $^1$H-NMR(90 MHz, $CDCl_3$) δ :
0.2 - 2.0(m,7H), 2.7(s,3H), 3.0 - 4.0(m,2H), 3.8-4.0(d,2H), 4.4 - 4.8(m,1+2H), 5.4 - 5.8(m,1H), 7.4(m,4H)
· MS m/z(Pos. FAB):468

Example 40

6-(2-Chlorophenyl)-3-cyclohexylmethoxycarbonyl-11-methyl-2,3,4,5-tetrahydro-8H-pyrido[4',3':4,5] thieno[3,2-f] [1,2,4]triazolo[4,3-a][1,4]diazepine

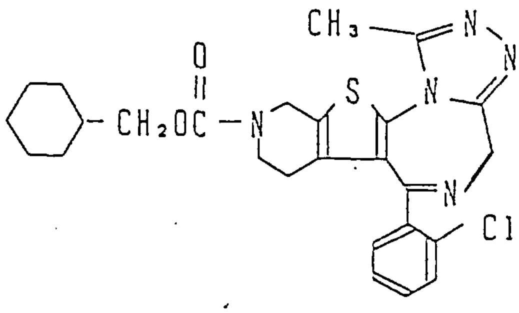

· $^1$H-NMR(90 MHz, $CDCl_3$) δ :
0.8 - 2.2(m,13H), 2.7(s,3H), 3.0 - 4.0(m,2H), 3.7-4.0(d,2H), 4.1 - 4.8(m,1+2H), 5.4 - 5.8(m,1H), 7.4(m,4H)
· MS m/z(Pos. FAB):510

Example 41

6-(2-Chlorophenyl)-11-methyl-3-(4-pyridylcarbonyl)-2,3,4,5-tetrahydro-8H-pyrido[4',3':4,5] thieno[3,2-f][1,2,4]triazolo [4,3-a][1,4]diazepine

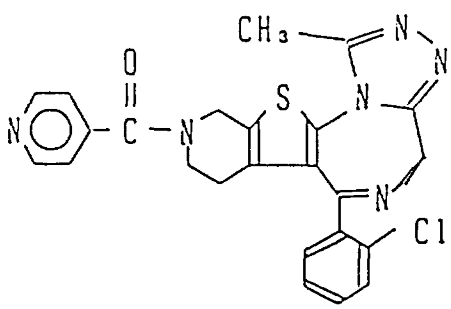

· $^1$H-NMR(90 MHz, $CDCl_3$) δ :
1.4 - 2.5(m,2H), 2.67(s,3H), 3.1 - 3.75(m,2H), 3.9-5.8(m,4H), 7.0 - 7.7(m,6H), 8.4 - 8.8(m,2H)
· MS m/z(Pos. FAB):475

Example 42

6-(2-Chlorophenyl)-3-cyclohexylmethylcarbonyl-11-methyl-2,3,4,5-tetrahydro-8H-pyrido[4',3':4,5] thieno[3,2-f][1,2,4] triazolo[4,3-a][1,4]diazepine

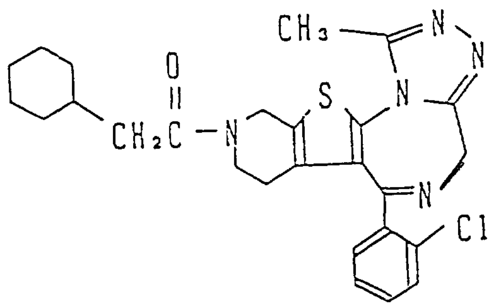

· $^{1}$H-NMR(90 MHz, $CDCl_3$) δ :
0.6 - 2.4(m,13H), 2.16(d, J=6.5Hz, 2H), 2.67(s,3H), 2.8-5.9(m,6H), 7.1 - 7.6(m,4H)
· MS m/z(Pos. Fab):494$(M+H)^+$

Example 43

6-(2-Chlorophenyl)-3-cyclohexanecarbonyl-11-methyl-2,3,4,5-tetrahydro-8H-pyrido[4',3':4,5] thieno[3,2-f][1,2,4] triazolo[4,3-a][1,4]diazepine

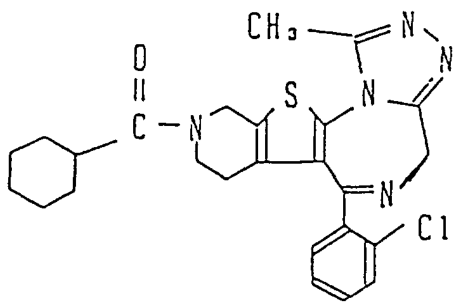

· $^{1}$H-NMR(90 MHz, $CDCl_3$) δ :
0.7 - 2.7(m,13H), 2.67(s,3H), 2.8 -5.8(m,6H), 7.1-7.6(m,4H)
· MS m/z(Pos. FAB):480$(M+H)^+$

Example 44

6-(2-Chlorophenyl)-11-methyl-3-(3-pyridylcarbonyl)-2,3,4,5-tetrahydro-8H-pyrido[4',3':4,5] thieno[3,2-f][1,2,4]triazolo [4,3-a][1,4]diazepine

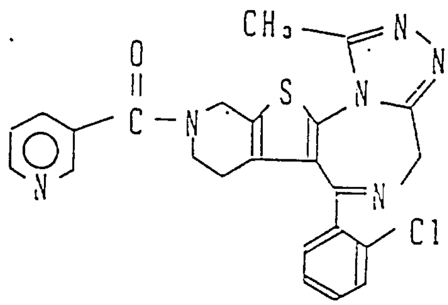

· $^1$H-NMR(90 MHz, $CDCl_3$) δ :
1.4 - 2.6(m,2H), 2.66(s,3H), 2.8 - 6.0(m,6H), 7.1-8.0(m,6H), 8.4 - 8.8(m,2H)
· MS m/z(Pos. FAB):475$(M+H)^+$

Example 45

3-[4'-(morpholin-4-yl-sulfonyl)phenylaminocarbonyl]-6-(2-chlorophenyl)-11-methyl-2,3,4,5-tetrahydro-8H-pyrido[4',3':4,5]thieno[3,2-f][1,2,4]triazolo[4,3-a] [1,4]diazepine

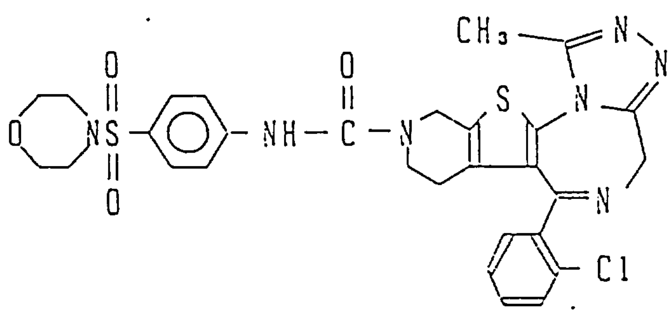

· $^1$H-NMR(90 MHz, $CDCl_3$) δ :
1.80 - 2.20(m,4H), 2.63(s,3H), 2.80 - 3.08(m,4H), 3.24-3.90(m,9H), 4.60 - 4.90(m,2H), 7.20 - 7.42(m,4H), 7.45-7.68(m,4H), 7.75(brs.1H)
· MS:m/z 638

Example 46

3-[4'-(N-piperidinosulfonyl)phenylaminocarbonyl]-6-(2-chlorophenyl)-11-methyl-2,3,4,5-tetrahydro-8H-pyrido[4',3':4,5]thieno[3,2-f][1,2,4]triazolo[4,3-a] [1,4]diazepine

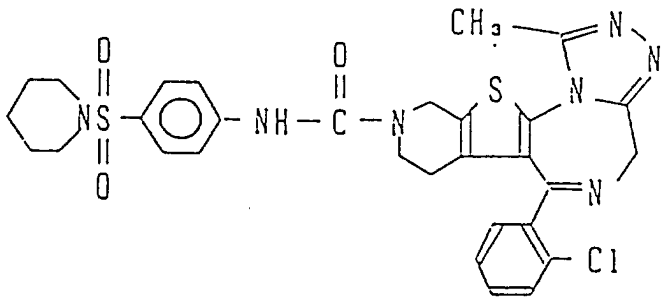

· $^1$H-NMR(90 MHz, $CDCl_3$) δ :

1.10 - 2.30(m,10H), 2.64(s,3H), 2.75 - 3.10(m,4H), 3.30-4.30(m,2H), 4.60 - 4.92(m,2H), 7.15 - 7.40(m,4H), 7.42-7.60(m,4H), 7.72(brs.1H)

· MS:m/z 636

Example 47

3-[4'-(N-imidazoylsulfonyl)phenylaminocarbonyl]-6-(2-chlorophenyl)-11-methyl-2,3,4,5-tetrahydro-8H-pyrido[4',3':4,5]thieno[3,2-f][1,2,4]triazolo[4,3-a] [1,4]diazepine

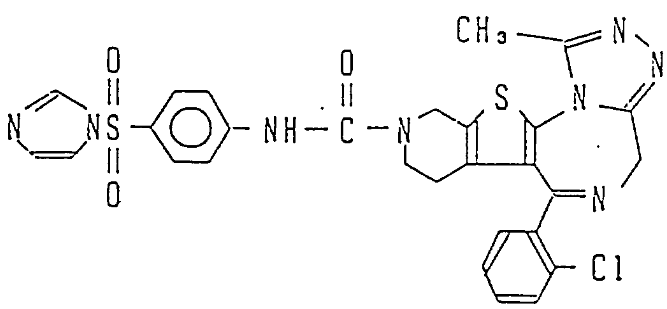

· $^1$H-NMR(90 MHz, $CDCl_3$) δ :

1.60 - 2.40(m,4H), 2.70(s,3H), 3.75 - 4.30(m,2H), 4.40-5.00(m,2H), 7.00 - 7.50(m,12H)

· MS:m/z 619

Example 48

3-[4'-(sulfamoyl)phenylaminocarbonyl]-6-(2-chlorophenyl)-11-methyl-2,3,4,5-tetrahydro-8H-pyrido[4',3':4,5]thieno [3,2-f][1,2,4]triazolo[4,3-a] [1,4]diazepine

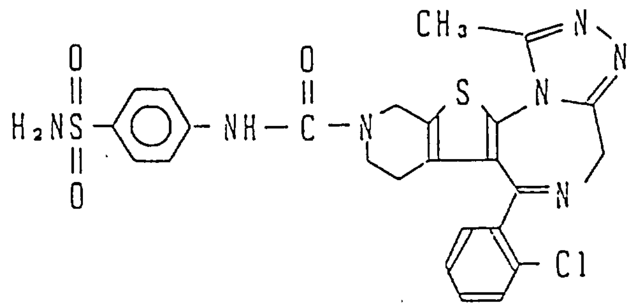

· $^1$H-NMR(90 MHz, $CDCl_3$) δ :

1.40 - 2.20(m,4H), 2.62(s,3H), 3.60 - 4.40(m,2H), 5.10-5.50(m,2H), 7,10(s,2H), 7.30 - 7.60(m,4H), 7.62(ABq,4H, J=9.0Hz), 8.95(s,1H)

· MS:m/z 568

Epample 49

3-[4'-(N,N'-diethylaminosulfonyl)phenylaminocarbonyl]-6-(2-chlorophenyl)-11-methyl-2,3,4,5-tetrahydro-8H-pyrido[4', 3':4,5]thieno[3,2-f][1,2,4]triazolo[4,3-a] [1,4]diazepine

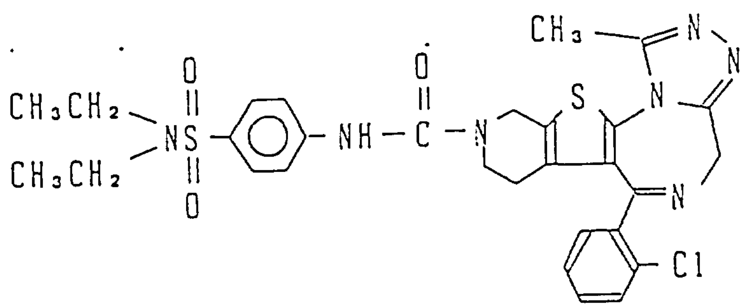

· $^1$H-NMR(90 MHz, $CDCl_3$) δ :

1.10(t, 6h,J=7.2H), 1.70 - 2.30(m,4H), 2.62(s,3H), 3.16(q,4H,J=7.2Hz), 3.40 - 4.20(m,2H), 3.55 - 3.60(m,2H), 7.18 - 7.40(m,4H), 7.54(ABq,4H,J=9.OHz), 7.70(s,1H)

· MS:m/z 624

Example 50

3-[4'-(N-cyclohexylaminosulfonyl)phenylaminocarbonyl]-6-(2-chlorophenyl)-11-methyl-2,3,4,5-tetrahydro-8H-pyrido [4',3':4,5]thieno[3,2-f][1,2,4]triazolo[4,3-a] [1,4]diazepine

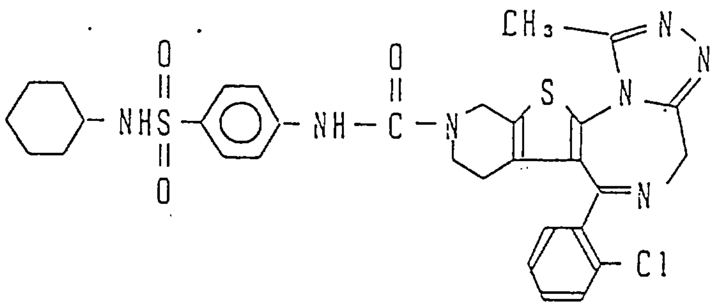

· $^1$H-NMR(90 MHz, $CDCl_3$) $\delta$ :

0.80 - 2.20(m,14H), 2.64(s,3H), 2.80 - 4.30(m,3H), 4.60-4.30(m,3H), 4.60 - 4.90(m,2H), 5.05(d,1H,J=7.2Hz), 7.20-7.40(m,4H), 7.56(ABq,4H,J=9.OHz), 7.76(s,1H)

· MS:m/z 650

Example 51

3-[4'-(2"-pyridylmethylaminosulfonyl) phenylaminocarbonyl]-6-(2-chlorophenyl)-11-methyl-2,3,4,5-tetrahydro-8H-pyrido[4',3':4,5]thieno[3,2-f][1,2,4]triazolo[4,3-a] [1,4]diazepine

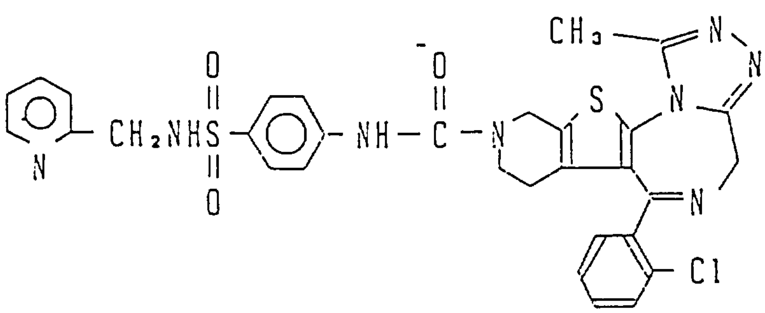

· $^1$H-NMR(90 MHz, $CDCl_3$) $\delta$ :

1.60 - 2.20(m,4H), 2.72(s,3H), 3.60 - 4.50(m,6H), 6.50-6.72(m,2H), 7.00 - 7.70(m,11H), 8.11 - 8.28(m,1H)

· MS m/z:659

Example 52

3-[3-(N-piperidinosulfonyl)-propyloxycarbonyl]-6-(2-chlorophenyl)-11-methyl-2,3,4,5-tetrahydro-8H-pyrido[4',3':4,5] thieno[3,2-f][1,2,4]triazolo[4,3-a] [1,4]diazepine

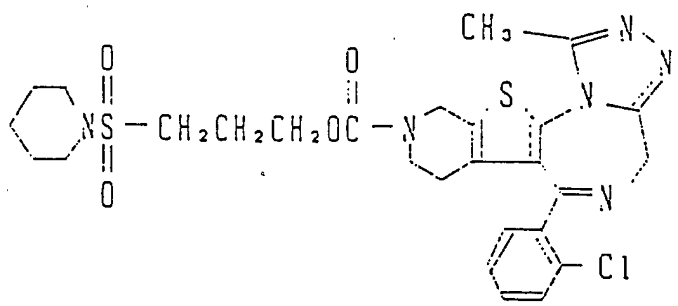

· $^1$H-NMR(90 MHz, $CDCl_3$) δ :

1.40 - 2.30(m,12H), 2.70(s,3H), 2.95(t,2H,J=7,2Hz), 3.10 - 3.40(m,4H), 3.50 - 4.20(m,2H), 4.22(t,2H,J=7.2Hz), 4.42 - 4.82(m,2H), 7.20 - 7.50(m,4H)

· MS:m/z 603

Example 53

3-[3-(N-morpholinosulfonyl)-propyloxycarbonyl]-6-(2-chlorophenyl)-11-methyl-2,3,4,5-tetrahydro-8H-pyrido[4',3':4,5] thieno[3,2-f][1,2,4]triazolo[4,3-a] [1,4]diazepine

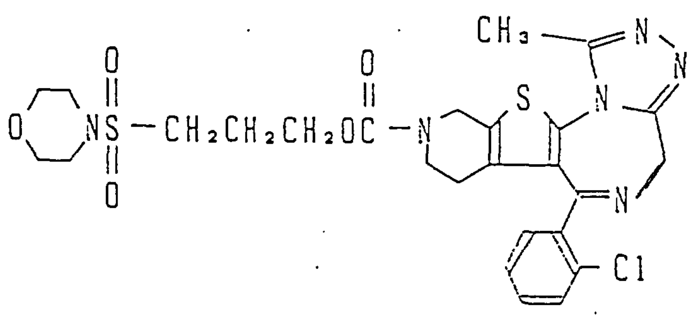

· $^1$H-NMR(90 MHz, $CDCl_3$) δ :

1.60 - 2.40(m,6H), 2.70(s,3H), 2.98(t,2H,J=7.2Hz), 3.60-3.90(m,4H), 2.80 - 4.40(m,2H), 4.22(t,2H,J=7.2Hz), 4.40-4.84(m,2H), 7.20 - 7.50(m,4H)

· MS:m/z 605

Example 54

3-[4'-(N-morpholinosulfonyl)phenyloxycarbonyl]-6-(2-chlorophenyl)-11-methyl-2,3,4,5-tetrahydro 8H-pyrido[4',3':4,5] thieno[3,2-f][1,2,4]triazolo[4,3-a] [1,4]diazepine

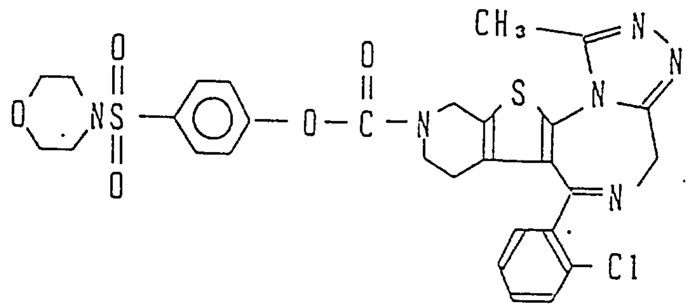

· $^1$H-NMR(90 MHz, $CDCl_3$) δ :
1.60 - 2.40(m,4H), 2.74(s,3H), 2.80 - 3.10(m,4H), 3.60-3.80(m,4H), 3.10 - 5.10(m,4H), 4.90 - 7.60(m,8H)
· MS:m/z 639

Example 55

6-(2-Chlorophenyl)-3-(2-cyanoethylmethylamino)carbonyl-11-methyl-2,3,4,5-tetrahydro-8H-pyrido[4',3':4,5] thieno [3,2-f][1,2,4]triazolo[4,3-a][1,4]diazepine

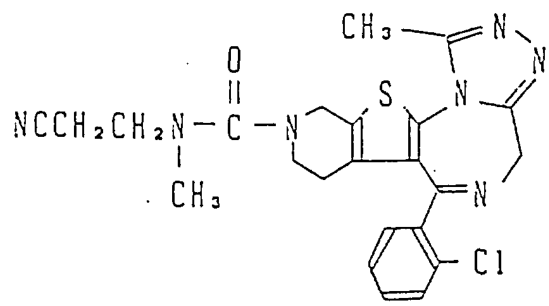

· $^1$H-NMR(90 MHz, $CDCl_3$) δ :
1.57 - 2.22(m,2H), 2.61(t,J=7Hz,2H), 2.67(s,3H), 2.94(s,3H), 3.00 - 5.80(m,6H), 3.43(t,J=7Hz,2H), 7.31(m,4H)
· FABMS(M+H$^+$) m/z: 480

Example 56

6-(2-Chlorophenyl)-3-(1-ethynyl-1-cyclohexyloxy-1 carbonyl-11-methyl-2,3,4,5-tetrahydro-8H-pyrido[4',3':4,5]thieno [3,2-f][1,2,4]-triazolo[4,3-a][1,4]diazepine

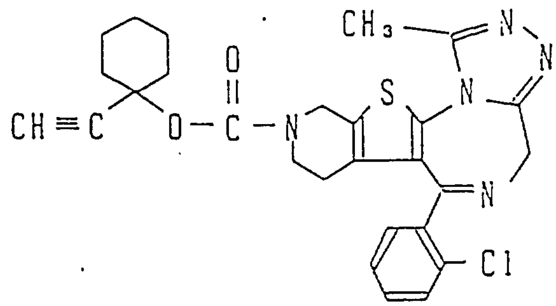

· $^1$H-NMR(90 MHz, $CDCl_3$) $\delta$ :
1.04 - 2.35(m,12H), 2.59(s,1H), 2.67(s,3H), 2.78-5.78(m,6H), 7.30(m,4H)
· FABMS(M+H$^+$) m/z: 520

Example 57

6-(2-Chlorophenyl)-11-methyl-3-(1-phenyl-2-propynyloxycarbonyl)-2,3,4,5-tetrahydro-8H-pyrido[4',3':4,5] thieno [3,2-f][1,2,4]triazolo[4,3-a][1,4]diazepine

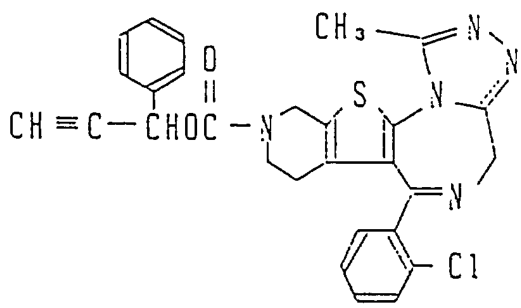

· $^1$H-NMR(90 MHz, $CDCl_3$) $\delta$ :
1.63 - 2.30(m,2H), 2.57(d,J=2Hz,1H), 2.65(s,3H), 2.97-5.71(m,6H), 6.35(d,J=2Hz,1H), 7.11 - 7.64(m,9H)
· FABMS(M+H$^+$) m/z: 528

Example 58

6-(2-Chlorophenyl)-3-(2-cyanoethoxy)carbonyl-11-methyl-2,3,4,5-tetrahydro-8H-pyrido[4',3':4,5] thieno[3,2-f][1,2,4] triazolo[4,3-a][1,4]diazepine

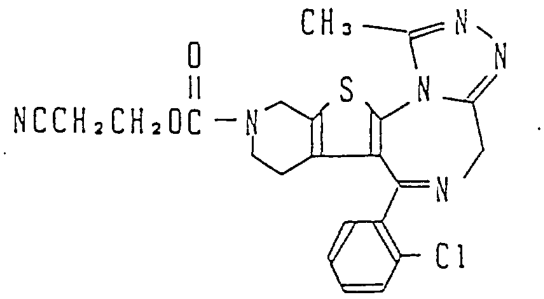

· $^1$H-NMR(90 MHz, $CDCl_3$) $\delta$ :
1.40 - 2.34(m,2H), 2.66(s,3H), 2.70(t,J=7Hz,2H), 2.79-5.76(m,6H), 4.28(t,J=7Hz,2H), 7.30(m,4H)
· FABMS(M+H$^+$) m/z: 467

Example 59

6-(2-Chlorophenyl)-11-methyl-3-(2-propynyl)-aminocarbonyl-2,3,4,5-tetrahydro-8H-pyrido[4',3':4,5] thieno[3,2-f] [1,2,4]triazolo[4,3-a][1,4]diazepine

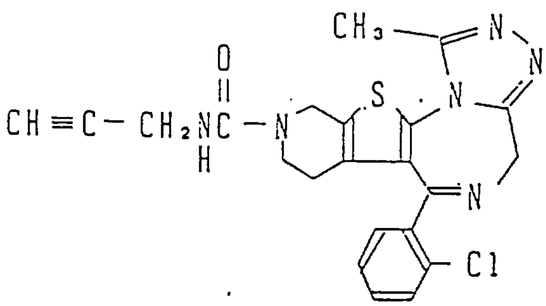

· $^1$H-NMR(90 MHz, $CDCl_3$) $\delta$ :
1.56 - 2.08(m,2H), 2.19(d,J=2Hz,1H), 2.65(s,3H), 2.96-5.70(dd,J=2Hz,7Hz,6H), 3.98(dd,J=2Hz,7Hz,2H), 4.83(t, J=7Hz,1H), 7.28(m,4H))
· FABMS(M+H$^+$) m/z: 451

Example 60

3-(2-Butynyloxycarbonyl)-6-(2-chlorophenyl)-11-methyl-2,3,4,5-tetrahydro-8H-pyrido[4',3':4,5] thieno[3,2-f][1,2,4] triazolo[4,3-a][1,4]diazepine

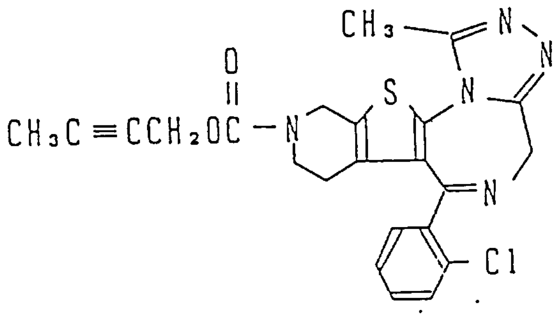

· $^{1}$H-NMR(90 MHz, $CDCl_3$) $\delta$ :
1.43 - 2.15(m,2H), 1.84(t,J=2Hz,3H), 2.66(s.3H), 2.80-5.74(m,6H), 4.64(q,J=2Hz,2H), 7.30(m,4H)
· FABMS($M+H^+$) m/z: 466

Example 61

6-(2-Chlorophenyl)-11-methyl-3-[2-(2-pyridyl)ethylamino carbonyl)]-2,3,4,5-tetrahydro-8H-pyrido[4',3':4,5] thieno [3,2-f][1,2,4]triazolo[4,3-a][1,4]diazepine

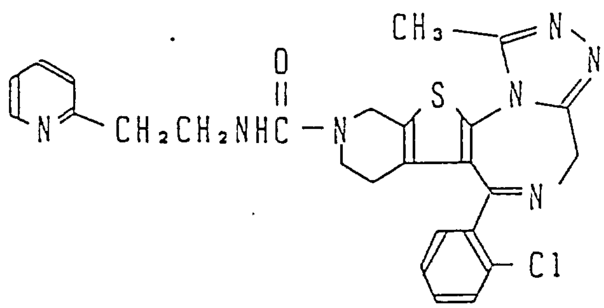

· $^{1}$H-NMR(90 MHz, $CDCl_3$) $\delta$ :
1.46 - 2.25(m,2H), 2.65(s,3H), 2.76 - 5.76(m,6H), 2.92(t,J=7Hz,2H), 3.42 - 3.68(m,2H), 5.95 - 6.24(m,1H), 6.93-7.39(m,6H), 7.40 - 7.66(m,1H), 8.25 - 8.40(m,1H)
· MS m/z: 517

Example 62

6-(2-Chlorophenyl)-3-[2-(morpholin-4-yl)ethylamino carbonyl)]-11-methyl-2,3,4,5-tetrahydro-8H-pyrido[4',3':4,5] thieno[3,2-f][1,2,4]triazolo [4,3-a][1,4]diazepine

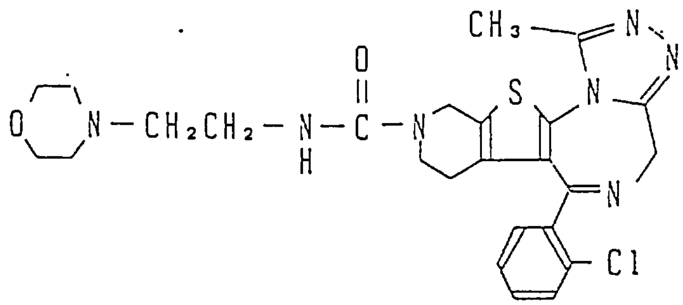

· $^1$H-NMR (90 MHz, $CDCl_3$) $\delta$ :

1.54 - 2.20(m,2H), 2.30 - 258 (m,6H), 2.67(s,3H), 2.88 5.80(m,6H), 3.17 - 3.42(m,2H), 3.55 - 3.74(m,4H), 5.03-5.19(m,1H), 7.30(m,4H)

· MS m/z: 525

Example 63

6-(2-Chlorophenyl)-3-[4-(morpholin-4-yl-carbonyloxy)-2-butynyloxycarbonyl]-11-methyl-2,3,4,5-tetrahydro-8H-pyrido [4',3':4,5] thieno[3,2-f][1,2,4]triazolo[4,3-a][1,4]diazepine

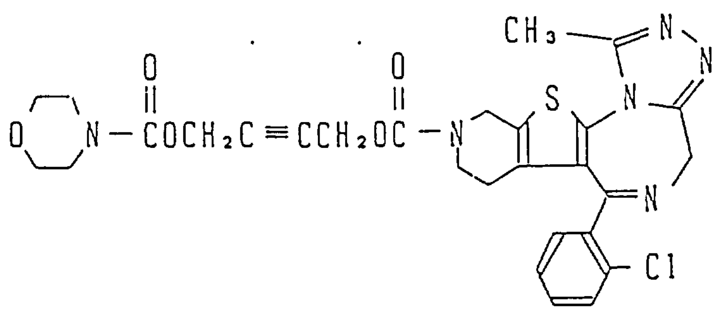

· $^1$H-NMR(90 MHz, $CDCl_3$) $\delta$ :

1.55 - 2.28(m,2H), 2.66(s,3H), 2.87 - 5.75(m,6H), 3.34-3.54(m,4H), 3.54 - 3.72(m,4H), 4.75(s,4H), 7.30(s,4H)

· MS m/z: 594

Example 64

6-(2-Chlorophenyl)-11-methyl-3-[4-(pyridin-2-yl-methylaminocarbonyloxy)-2-butynyloxycarbonyl]-2,3,4,5-tetrahydro-8H-pyrido[4',3':4,5] thieno[3,2-f][1,2,4]triazolo[4,3-a][1,4]diazepine

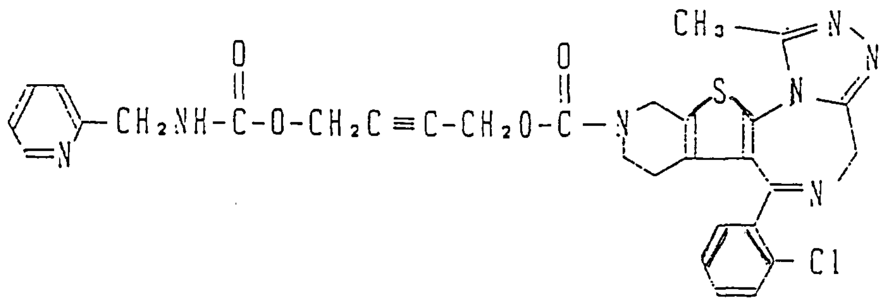

· $^1$H-NMR(90 MHz, $CDCl_3$) δ :

1.6 - 2.2(m,2H), 2.70(s,3H), 3.00 - 5.75(m,6H), 4.46(d,J=5Hz,2H), 4.72(s,4H), 5.90 - 6.20(m,1H), 7.1-7.6(m,6H), 7.5 - 7.9(m,1H), 8.40 - 8.70(m,1H)

· MS m/z: 615

Example 65

6-(2-Chlorophenyl)-11-methyl-3-(4-pentynyloxycarbonyl)-2,3,4,5-tetrahydro-8H-pyrido[4',3':4,5] thieno[3,2-f][1,2,4] triazolo[4,3-a][1,4]diazepine

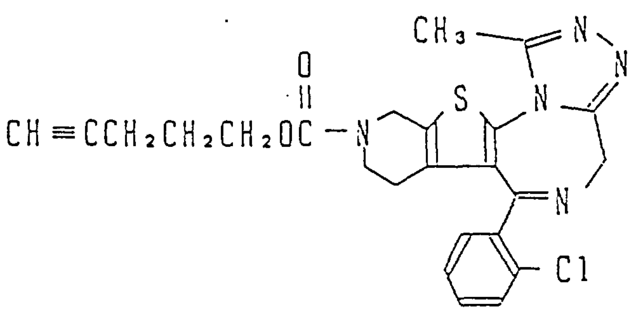

· $^1$H-NMR(90 MHz, $CDCl_3$) δ :

1.52 - 2.08(m,4H), 1.92(t,J=2Hz,1H), 2.08 - 2.40(m,2H), 2.66(s,3H), 2.84 - 5.72(m,6H), 4.17(t,J=7Hz,2H), 7.29 (m,4H)

· MS m/z: 479

Example 66

6-(2-Chlorophenyl)-11-methyl-3-(2-propynyloxycarbonyl)-2,3,4,5-tetrahydro-8H-pyrido[4',3':4,5] thieno[3,2-f][1,2,4] triazolo[4,3-a][1,4]diazepine

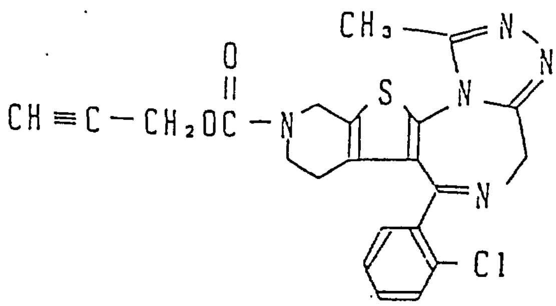

· $^1$H-NMR(90 MHz, $CDCl_3$) δ :
1.68 - 2.15(m,2H), 2.50(t,J=3Hz,1H), 2.62(s,3H), 2.85-5.79(m,6H), 4.65(d,J=3Hz,2H), 7.40(m,4H)
· MS m/z: 451

Example 67

6-(2-Chlorophenyl)-11-methyl-3-[2-(pyridin-2-yl)-ethoxycarbonyl]-2,3,4,5-tetrahydro-8H-pyrido[4',3':4,5] thieno[3,2-f][1,2,4]triazolo[4,3-a][1,4]diazepine

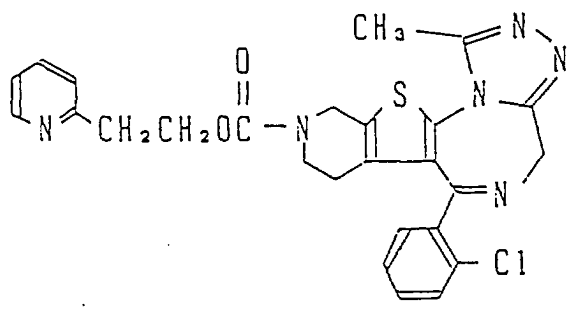

· $^1$H-NMR(90 MHz, $CDCl_3$) δ :
1.64 - 2.25(m,2H), 2.50 - 5.74(m,6H), 2.71(s,3H), 2.90(t,J=7Hz,2H), 3.91(t,J=7Hz, 2H), 6.86 - 7.80(m,7H), 8.36-8.76(m,1H)
· MS m/z: 518

Example 68

6-(2-Chlorophenyl)-11-methyl-3-(tetrahydropyran-2-yl)-methoxycarbonyl)-2,3,4,5-tetrahydro-8H-pyrido[4',3':4,5] thieno[3,2-f][1,2,4]triazolo[4,3-a][1,4]diazepine

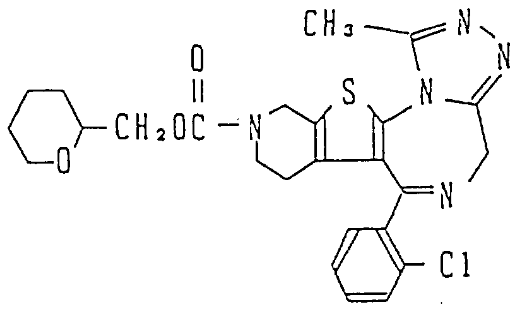

· $^1$H-NMR(90 MHz, $CDCl_3$) δ :
1.12 - 2.32(m,8H), 2.66(s,3H), 2.92 - 5.72(m,11H), 7.30(m,4H)
· MS m/z: 499

Example 69

6-(2-Chlorophenyl)-11-methyl-3-[2-(morpholin-2-yl)-ethoxycarbonyl]-2,3,4,5-tetrahydro-8H-pyrido[4',3':4,5] thieno [3,2-f][1,2,4]triazolo[4,3-a][1,4]diazepine

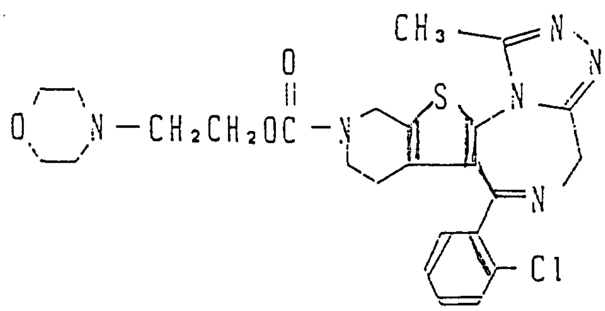

· $^1$H-NMR(90 MHz, $CDCl_3$) δ :
1.54 - 2.24(m,2H), 2.36 - 2.64(m,6H), 2.68(s,3H), 3.04-5.84(m,6H), 3.52 - 3.80(m,4H), 4.24(t,J=7Hz,2H), 7.39 (m,4H)
· MS m/z: 526

Example 70

3-[2-(Tetrahydropyran-4-yl)oxyethyl]oxycarbonyl-6-(2-chlorophenyl)-11-methyl-2,3,4,5-tetrahydro-8H-pyrido[4',3':4,5] thieno[3,2-f][1,2,4]triazolo[4,3-a] [1,4]diazepine

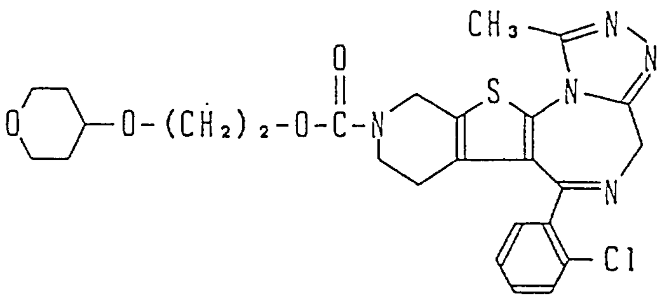

· $^1$H-NMR(90MHz, $CDCl_3$) δ :
1.30 - 2.40(m,6H), 2.68(s,3H), 2.80 - 5.70(m,15H), 7.20-7.54(m,4H)

Example 71

6-(2-Chlorophenyl)-3-cyclohexylethoxycarbonyl-11-methyl-2,3,4,5-tetrahydro-8H-pyrido[4',3':4,5] thieno[3,2-f][1,2,4] triazolo[4,3-a][1,4]diazepine

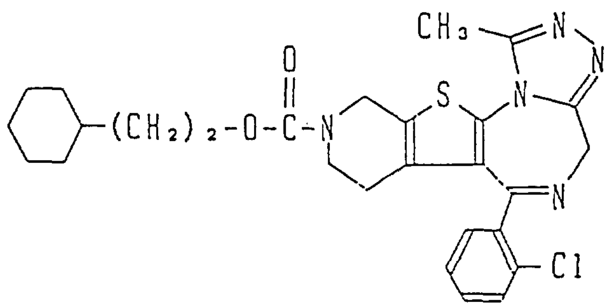

· $^1$H-NMR(90MHz, $CDCl_3$) δ :
0.6 - 2.3(m,15H), 2.7(s,3H), 3.0 - 4.0(m,2H), 4.0-4.4(m,1+2H), 4.4 - 4.8(m,2H), 5.4 - 5.8(m,1H), 7.4(m,4H)
MS m/z(Pos. FAB): 524

Example 72

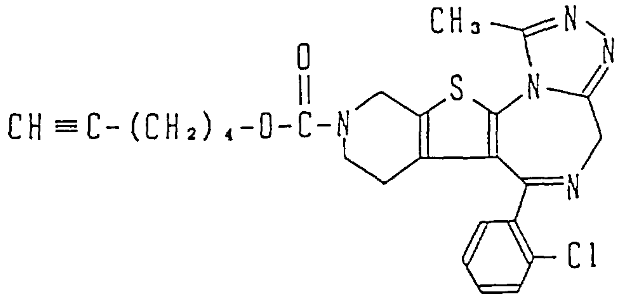

· $^1$H-NMR(90MHz, $CDCl_3$) $\delta$ :
1.20 - 2.32(m,6H), 1.94(t,J=2Hz,1H), 2.21(dt,J=2Hz,7Hz, 2H), 2.66(s,3H), 2.84 - 5.76(m,6H), 4.08(t,J=7Hz,2H), 7.28(m,4H)
· MS m/z(Pos. FAB): 495($M+H^+$)

Example 73

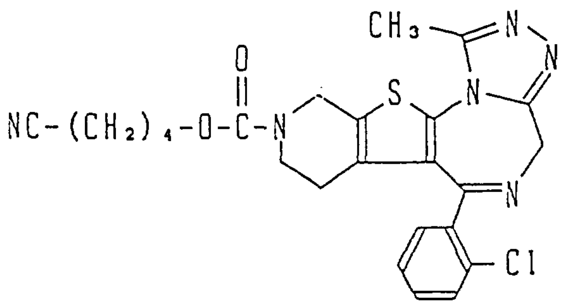

· $^1$H-NMR(90MHz, $CDCl_3$) $\delta$ :
1.40 - 2.21(m,6H), 2.37(t,J=7Hz,2H), 2.67(s,3H), 2.92-5.80(m,6H), 4.11(t,J=7Hz,2H), 7.31(m,4H)
· MS m/z(Pos. FAB): 494($M+H^+$)

Example 74

6-(2-Chlorophenyl)-3-(1-cyanoethoxy)carbonyl-11-methyl-2,3,4,5-tetrahydro-8H-pyrido[4',3':4,5]thieno[3,2-f] [1,2,4] triazolo[4,3-a][1,4]diazepine

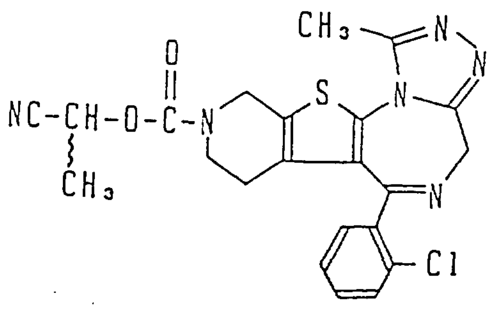

· $^1$H-NMR(90MHz, $CDCl_3$) δ :

1.70(t,J=7.0Hz,3H), 1.75(m,1H), 2.15(m,1H), 2.69(s,3H), 3.25(m,1H), 3.85(m,1H), 4.20(m,1H), 4.53(m,1H), 4.85 (m,1H), 5.43(m,1H), 5.65(m,1H), 7.23 - 7.65(m,4H)

· MS m/z(Pos. FAB): 467($M^+$)

Example 75

6-(2-Chlorophenyl)-3-cyclobutyloxycarbonyl-11-methyl-2,3,4,5-tetrahydro-8H-pyrido[4',3':4,5]thieno[3,2-f] [1,2,4] triazolo[4,3-a][1,4]diazepine

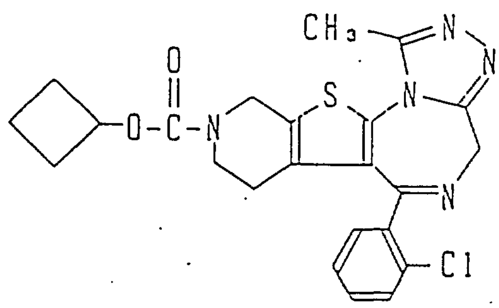

· $^1$H-NMR(90MHz, $CDCl_3$) δ :

1.5 - 2.5(m,8H), 2.7(s,3H), 3.0 - 4.0(m,2H), 4.0-4.9(m, 1+1+2H), 5.4 - 5.8(m,1H), 7.4(m,4H)

· MS m/z(Pos. FAB): 468

Example 76

6-1(2-Chlorophenyl)-3-(2methyl-2-cyanopropyloxy) carbonyl-11-methyl-2,3,4,5-tetrahydro-8H-pyrido[4',3':4,5]thieno [3,2-f][1,2,4]triazolo [4,3-a][1,4]diazepine

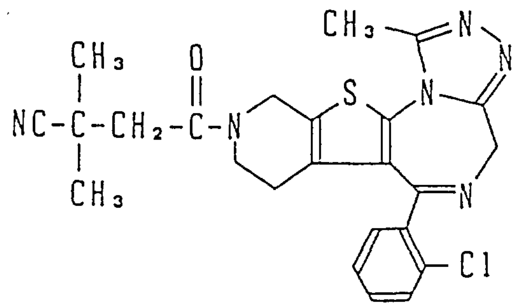

· $^1$H-NMR (90MHz, $CDCl_3$) δ :

1.35(s,3H), 1.45(s,3H), 1.75(m,1H), 2.12(m,1H), 2.70(s,3H), 3.25(m,1H), 3.90(m,1H), 4.08(s,2H), 4.22(m,1H), 4.55(m,1H), 4.56(m,1H), 5.62(m,1H), 7.30 - 7.45(m,4H)

· MS m/z(Pos. FAB): 495($M^+$)

Example 77

6-(2-Chlorophenyl)-11-methyl-3-(2-methylcyclohexyloxycarbonyl)-2,3,4,5-tetrahydro-8H-pyrido[4',3':4,5] thieno[3,2-f][1,2,4]triazolo[4,3-a][1,4]diazepine

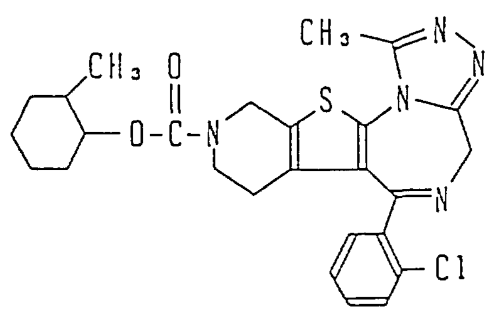

6-(2-Chlorophenyl)-11-methyl-3-(3-methylcyclohexyloxycarbonyl)-2,3,4,5-tetrahydro-8H-pyrido[4',3':4,5] thieno[3,2-f][1,2,4]triazolo[4,3-a][1,4]diazepine

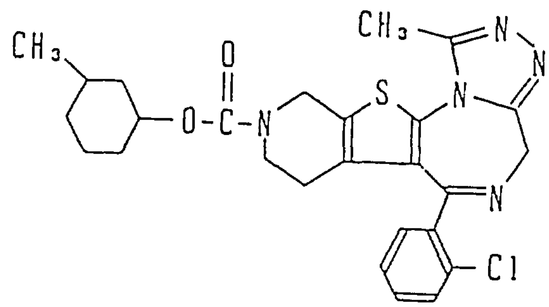

6-(2-Chlorophenyl)-11-methyl-3-(4-methylcyclohexyloxycarbonyl)-2,3,4,5-tetrahydro-8H-pyrido[4',3':4,5] thieno[3,2-f][1,2,4]triazolo[4,3-a][1,4]diazepine

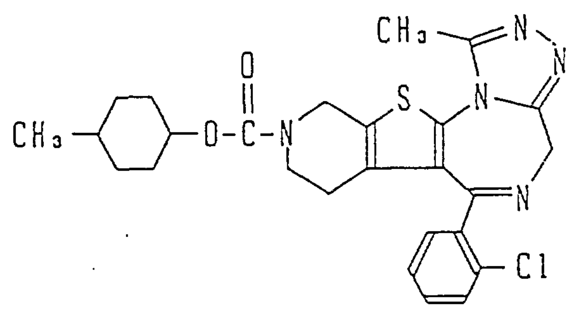

The above compounds had all the same NMR values as indicated below.

· $^1$H-NMR(90MHz, $CDCl_3$) $\delta$ :

0.7 - 1.0(d,3H), 1.0 - 2.2(m,11H), 2.7(s,3H), 3.0-4.0(m,2H), 4.0 - 4.9(m,1+1+2H), 5.3 - 5.8(m,1H), 7.4(m,4H)

· MS m/z(Pos. FAB): 510

Preparatory Example 1

3-Cyclopropylpropionic acid

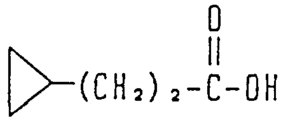

100 ml of methanol, 100 ml of tetrahydrofuran and 2 g of 10% palladium-carbon (containing 50% water) were added to 5.06 g of ethyl 3-cyclopropylacrylate, followed by hydrogenation reaction overnight at normal temperature and normal pressure conditions. The catalyst was removed by filtration and the solvent was distilled off. 20 ml of methanol, 20 ml of tetrahydrofuran, 10 ml of water and 7 g of sodium hydroxide were added to the residue and agitated at 80°C for 3.5 hours. The solvent was distilled off, to which water was added, followed by washing with ethyl acetate. A hydrochloric acid aqueous solution was added to the resultant aqueous phase under ice-cooling conditions to adjust

the pH to 3, followed by extraction with chloroform under slating-out conditions and drying with anhydrous magnesium sulfate. This was filtered and, after removal of the solvent by distillation, the resulting residue was subjected to silica gel column chromatography (developing solvent: dichloromethane) to obtain 1.80 g of the intended compound.

· $^1$H-NMR(90MHz, $CDCl_3$) δ :

0.65 - 1.1(m,2H), 1.1 - 1.85(m,5H), 2.33(t, J=7.2Hz,2H), 8.9(bs,1H)

Example 78

6-(2-Chlorophenyl)-3-(3-cyclopropyl)propionyl)-11-dimethyl-2,3,4,5-tetrahydro-8H-pyrido[4',3':4,5] thieno[3,2-f][1,2,4] triazolo[4,3-a][1,4]diazepine

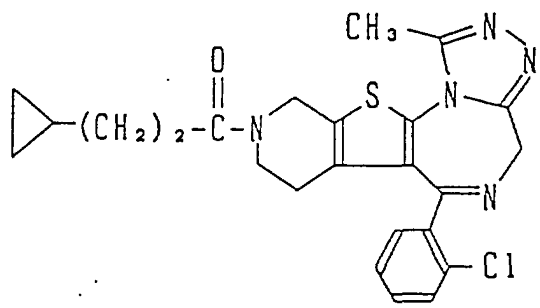

50 mg of 3-cyclopropylpropionic acid, 120 mg of 6-(2-chlorophenyl)-11-methyl-2,3,4,5-tetrahydro-8H-pyrido [4', 3':4,5]thieno[3,2-f][1,2,4]triazolo[4,3-a][1,4]diazepine and 60 mg of 1-hydroxybenzotriazole · monohydrate were dissolved in 8 ml of N,N-dimethylformamide, to which 80 mg of N,N'-dicyclohexylcarbodiimide was added under ice-cooling conditions. After agitation for about 10 minutes, the mixture was further agitated overnight at 4°C. Thereafter, it was agitated at room temperature for about 1 hour, after which the solvent was distilled off. A saturated sodium hydrogencarbonate aqueous solution was added to the distilled product, which was extracted with chloroform and dried with anhydrous magnesium sulfate. The solution was filtered and the solvent was distilled off, after which the residue was subjected to silica gel column chromatography (developing solvent: dichloromethane:methanol = : ), thereby obtaining mg of the intended compound

· $^1$H-NMR(90MHz, $CDCl_3$) δ :

0.7 - 1.05(m,3H), 1.05 - 2.4(m,6H), 2.27(t, J=7Hz,2H), 2.67(s,3H), 2.8 - 5.9(m,6H), 7.1 - 7.55(m,4H)

· MS m/z(Pos. FAB):

Example 79

6-(2-Chlorophenyl)-3-cinnamoyl)-11-methyl-2,3,4,5-tetrahydro-8H-pyrido[4',3':4,5] thieno[3,2-f][1,2,4]triazolo[4,3-f] [1,4]diazepine

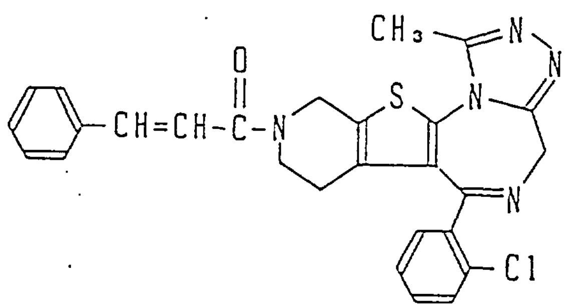

80 mg of cinnamoyl chloride was dissolved in 8 ml of N,N-dimethylformamide, into which 4 ml of an N,N-dimethylformamide solution of 120 mg of 6-(2-chlorophenyl)-11-methyl-2,3,4,5-tetrahydro-8H-pyrido[4',3':4,5]thieno [3,2-f][1,2,4]triazolo[4,3-a][1,4] diazepine and 160 mg of triethylamine was dropped at -60°C, followed by agitation for 30 minutes as it is. After removal of the solvent by distillation, a saturated sodium hydrogencarbonate aqueous solution was added, followed by extraction with chloroform and drying with anhydrous magnesium sulfate. This was filtered off and the solvent was distilled off. The resultant residue was subjected to silica gel column chromatography (developing solvent: methanol:dichloromethane = 1:99) to obtain 110 mg of the intended compound (yield 68%).

· $^1$H-NMR($CDCl_3$) δ :

1.2 - 2.6(m,2H), 2.48(s,3H), 2.8 - 5.9(m,6H), 6.74(t, J=15.1Hz,1H), 7.1 - 7.7(m,9H), 7.64(d,J=15.1Hz,1H)

· MS m/z(Pos. FAB): 500$(M+H)^+$

Example 80

6-(2-Chlorophenyl)-11-methyl-3-(2-methylcyclopropanecarbonyl)-2,3,4,5-tetrahydro-8H-pyrido [4',3':4,5]thieno[3,2-f][1,2,4]triazolo [4,3-a][1,4]diazepine

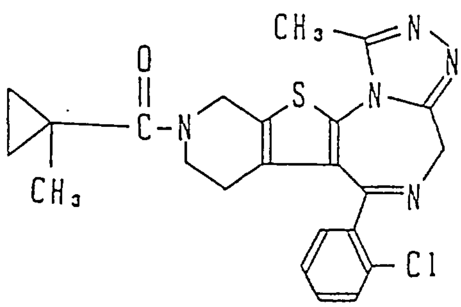

50 mg of 2-methylcyclopropanecarboxylic acid, 130 mg of 6-(2-chlorophenyl)-11-methyl-2,3,4,5-tetrahydro-8H-pyrido[4', 3':4,5]thieno[3,2-f][1,2,4]triazolo[4,3-a][1,4]diazepine and 70 mg of 1-hydroxybenzotriazole · monohydrate were dissolved in 8 ml of N,N-dimethylformamide, to which 90 mg of N,N'-dicyclohexylcarbodiimide was added under ice-cooling conditions, followed by agitation for about 10 minutes. Thereafter, agitation was continued at 4°C overnight and then at room temperature for 1 hour. After removal of the solvent by distillation, a saturated sodium hydrogencarbonate aqueous solution was added, followed by extraction with chloroform and drying with anhydrous magnesium sulfate. This was filtered off and the solvent was distilled off, followed by subjecting the resultant residue to silica gel column chromatography (developing solvent: dichloromethane:methanol = 99:1) to obtain 120 mg of the intended compound (yield 76%).

· $^1$H-NMR($CDCl_3$) δ :

0.4 - 0.72(m,1H), 0.72 - 1.0(m,1H), 1.26(s,3H), 1.4-2.4(m,2H), 2.68(s,3H), 2.9 - 5.9(m,6H), 7.1 - 7.7(m,4H)

· MS m/z(Pos. FAB): 452$(M+H)^+$

Example 81.

6-(2-Chlorophenyl)-11-methyl-3-phenylacetyl-2,3,4,5-tetrahydro-8H-pyrido[4',3':4,5] thieno[3,2-f][1,2,4]triazolo[4,3-a][1,4]diazepine

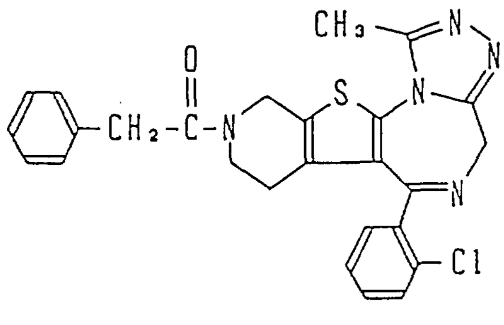

120 mg of 6-(2-chlorophenyl)-11-methyl-2,3,4,5-tetrahydro-8H-pyrido[4',3':4,5]thieno[3,2-f][1,2,4] triazolo [4,3-a][1,4]diazepine and 160 ml of triethylamine were dissolved in 4 ml of N,N-dimethylformamide, which was dropped into 5 ml of an N,N-dimethylformamide solution dissolving 60 mg of phenylacetic acid chloride at -60°C. After completion of the reaction, the solvent was distilled off from the reaction solution, to which a saturated sodium hydrogencarbonate solution was added, followed by extraction with chloroform and drying with anhydrous magnesium sulfate. This was filtered off and the solvent was distilled off, after which the resultant residue was subjected to silica gel column chromatography (developing solvent: dichloromethane:methanol = 99:1) to obtain 110 mg of the intended compound. (yield 69%).

· $^1$H-NMR($CDCl_3$) δ :

1.0 - 2.6(m,2H), 2.61 and 2.66(each s, total 3H), 2.8-6.0(m,6H), 3.69 and 3.77(each bs, total 2H), 6.8 - 7.7(m,9H)

· MS m/z(Pos. FAB): 488$(M+H)^+$

Example 82

6-(2-Chlorophenyl)-11-methyl-3-(3-methylcrotonoyl)-2,3,4,5-tetrahydro-8H-pyrido[4',3':4,5] thieno[3,2-f][1,2,4]triazolo [4,3-a][1,4]diazepine

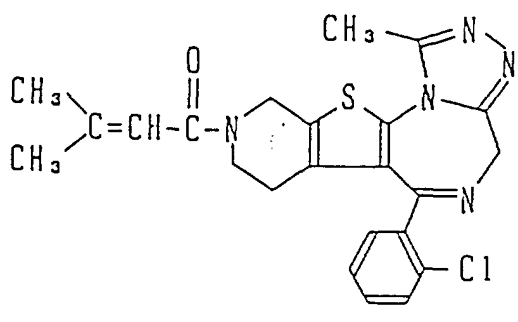

120 mg of 6-(2-chlorophenyl)-11-methyl-2,3,4,5-tetrahydro-8H-pyrido[4',3':4,5]thieno[3,2-f] [1,2,4]triazolo [4,3-a][1,4]diazepine and 160 mg of triethylamine were dissolved in 4 ml of N,N-dimethylformamide, which was dropped into 5 ml of an N,N-dimethylformamide solution dissolving 50 mg of 3-methylcrotonic acid chloride at -60°C. After completion of the reaction, the reaction solution was distilled off, to which a saturated sodium hydrogencarbonate aqueous solution was added, followed by extraction with chloroform and drying with anhydrous magnesium sulfate. This was filtered off and the solvent was also distilled off, after which the resultant residue was subjected to silica gel column chromatography (developing solution: dichloromethane:methanol = 99:1) to obtain 130 mg of the intended

compound.

· $^1$H-NMR($CDCl_3$) δ :

1.0 - 2.5(m,8H), 2.70(s,3H), 2.8 - 5.9(m,6H), 6.73(bs,1H), 7.1 - 7.6(m,4H)

· MS m/z(Pos. FAB): 452(M+H)$^+$

Example 83

6-(2-Chlorophenyl)-11-methyl-3-((trans)-2-phenylcyclopropanecarbonyl-2,3,4,5-tetrahydro-8H-pyrido[4',3':4,5] thieno [3,2-f][1,2,4]triazolo[4,3-a][1,4]diazepine

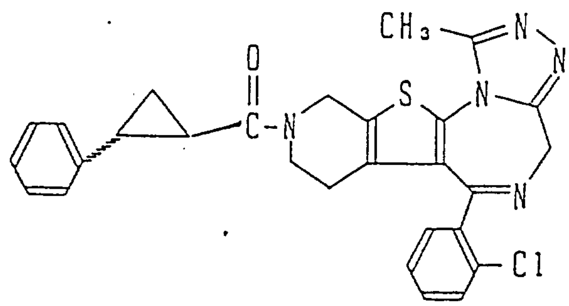

70 mg of (trans)-2-phenyl-1-cyclopropanecarboxylic acid, 120 mg of 6-(2-chlorophenyl)-11-methyl-2,3,4,5-tetrahydro-8H-pyrido[4',3':4,5]thieno[3,2-f][1,2,4]triazolo [4,3-a][1,4]diazepine and 60 mg of 1-hydroxybenzotriazole monohydrate were dissolved in 12 ml of N,N-dimethylformamide, to which 80 mg of N,N'-dicyclohexylcarbodiimide was added under ice-cooling conditions. After agitation for about 10 minutes, the mixture was further agitated overnight at 4°C and then at room temperature for 1 hour. After removal of the solvent by distillation, a saturated sodium hydrogencarbonate aqueous solution was added, followed by extraction with chloroform and drying with anhydrous magnesium sulfate. This sulfate was removed by filtration and the solvent was distilled off. The resultant residue was subjected to silica gel column chromatography (developing solvent: dichloromethane:methanol = 99:1) to obtain 160 mg of the intended compound.

· $^1$H-NMR($CDCl_3$) δ :

1.4 - 2.8(m,6H), 2.66(s,3H), 2.8 - 5.8(m,6H), 6.5-7.7(m,9H)

· MS m/z(Pos. FAB): 514(M+H)$^+$

6-(2-Chlorophenyl)-11-methyl-3-(4-pyridylthio)acetyl-2,3,4,5-tetrahydro-8H-pyrido[4',3':4,5] thieno[3,2-f][1,2,4]triazolo [4,3-a][1,4]diazepine

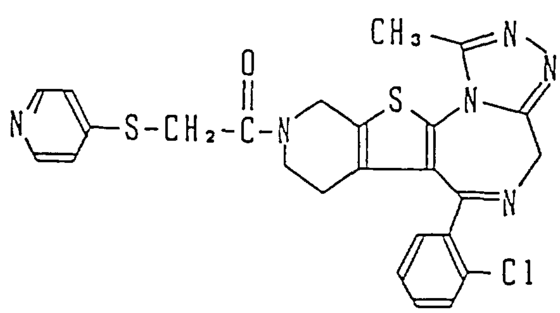

· $^1$H-NMR($CD_3OD$-$CDCl_3$) δ :

1.4 - 2.6(m,2H), 2.68(s,3H), 2.8 - 5.9 (m,6H), 3.82(bs,2H), 7.05 - 7.6(m,6H), 8.1 - 8.6(m,2H)

· MS m/z(Pos. FAB): 521((M+H)$^+$

Example 85

6-(2-Chlorophenyl)-11-methyl-3-(3-phenylpropionyl)-2,3,4,5-tetrahydro-8H-pyrido[4',3':4,5] thieno[3,2-f][1,2,4]triazolo [4,3-a][1,4]diazepine

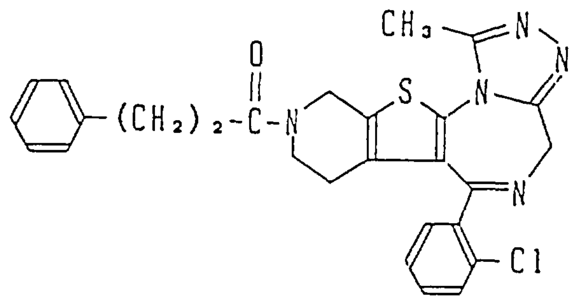

· $^1$H-NMR($CDCl_3$) δ :
1.0 - 2.3(m,4H), 2.66(s,3H), 2.65 - 3.15(m,2H), 2.8-5.9(m,6H), 6.65 - 7.65(m,9H)
MS m/z(Pos. FAB): 502((M+H)$^+$

Example 86

6-(2-Chlorophenyl)-11-methyl-3-[3-(3-pyridyl)acryloyl]-2,3,4,5-tetrahydro-8H-pyrido[4',3':4,5] thieno[3,2-f][1,2,4] triazolo[4,3-a][1,4]diazepine

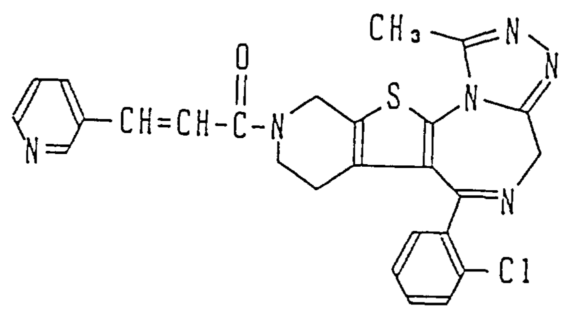

· $^1$H-NMR($CDCl_3$) δ :
1.5 - 2.5(m,2H), 2.68(s,3H), 3.0 - 5.8(m,6H), 6.83(bd,J=15.5Hz,1H), 7.15 - 7.9(m,6H), 7.60(d,J=15.5Hz,1H), 8.3 - 8.5(m,1H), 8.64(bs,1H)
· MS m/z(Pos. FAB): 501((M+H)$^+$

Example 87

6-(2-Chlorophenyl)-3-(3-cyclohexylpropionyl)-11-methyl-2,3,4,5-tetrahydro-8H-pyrido[4',3':4,5] thieno[3,2-f][1,2,4] triazolo[4,3-a][1,4]diazepine

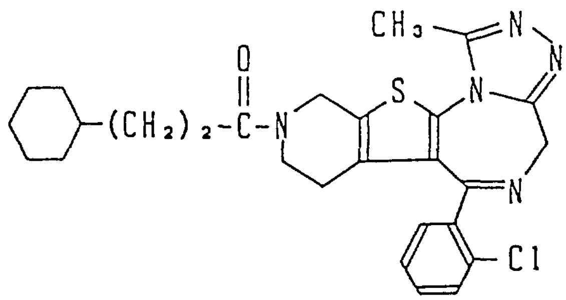

· $^1$H-NMR($CDCl_3$) δ :
0.6 - 2.5(m,15H), 2.28(bt,J=8Hz,2H), 2.66(s,3H), 2.8-5.9(m,6H), 7.1 - 7.6(m,4H)
· MS m/z(Pos. FAB): 508($(M+H)^+$)

Example 88

6-(2-Chlorophenyl)-3-(4-fluorophenyl)acetyl-11-methyl-2,3,4,5-tetrahydro-8H-pyrido[4',3':4,5] thieno[3,2-f][1,2,4] triazolo[4,3-a][1,4]diazepine

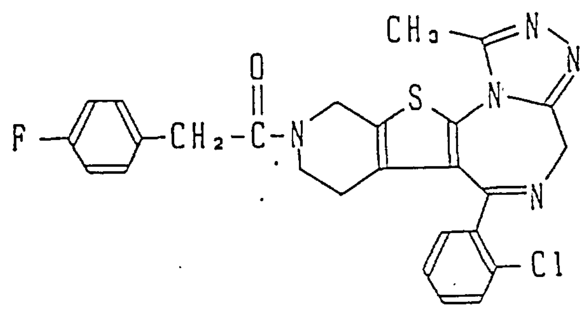

· $^1$H-NMR($CDCl_3$) δ :
1.0 - 2.4(m,2H), 2.66(s,3H), 2.8 - 5.9(m,6H), 3.65(bs,2H), 6.65 - 7.6(m,8H)
· MS m/z(Pos. FAB): 506($(M+H)^+$)

Example 89

6-(2-Chlorophenyl)-3-(4-cyanobutanoyl)-11-methyl-2,3,4,5-tetrahydro-8H-pyrido[4',3':4,5] thieno[3,2-f][1,2,4]triazolo [4,3-a][1,4]diazepine

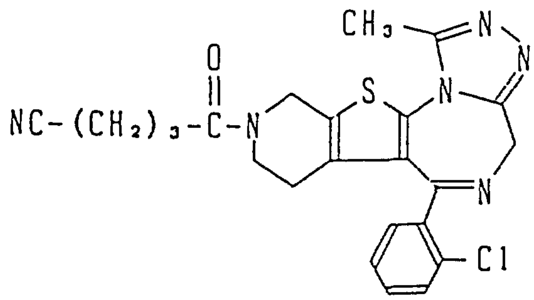

· $^1$H-NMR($CDCl_3$) δ :
1.4 - 2.3(m,4H), 2.3 - 2.65(m,4H), 2.67(s,3H), 2.8-5.8(m,6H), 7.1 - 7.6(m,4H)
· MS m/z(Pos. FAB): 465($(M+H)^+$)

Preparatory Example 2

Ethyl tetrahydropyrane- $\Delta^{4,a}$-acetate

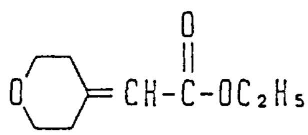

1.2 g (30 mmols) of sodium hydride was added to 100 ml of a dimethylformamide solution of 6.72 g (30 mmols) of diethylphosphonoethyl acetate under ice-cooling conditions, and was agitated for 10 minutes. 2.5 g (25 mmols) of tetrahydro-4H-pyran-4-one was further added to the mixture under ice-cooling conditions and was returned to room temperature, followed by agitation at 80°C for 2 hours. After completion of the reaction, ethyl acetate was added, followed by washing with a saturated saline solution and drying with magnesium sulfate. This was concentrated under reduced pressure and the resultant residue was subjected to silica gel column chromatography (elution solvent: hexane: ethyl acetate = 9:1) thereby obtaining 4.2 g of the intended compound as a light yellow oily substance.
· $^1$H-NMR($CDCl_3$) δ :
1.3(t,J=7,2Hz,3H), 2.0 - 2.3(m,2H), 3.0(bs,2H), 3.8(t,J=5.4Hz,2H), 4.0 - 4.3(m,2H), 4.1(q,J=7.2Hz,2H), 5.6(bs, 1H)

Preparatory Example 3

Ethyl 4-tetrahydropyranylacetate

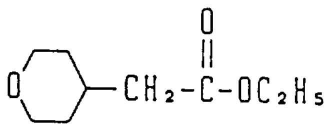

2.0 g of ethyl tetrahydropyran- $\Delta^{4,a}$-acetate was dissolved in 50 ml of methanol, to which 10% palladium-carbon was added, followed by hydrogenation for 3 hours. After removal of the catalyst by filtration, the reaction mixture was concentrated under reduced pressure to obtain 1.6 g of the intended compound.

· $^1$H-NMR($CDCl_3$) δ :

1.1 - 2.3(m,7H), 1.3(t,J=7,2Hz,3H), 3.2-3.6(td,J=12,6Hz,2.9Hz,2H), 3.8 - 4.3(m,2H), 4.1(q,J=7.2Hz,2H)

Preparatory Example 4

4-Tetrahydropyranylacetic acid

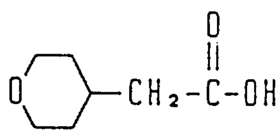

20 ml of methanol, 10 ml of water and 1 g of sodium hydroxide were added to 0.9 g of ethyl 4-tetrahydropyranylacetate and agitated at 80°C for 1 hour. The solvent was removed by distillation, to which water was added. After washing with ethyl acetate, a hydrochloric acid aqueous solution was added to the resultant aqueous phase to an extent of pH of 3, followed by extraction with chloroform under salting-out conditions and drying with anhydrous magnesium sulfate. This was removed by filtration and the solvent was distilled off, thereby obtaining 0.87 g of a crude intended compound.

· $^1$H-NMR($CDCl_3$) δ :

1.0 - 2.4(m,5H), 2.28(bd,J=6.5Hz,2H), 3.37(td,J-11.5Hz,2.9Hz,2H), 3.7 - 4.1(m,2H), 7.85(bs,1H)

Example 90

6-(2-Chlorophenyl)-11-methyl-3-(tetrahydropyran-4-yl)-acetyl-2,3,4,5-tetrahydro-8H-pyrido[4',3':4,5] thieno[3,2-f] [1,2,4]triazolo[4,3-a][1,4]diazepine

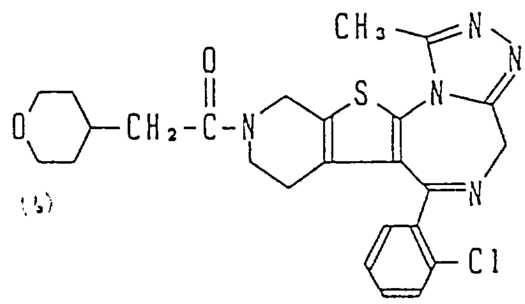

· $^{1}H$-NMR($CD_3OD$-$CDCl_3$) $\delta$ :
0.9 - 2.4(m,9H), 2.67(s,3H), 2.8 - 5.9 (a,10H), 7.1-7.5(m,4H)
· MS m/z(Pos. FAB): 496($(M+H)^+$

Preparatory Example 5

Tetrahydropyran- $\Delta^{4,a}$-acetic acid

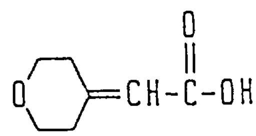

20 ml of methanol, 10 ml of water and 1 g of sodium hydroxide were added to 0.8 g of ethyl tetrahydropyran-$\Delta^{4,a}$-acetate and agitated at 80°C for 2 hours. After removal of the solvent by distillation, water was added to the mixture, followed by washing with ethyl acetate. A hydrochloric acid aqueous solution was added to the aqueous phase to render it acidic, followed by extraction with chloroform under salting-out conditions and drying with anhydrous magnesium sulfate. This was filtered off and the solvent was distilled off, followed by subjecting the resultant residue to silica gel column chromatography (developing solvent: dichloromethane) thereby obtaining 0.17 g of the intended compound (yield 25%).

· $^{1}H$-NMR($CD_3OD$-$CDCl_3$) $\delta$ :
2.34(bt, J=5.4Hz, 2H), 2.98(bt,J=5.4Hz,2H), 3.5-3.95(m,4H), 5.66(bs,1H), 8.45(bs,1H)

Example 91

6-(2-Chlorophenyl)-11-methyl-3-(tetrahydropyran- $\Delta^{4,a}$-acetyl-2,3,4,5-tetrahydro-8H-pyrido[4',3':4,5] thieno[3,2-f] [1,2,4]triazolo[4,3-a][1,4]diazepine

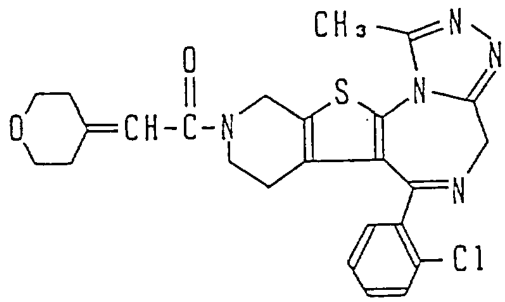

· $^1H$-NMR($CDCl_3$) δ :

1.4 - 2.5(m,2H), 2.1 - 2.41(m,2H), 2.41 - 2.8(m,2H), 2.67(s,3H), 2.8 - 5.9 (m,6H), 3.45 - 3.9(m,4H), 5.72(bs,1H), 7.15 - 7.5(m,6H)

· MS m/z(Pos. FAB): 494($(M+H)^+$)

Example 92

6-(2-Chlorophenyl)-3-[(trans)-3-cyclopropylacryloyl]-11-methyl-2,3,4,5-tetrahydro-8H-pyrido[4',3':4,5] thieno[3,2-f] [1,2,4]triazolo[4,3-a][1,4]diazepine

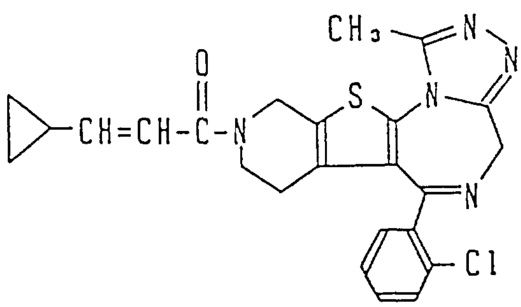

· $^1H$-NMR($CDCl_3$) δ :

0.4 - 1.15(m,4H), 1.2 - 2.6(m,3H), 2.66(s,3H), 2.8-6.0(m,6H), 6.13(d,J=21.6Hz,1H), 6.25(dd,J=21.6, 14.4Hz,1H), 7.1 - 7.5(m,4H)

· MS m/z(Pos. FAB): 464($(M+H)^+$)

(1) Preparation of ethyl (trans)-3-cyclopropylacrylate

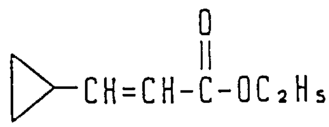

38.38 g of ethyl diethylphosphonoacetate was dissolved in 300 ml of N,N-dimethylformamide, to which 6.85 g of 60% sodium hydride at 0°C, followed by agitation at room temperature for 30 minutes and dropping 10 g of cyclopropanealdehyde at 0°C. After agitation at room temperature for 2 hours, iced water was added, followed by extraction with ether, washing with water and drying with anhydrous magnesium sulfate. This was removed by filtration and a concentrated residue was subjected to silica gel column chromatography (developing solvent: ethyl acetate:hexane = 2:98), thereby obtaining 11.18 g of the intended compound as a trans product (yield 60%).

· $^1$H-NMR($CDCl_3$) δ :

0.4 - 1.1(m,4H), 1.26(t,J=7.2Hz, 3H), 1.3 - 1.8(m,1H), 4.13(q,J=7.2Hz,2H), 5.82(d,J=15.5Hz,1H), 6.37(dd, J=15.5Hz, 10.1Hz,1H)

(2) Preparation of (trans)-3-cyclopropylacrylic acid

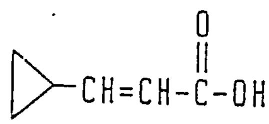

100 ml of methanol, 50 ml of water and 6.4 g of sodium hydroxide were added to 11.18 g of ethyl (trans)-3-cyclopropylacrylate obtained in the above method and subjected to reaction at 80°C for 1.5 hours. After concentration, concentrated hydrochloric acid was added so as to render the reaction mixture acidic, followed by extraction with chloroform, washing with a saturated saline solution and drying with anhydrous magnesium sulfate. This sulfate was removed by filtration and the filtrate was concentrated to obtain 8.82 g of the intended compound (yield 99%).

· $^1$H-NMR($CDCl_3$) δ :

0.3 - 1.2(m,4H), 1.2 - 1.9(m,1H), 5.83(d,J=15.1Hz,1H), 6.47(dd,J=15.1Hz, 6.5Hz,1H), 9.06(bs,1H)

Preparatory Example 6

N-(Benzyloxycarbonyl)-3-pyrrolidinol

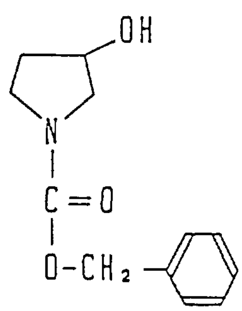

30 g of 3-pyrrolidinol was dissolved in 500 ml of chloroform, to which 53 ml of triethylamine was added, followed by gradually dropping 52 ml of benzyloxycarbonyl chloride at room temperature. After completion of the reaction, the reaction solution was poured into water and extracted with chloroform. The solvent was distilled off under reduced pressure and the resultant residue was purified by column chromatography (eluting solvent: hexane-ethyl acetate) to obtain 70.67 g of the intended compound.

$^1$H-NMR(90 MHz, $CDCl_3$) δ :
1.7-2.1(m,2H), 2.8-3.2(m,1H), 3.2-3.7(m,4H),
4.2-4.5(m,1H), 5.1(s,2H), 7.3(s,5H)

Preparatory Example 7

N-(Benzyloxycarbonyl)-3-pyrrolidone

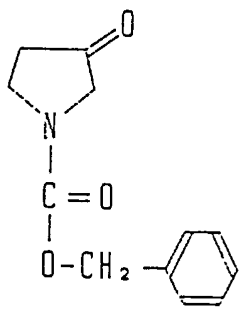

150 ml of oxalic acid chloride was added to 2 liters of dichloromethylene. 245 ml of dimethylsulfoxide was gradually added at -70 to -50°C in a stream of argon. 70.67 g of N-(N-(benzyloxycarbonyl)-3-pyrrolidinol dissolved in dichloromethylene was dropped. After gradual dropping of 720 ml of triethylamine, the mixture was raised to room temperature. After completion of the reaction, the reaction solution was poured into water and extracted with dichloromethylene, followed by removal of the solvent by distillation under reduced pressure. The residue was purified by column chromatography (eluting solvent: hexane-ethyl acetate), thereby obtaining 66.55 g of the intended compound.

$^1$H-NMR(90 MHz, $CDCl_3$) δ :
2.6(t,J=7.5Hz,2H), 3.7-4.0(m,4H), 5.12(s,2H),
7.3(s,5H)

Preparatory Example 8

2-Amino-3-(2-chlorobenzoyl)-5-benzyloxycarbonyl-4,6-dihydro-thieno[2,3-c]pyrrole

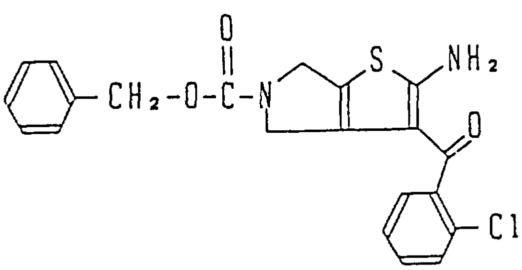

66.55 g of the compound obtained in Preparatory Example 7, 54.3 g of 2-chlorocyanoacetophenone and 9.9 g of sulfur were dissolved in 300 ml of dimethylformamide, to which 45 ml of triethylamine was added, followed by heating at 60°C for 2 hours. After completion of the reaction, the solvent was distilled off under reduced pressure and the resultant residue was purified by column chromatography (eluting solvent: hexane-ethyl acetate), thereby obtaining

68.4 g of the intended compound.

$^1$H-NMR(90 MHz, $CDCl_3$) δ :

3.4-3.9(m,2H), 4.3-4.6(m,2H), 4.98 and 5.02(each s, total 2H), 70-7.6(m,11H)

Preparatory Example 9

2-Bromoacetylamino-3-(2-chlorobenzoyl)-5-benzyloxycarbonyl-4,6-dihydro-thieno[2,3-c]pyrrole

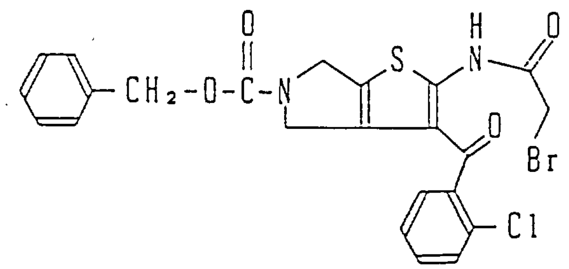

600 ml toluene/150 ml water was added to 68.4 g of the compound obtained in Preparatory Example 8, to which 33.5 g of sodium hydrogencarbonate, followed by dropping 25 ml of bromoacetyl bromide at 60°C. After completion of the reaction, the reaction solution was poured into dichloromethylene and washed with water, followed by removal of the solvent by distillation under reduced pressure. The resultant residue was used for subsequent reaction as it is without any purification.

$^1$H-NMR(90 MHz, $CDCl_3$) δ :

3.5-3.95(m,2H), 4.08(s,2H), 4.3-4.7(m,2H), 5.03 and 5.07(each s, total 2H), 7.0-7.6(m,10H)

Preparatory Example 10

2-Aminoacetylamino-3-(2-chlorobenzoyl)-5-benzyloxycarbonyl-4,6-dihydro-thieno[2,3-c]pyrrole

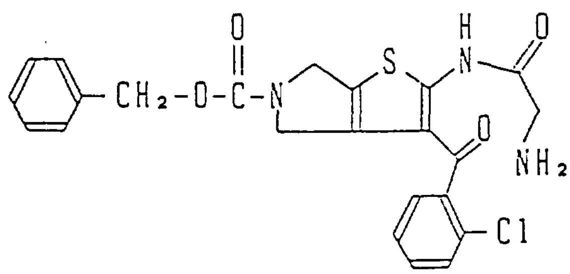

The compound obtaining Preparatory Example 9 was dissolved in 3.5 liters of ethyl acetate, which was saturated with ammonia gas. After introduction of the gas for 8 hours, insoluble inorganic matters were filtered off and the resultant filtrate was subjected to distillation under reduced pressure to remove the solvent, and the resultant crystals were collected by filtration to obtain 41.9 g of the intended compound.

$^1$H-NMR(90 MHz, $CDCl_3$) δ :

3.3-3.9(m,4H), 4.4-4.7(m,2H), 5.02 and 5.06(each s, total 2H), 7.0-7.5(m,11H)

Preparatory Example 11

5-(2-Chlorophenyl)-7-benzyloxycarbonyl-6,8-dihydro-1-3H,pyrrolo[4',3':4,5]thieno[3,2-f][1,4]diazepin-2-one

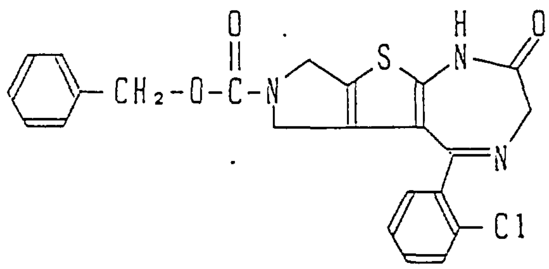

38.5 g of the compound obtained in Preparatory Example 10 was dissolved in 250 ml benzene/500 ml pyridine, to which 5.2 ml of acetic acid was added, followed by removing produced water from the system while heating at 120°C. After completion of the reaction, the reaction solution was concentrated under reduced pressure and the resultant residue was purified by column chromatography (eluting solvent: hexane-ethyl acetate) to obtain 21.0 g of the intended compound.

$^1$H-NMR(90 MHz, $CDCl_3$) δ :
3.6-3.9(m,2H), 4.42(s,2H), 4.3-4.7(m,2H), 5.06(s,2H), 7.0-7.5(m,10H)

Preparatory Example 12

5-(2-Chlorophenyl)-7-benzyloxycarbonyl-6,8-dihydro-1,3H-pyrrolo[4',3':4,5]thieno[3,2-f][1,4]-diazepin-2-thione

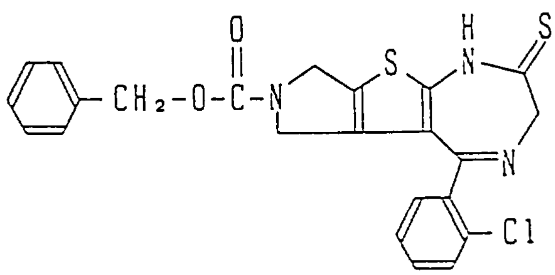

13.7 g of the compound obtained in Preparatory Example 11 was dissolved in 300 ml of toluene, to which 12.3 g of the Rhoson agent, [2,4-bis(4-methoxyphenyl)-1,3-dithia-2,4-diphosphetan-2,4-disulfide) was added, followed by heating at 80°C for 15 minutes. After completion of the reaction, the solvent was distilled off under reduced pressure and the resultant reside was purified by column chromatography (elusion solvent: hexane-ethyl acetate) to obtain 9.3 g of the intended compound.

$^1$H-NMR(90 MHz, $CDCl_3$) δ :
3.5-3.9(m,2H), 4.3-4.7(m,4H), 5.0 and 5.06(each s, total 2H), 6.9-7.5(m,10H)

Preparatory Example 13

3-Benzyloxycarbonyl-5-(2-chlorophenyl)-10-methyl-2,4-dihydro-2H,7H-pyrrolo[4',3':4,5]thieno[3,2-f][1,2,4]- triazolo [4,3-a][1,4]diazepine

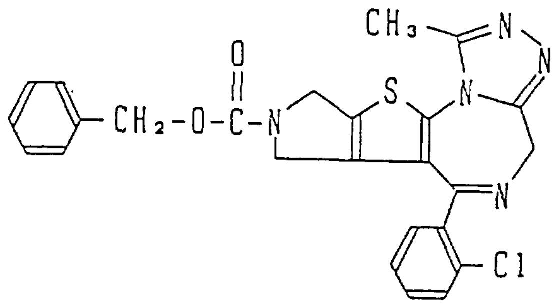

250 ml of methanol was added to 10.2 g of the compound obtained in Preparatory Example 12, to which 4.8 g of hydrazine monohydrate was added, followed by agitation at room temperature for 1 hour. After completion of the reaction, precipitated hydrazide was collected by filtration. 200 ml of triethyl ortho-acetate was added to it and heated at 80°C for 40 minutes. After completion of the reaction, the reaction mixture was concentrated under reduced pressure and the resultant residue was purified by column chromatography (eluting solvent: benzene-acetone) to obtain 7.39 g of the intended compound.

$^1$H-NMR(90 MHz, $CDCl_3$) δ :

2.7(s,3H), 3.6-3.9(m,2H), 4.6-4.8(m,2H), 4.8-5.0(m,2H),

5.05 and 5.09(each s, total 2H), 7.0-7.5(m,9H)

Preparatory Example 14

3H-5-(2-Chlorophenyl)-10-methyl-2,4-dihydro-2H,7H-pyrrolo[4',3':4,5]thieno[3,2-f][1,2,4]triazolo-[4,3-a][1,4]diazepine

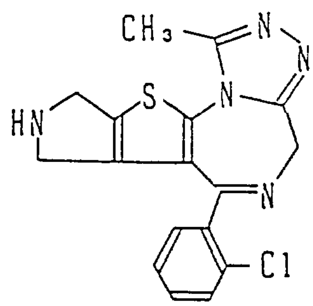

0.23 g of the compound obtained in Preparatory Example 13 was added 3 ml of dichloromethylene, to which 0.7 ml of iodotrimethylsilane, followed by agitation at room temperature. After completion of the reaction, methanol was added and the solvent was distilled off under reduced pressure. The resultant residue was purified by column chromatography (eluting solvent: dichloromethylene-methanol/ammonia solution) to obtain 0.1 g of the intended compound.

$^1$H-NMR(90 MHz, $CDCl_3$) δ :

2.7(s,3H), 3.6-3.9(m,2H), 4.5-4.7(m,2H), 4.8-5.1(m,2H),

7.0-7.6(m,4H)

Example 93

3-(2'-Cyanoethyl)oxycarbonyl-5-(2-chlorophenyl)-10-methyl-2,4-dihydro-2H,7H-pyrrolo[4',3':4,5]thieno[3,2-f] [1,2,4] triazolo[4,3-a][1,4]diazepine

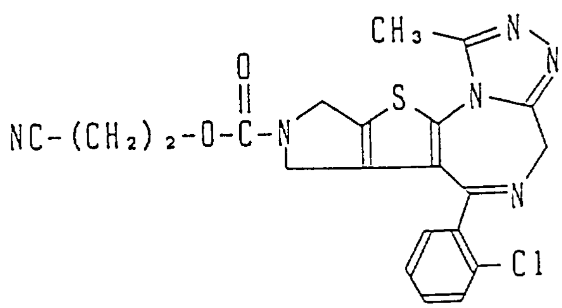

$^1$H-NMR(CDCl$_3$) δ :
2.67(t,J=7Hz,1H), 2.75(t,J=7Hz,1H), 2.75(s,3H), 3.64-3.90(m,2H), 4.25(t,J=7Hz,1H), 4.31(t,J=7Hz,1H), 4.64-4.82(m,2H), 4.80-5.12(m,2H), 7.26-7.46(m,4H)
MS m/z: 453

Example 94

3-(3'-Butynyl)oxycarbonyl-5-(2-chlorophenyl)-10-methyl-2,4-dihydro-2H,7H-pyrrolo[4',3':4,5]thieno[3,2-f] [1,2,4] triazolo[4,3-a][1,4]diazepine

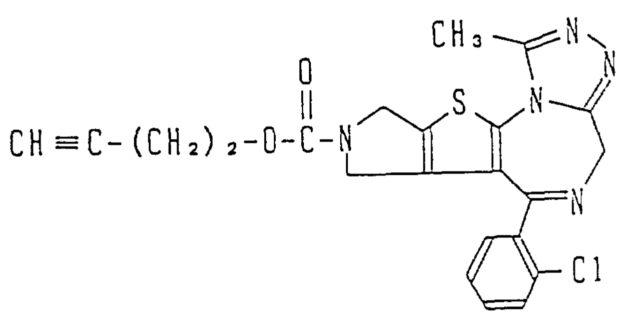

$^1$H-NMR(CDCl$_3$) δ :
2.00(t,J=7Hz,1H), 2.46(dt,J=7Hz,2Hz,1H), 2.55(s,3H), 2.75(s,3H), 3.60-3.85(m,2H), 4.14(t,J=7Hz,1H), 4.20(t,J=7Hz,1H), 4.60-4.85(m,2H), 4.75-5.10(m,2H), 7.10-7.54(m,4H)
MS m/z: 452

Example 95

3-[2-(Morpholin-4-yl)ethyl]-oxycarbonyl-5-(2-chlorophenyl)-10-methyl-2,4-dihydro-2H,7H-pyrrolo[4',3':4,5]thieno [3,2-f] [1,2,4]triazolo[4,3-a][1,4]diazepine

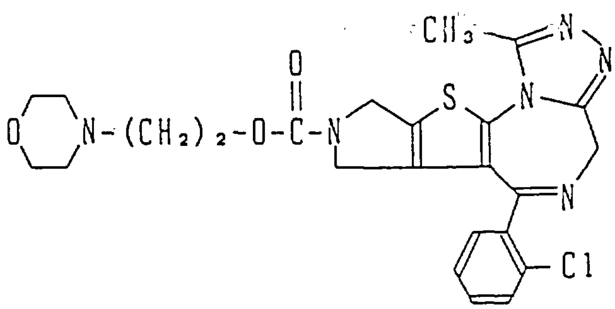

$^1$H-NMR($CDCl_3$) δ :
2.20-2.85(m,6H), 2.76(s,3H), 3.50-3.90(m,6H),
4.20(t,J=7Hz,1H), 4.28(t,J=7Hz,1H),
4.55-4.80(m,2H), 4.80-5.15(m,2H), 7.20-7.55(m,4H)
MS m/z: 513

Example 96

3-(3-Pyridylethyl)oxycarbonyl-5-(2-chlorophenyl)-10-methyl-2,4-dihydro-2H,7H-pyrrolo[4',3':4,5]thieno[3,2-f] [1,2,4] triazolo[4,3-a][1,4]diazepine

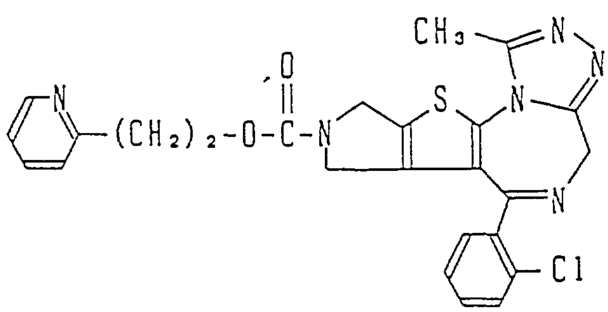

$^1$H-NMR($CDCl_3$) δ :
2.72(s,3H), 3.04(t,J=7Hz,1H), 3.12(t,J=7Hz,1H),
3.48-3.85(m,2H), 4.30-4.80(m,4H), 4.70-5.18(m,2H),
4.70-5.18(m,2H), 6.95-7.70(m,7H), 8.40-8.56(m,1H)
MS m/z: 505

Example 97

3-(Tetrahydropyran-4-yl)oxycarbonyl-5-(2-chlorophenyl)-10-methyl-2,4-dihydro-2H,7H-pyrrolo[4',3':4,5]thieno[3, 2-f][1,2,4]triazolo[4,3-a][1,4]diazepine

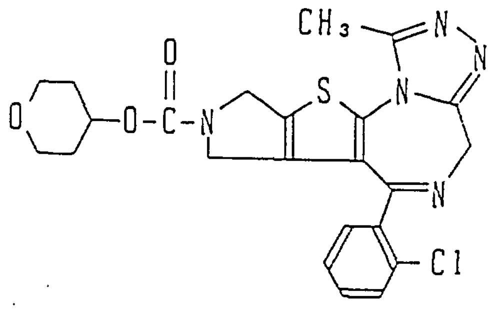

$^1$H-NMR($CDCl_3$) δ :
1.40-2.30(m,4H), 2.74(s,3H), 3.32-4.05(m,7H),
4.60-5.08(m,4H), 7.20-7.56(m,4H)
MS m/z: 484

Example 98

3-(3'-butyn-2'-yl)oxycarbonyl-5-(2-chlorophenyl)-10-methyl-2,4-dihydro-2H,7H-pyrrolo[4',3':4,5]thieno[3,2-f] [1,2,4]triazolo[4,3-a][1,4]diazepine

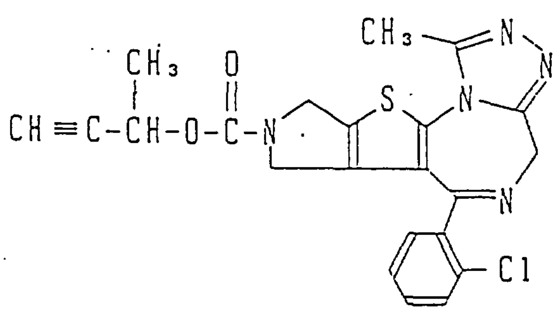

$^1$H-NMR($CDCl_3$)δ :
1.54(d,J=7Hz,3H), 2.48(d,J=2Hz,1H), 2.72(s,3H),
3.60-3.86(m,2H), 4.60-4.82(m,2H), 4.78-5.10(m,2H),
5.18-5.46(m,1H), 7.12-7.54(m,4H)
MS m/z: 452

Example 99

3-Cyclohexylmethyloxycarbonyl-5-(2-chlorophenyl)-10-methyl-2,4-dihydro-2H,7H-pyrrolo[4',3':4,5]thieno[3,2-f] [1,2,4] triazolo[4,3-a][1,4]diazepine

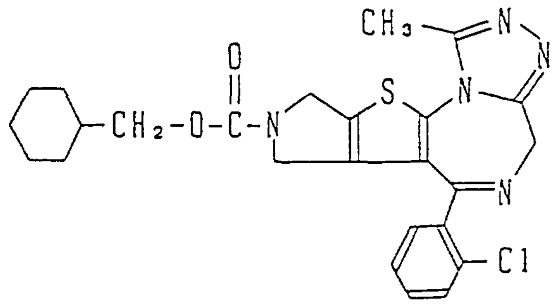

$^1$H-NMR($CDCl_3$) δ :
0.68-1.96(m,11H), 2.75(s,3H), 3.56-4.00(m,4H),
4.65-4.80(m,2H), 4.82-5.18(m,2H), 7.20-7.58(m,4H)
MS m/z: 496

Example 100

3-Benzylaminocarbonyl-5-(2-chlorophenyl)-10-methyl-2,4-dihydro-2H,7H-pyrrolo[4',3':4,5]thieno[3,2-f] [1,2,4]triazolo [4,3-a][1,4]diazepine

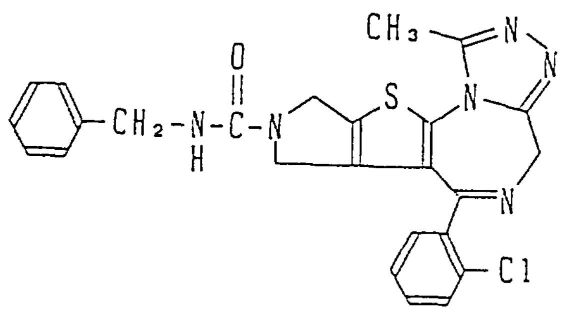

$^1$H-NMR($CDCl_3$) δ :
2.68(s,3H), 3.60-3.94(m,2H), (4.36(d,J=5.4Hz,2H),
4.50-5.08(m,4H), 7.04-7.50(m,10H)
MS m/z: 489

Example 101

3-(n-hexyl)oxycarbonyl-5-(2-chlorophenyl)-10-methyl-2,4-dihydro-2H,7H-pyrrolo[4',3':4,5]thieno[3,2-f] [1,2,4]triazolo [4,3-a][1,4]diazepine

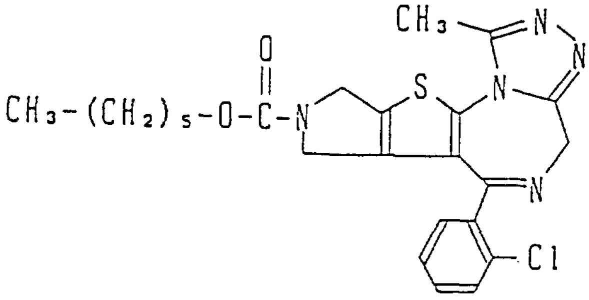

$^1$H-NMR($CDCl_3$) δ :
0.75-1.04(m,3H), 1.10-1.80(m,8H), 2.72(s,3H),
3.54-3.84(m,2H), 4.02(t,J=7Hz,1H), 4.08(t,J=7Hz,1H).
4.58-4.80(m,2H), 4.78-5.10(m,2H), 7.20-7.48(m,4H)
MS m/z: 484

Example 102

3-Phenetyloxycarbonyl-5-(2-chlorophenyl)-10-methyl-2,4-dihydro-2H,7H-pyrrolo[4',3':4,5]thieno[3,2-f] [1,2,4]triazolo [4,3-a][1,4]diazepine

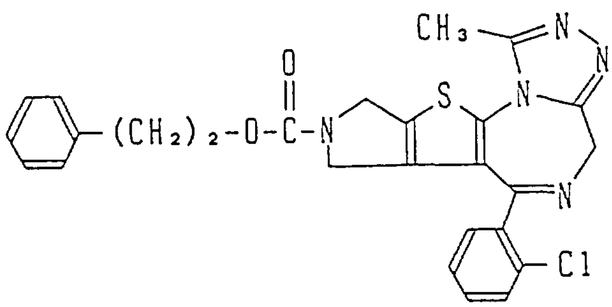

$^1$H-NMR($CDCl_3$) δ :
2.72(s,3H), 2.86(t,J=7Hz,1H), 2.94(t,J=7Hz,1H),
3.55-3.84(m,2H), 4.24(t,J=7Hz,1H), 4.30(t,J=7Hz,1H),
4.50-4.75(m,2H), 4.78-5.10(m,2H), 6.98-7.50(m,9H)
MS m/z: 504

Example 103

3-(4'-Chlorobenzyl)oxycarbonyl-5-(2-chlorophenyl)-10-methyl-2,4-dihydro-2H,7H-pyrrolo[4',3':4,5]thieno[3,2-f] [1,2,4] triazolo[4,3-a][1,4]diazepine

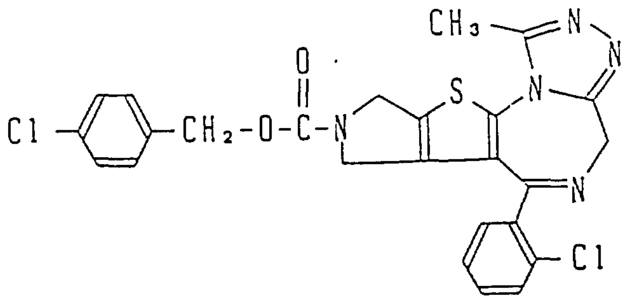

$^1$H-NMR($CDCl_3$) δ :
2.72(s,3H), 3.60-3.92(m,2H), 4.60-4.80(m,2H), 4.80-5.04(m,2H), 5.08(s,2H), 7.26(ABq,J=6Hz,4H), 7.10-7.46(m,4H)
MS m/z: 524

Example 104

3-(1'-Cyano-1-'-methylethyl)oxycarbonyl-5-(2-chlorophenyl)-10-methyl-2,4-dihydro-2H,7H-pyrrolo[4',3':4,5]thieno [3,2-f][1,2,4]triazolo-[4,3-a][1,4]diazepine

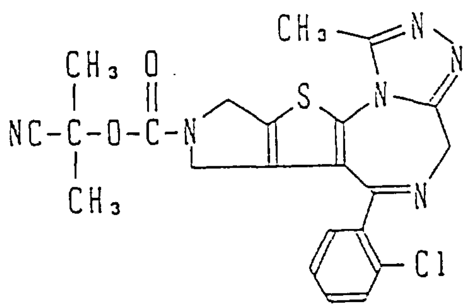

$^1$H-NMR($CDCl_3$) δ :
1.52(s,3H), 1.80(s,3H), 2.72(s,3H), 3.50-3.86(m,2H), 4.56-4.76(m,2H), 4.78-5.10(m,2H), 7.20-7.45(m,4H)
MS m/z: 467

Example 105

3-(3'-Nitrobenzyl)oxycarbonyl-5-(2-chlorophenyl)-10-methyl-2,4-dihydro-2H,7H-pyrrolo[4',3':4,5]-thieno[3,2-f][1,2,4]triazolo[4,3-a][1,4]diazepine

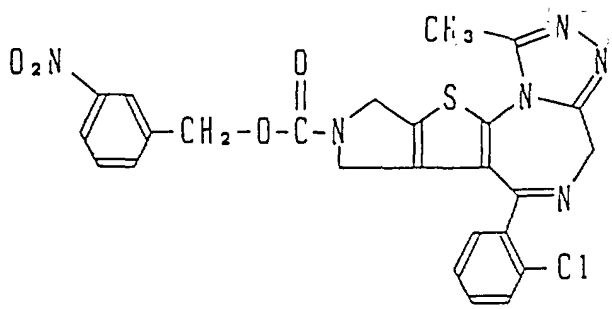

$^1$H-NMR($CDCl_3$) δ :
2.72(s,3H), 3.66-3.88(m,2H), 4.67-4.82(m,2H),
4.82-5.08(m,2H), 5.18(d,J=2.8Hz, ), 7.20-7.72(m,6H), 7.96-8.24(m,2H)
MS m/z: 535

Example 106

3-(4'-Trifluoromethylbenzyl)oxycarbonyl-5-(2-chlorophenyl)-10-methyl-2,4-dihydro-2H,7H-pyrrolo[4',3':4,5]thieno[3,2-f][1,2,4]triazolo-[4,3-a][1,4]diazepine

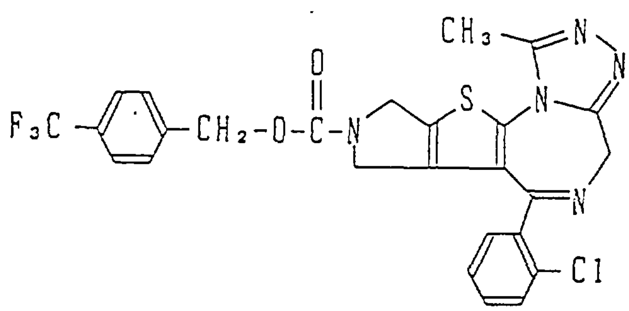

$^1$H-NMR($CDCl_3$)δ :
2.76(s,3H), 3.70-3.92(m,2H), 4.70-4.86(m,2H),
4.88-5.10(m,2H), 5.22(s,2H), 7.25-7.60(m,4H),
7.56(ABq,J=7Hz,4H)
MS m/z: 558

## Claims

**1.** A triazolo-1,4-diazepine compounds of the formula (I) and a pharmacologically acceptable salt thereof

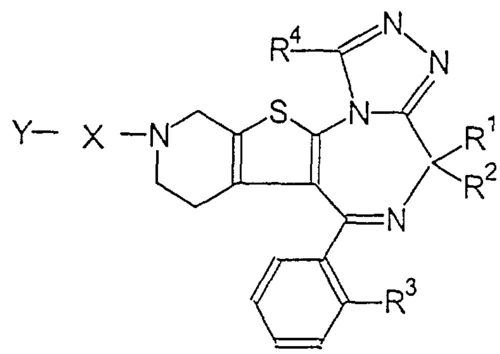

(I)

wherein $R^1$ and $R^2$ independently represent a hydrogen atom or a $C_1$-$C_6$ alkyl group, under the proviso that compounds wherein $R^1$ is hydrogen and $R^2$ is methyl are excluded, $R^3$ represents a hydrogen atom or a halogen atom, $R^4$ represents a hydrogen atom or a $C_1$-$C_6$ alkyl group, X represents

(a) a group of the formula,

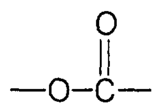

(b) a group of the formula,

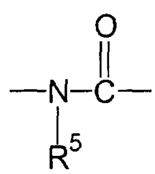

wherein $R^5$ represents a hydrogen atom or a $C_{1-6}$ alkyl group,

(c) a group of the formula,

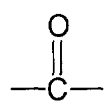

or

(d) a group of the formula,

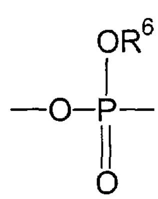

(wherein $R^6$ represents a $C_1$-$C_6$ alkyl group) and Y represents

(1) a $C_3$-$C_7$ cycloalkyl group which may have a methyl group as a substituent(s),

(2) a $C_3$-$C_7$ cycloalkyl-$C_1$-$C_6$ alkyl,

(3) an alkynyl group having up to 6 carbon atoms,

(4) a group of the formula

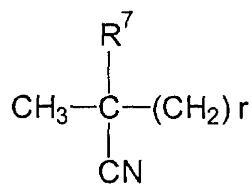

in which $R^7$ is hydrogen or methyl and r is zero, 1 or 2,

(5) a group of the formula NC-$(CH_2)_p$- (wherein p is an integer of from 1 to 6),

(6) a group of the formula A-$(CH_2)_q$- (wherein A represents a group selected from a pyridyl group, a pyranyl group and a morpholino group and q is an integer of from 0 to 6),

(7) an alkynyl group having up to 6 carbon atoms wherein a phenyl group or a $C_3$-$C_7$ cycloalkyl group is joined to any carbon atom,

(8) a group of the formula

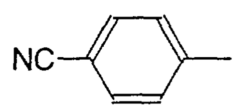

(9) a group of the formula

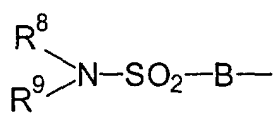

wherein $R^8$ and $R^9$ are the same or different and represent a hydrogen atom, a $C_1$-$C_6$ alkyl group, a pyridylmethyl group or a $C_3$-$C_7$ cycloalkyl group or $R^8$ and $R^9$ may be joined to form a morpholino, piperidino, pyrrolidino, azepano, imidazolyl or pyrrolyl ring, and B represents a phenylene group or a $C_1$-$C_3$ alkylene group,

(10) a group of the formula

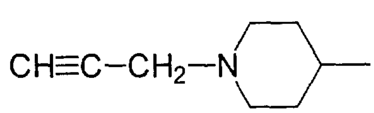

(11) a group of the formula

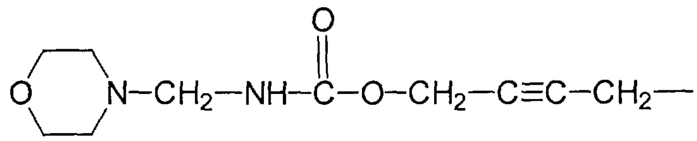

(12) a group of the formula

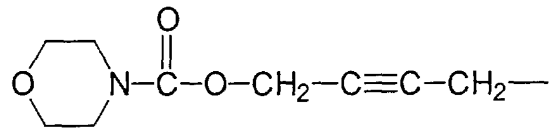

(13) a group of the formula

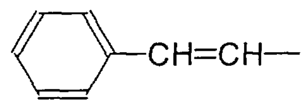

(14) a group of the formula

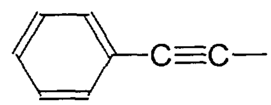

(15) a $C_1$-$C_6$ alkyl group, or

(16) a $C_3$-$C_7$ cycloalkyl-alkenyl group, wherein the alkenyl group has upto 6 C-atoms with the following proviso

when X is

(a)

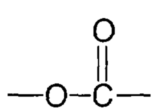

,

(b)

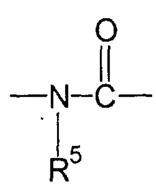

or

(c)

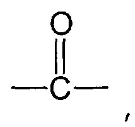

Y is selected from (1) to (14) or (16), and

when X is (d)

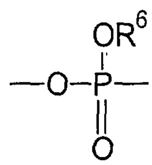

Y is selected from (15).

**2.** A triazolo-1,4-diazepine compound of the formula given below

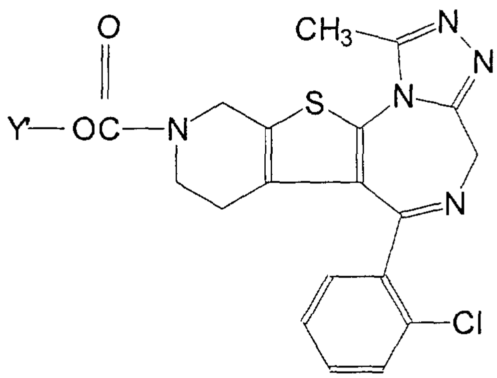

wherein Y' is selected from

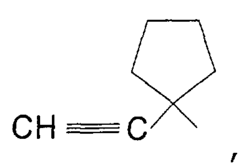

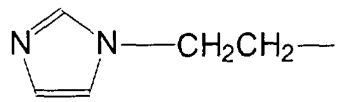

,

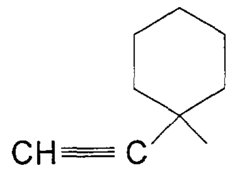

,

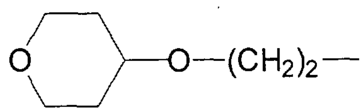

or

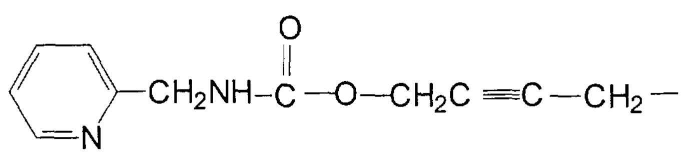

3. A triazolo-1,4-diazepine compound of the formula given below

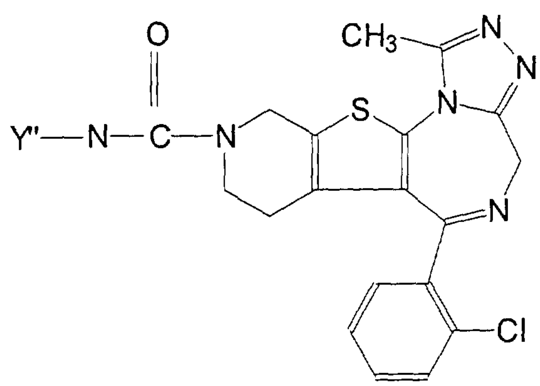

wherein Y" is selected from

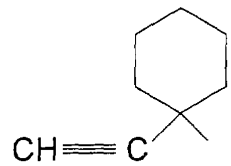

,

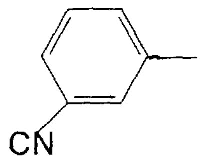

or

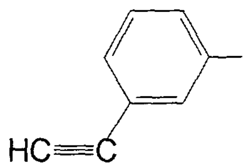

**4.** A triazolo-1,4-diazepine compound of the formula given below

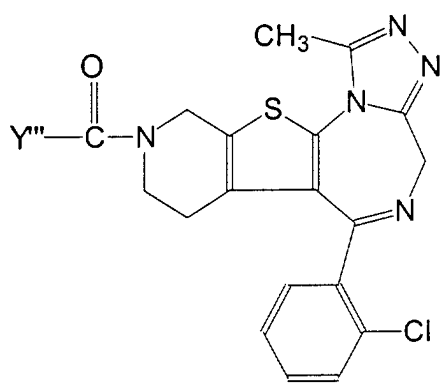

,

wherein Y'" is selected from

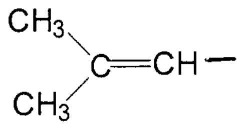

,

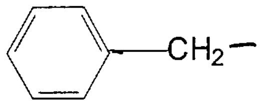

,

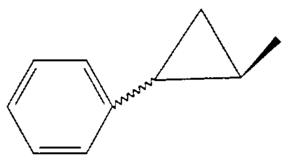

,

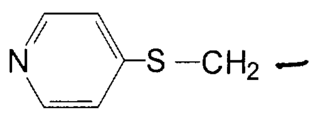

,

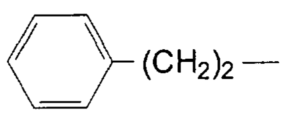

,

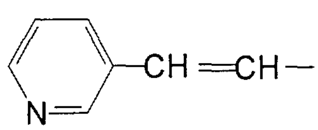

,

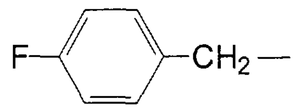

or

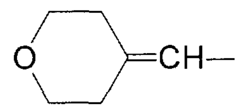
.

5. The compound and the salt as claimed in claim 1, in which X is (c) [-CO-].

6. The compound and the salt as claimed in claim 1, in which X is (a) [-COO-].

7. The compound and the salt as claimed in claim 1, in which X is (b) [-$NR^5$-CO-].

8. The compound and the salt as claimed in any one of claims 1 or 5-7 in which Y is a $C_3$-$C_7$ cycloalkyl.

9. The compound and the salt as claimed in any one of claims 1 or 5-7 in which Y is cyclopropyl.

10. The compound and the salt as claimed in claim 1 in which Y is one of (4), (3), (16) and (2).

11. The compound and the salt as claimed in claim 1 in which Y is

$$HC \equiv C—C(CH_3)_2—$$

12. The compound and the salt as claimed in claim 1 in which $R^3$ is chlorine, $R^1$ and $R^2$ are hydrogen, $R^4$ is methyl, and Y-Xis defined as follows:

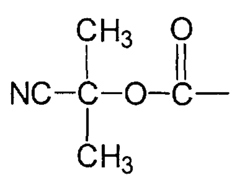

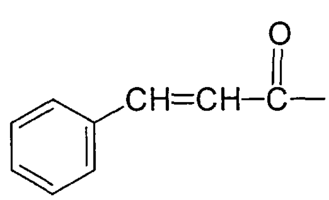

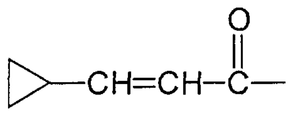

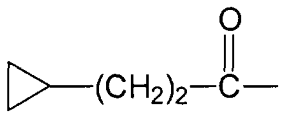

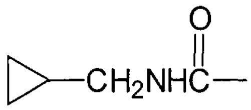

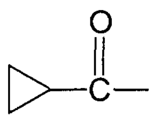

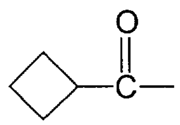

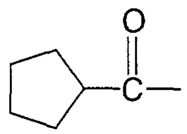

**13.** A triazolo-1,4-di-azepine compound of the formula (II) and a pharmacologically acceptable salt thereof

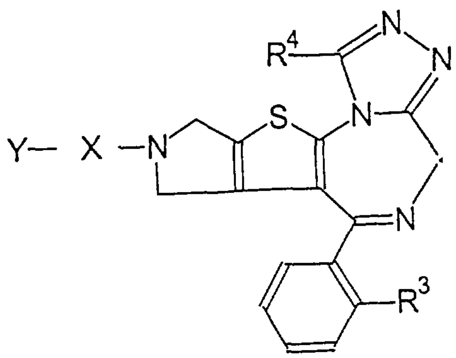

(II)

wherein $R^3$ represents a halogen atom, $R^4$ represents a $C_{1-6}$alkyl group, X represents

(a) a group of the formula,

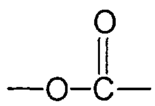

and Y represents

(2) a $C_3$-$C_7$ cycloalkyl-$C_1$-$C_6$ alkyl,

(3) an alkynyl group having up to 6 carbon atoms,

(4) a group of the formula

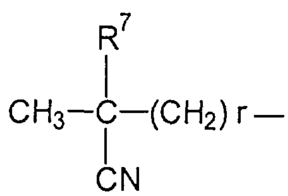

in which $R^7$ is hydrogen or methyl and r is zero, 1 or 2,

(5) a group of the formula NC-$(CH_2)_p$- (wherein p is an integer of from 1 to 6), or

(6) a group of the formula A-$(CH_2)_q$- (wherein A represents a group selected from a pyridyl group, a pyranyl group and a morpholino group and q is an integer of from 0 to 6).

**14.** A triazolo-1,4-di-azepine compound according to claim 13, having the following formula:

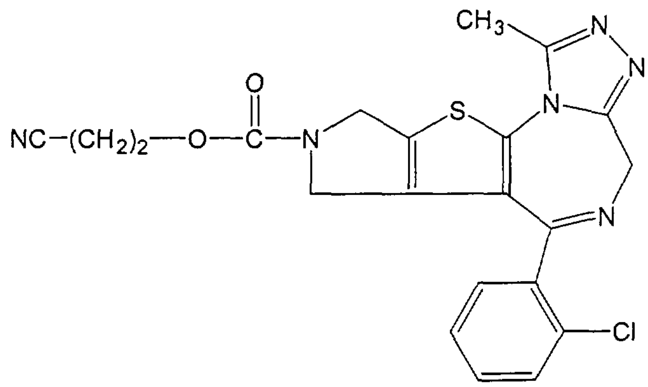

**15.** A triazolo-1,4-di-azepine compound according to claim 13, having the following formula:

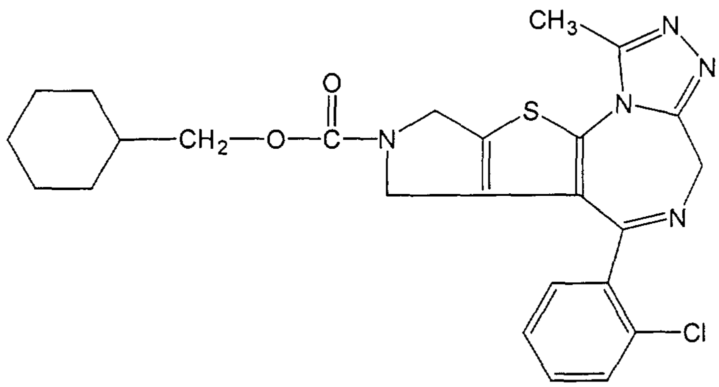

**16.** A triazolo-1,4-di-azepine compound according to claim 13, having the following formula:

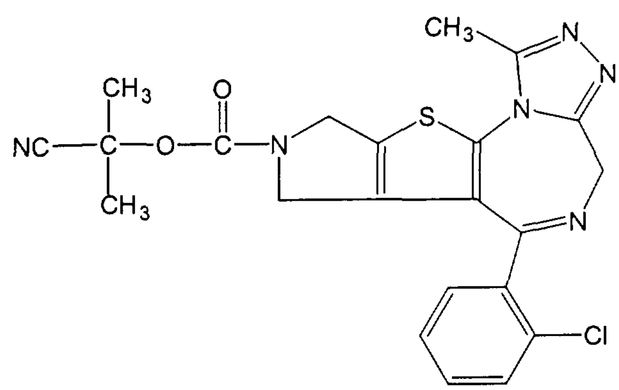

**17.** A triazolo-1,4-diazepine compound of the following formula:

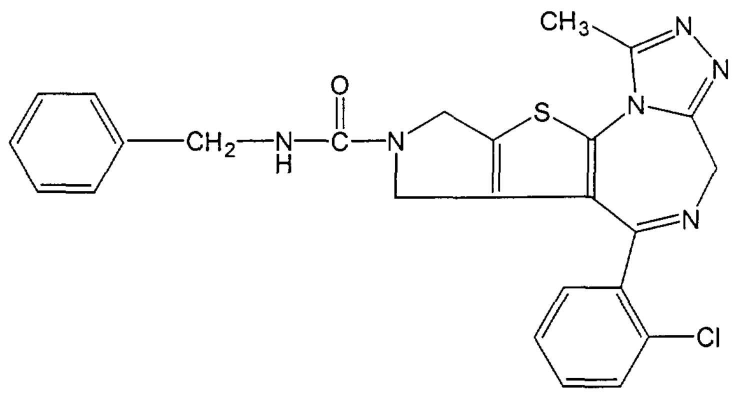

**18.** A triazolo-1,4-di-azepine compound having the following formula:

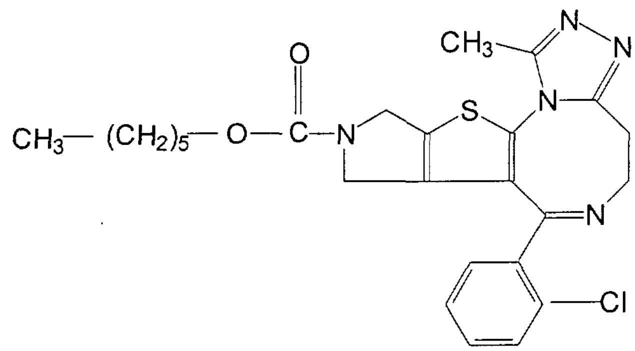

**19.** A triazolo-1,4-di-azepine compound having the following formula:

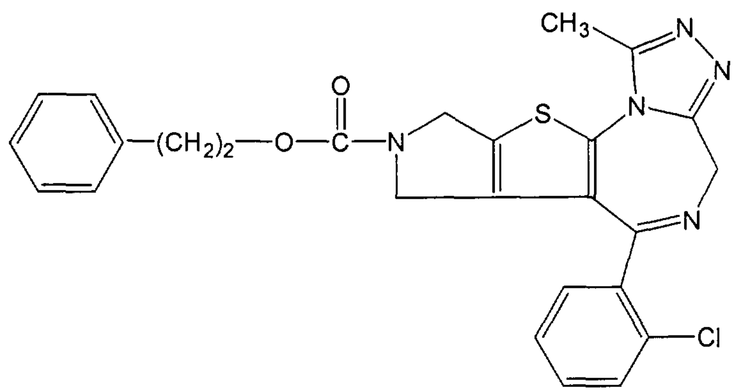

**20.** A triazolo-1,4-diazepine compound of the following formula:

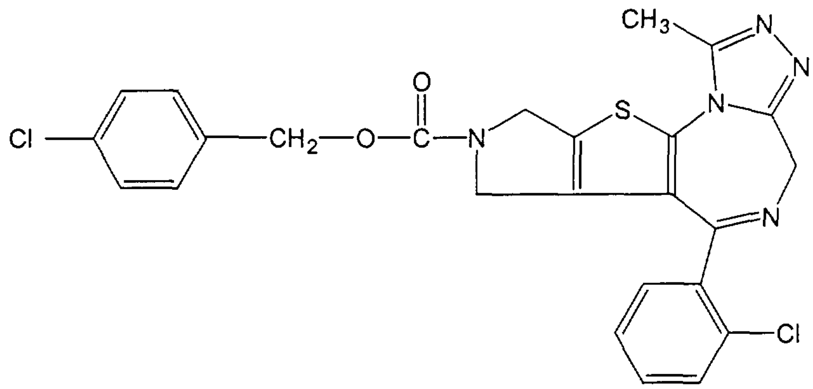

**21.** A triazolo-1,4-diazepine compound of the following formula:

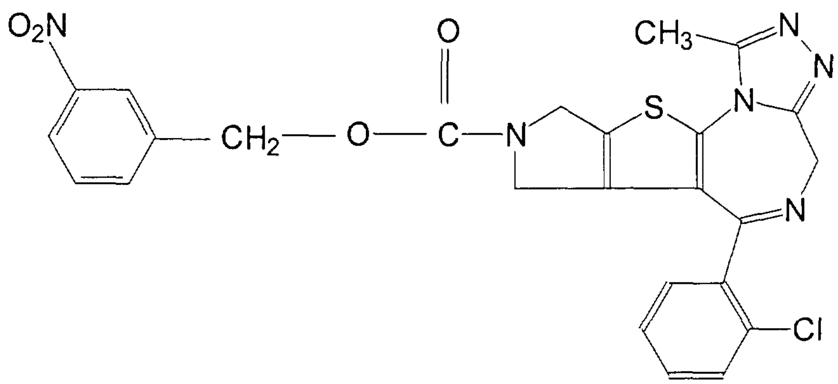

**22.** A triazolo-1,4-diazepine compound of the following formula

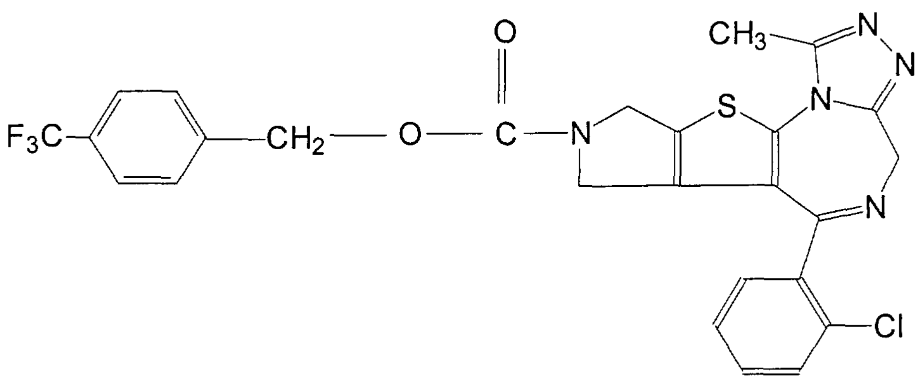

**23.** A pharmaceutical composition which comprises a pharmacologically effective amount of a compound as defined in claims 1-22 or a salt thereof and a pharmacologically acceptable carrier.

**24.** The use of a compound or a salt thereof as defined in any of preceding claims 1-22 for making a medicament for treatment of a disease against which anti-PAF activity is effective.

**25.** The use as claimed in claim 24 in which the disease is an allergic disease.

**26.** The use as claimed in claim 24 in which the disease is asthma.

## Patentansprüche

**1.** Triazol-1,4-diazepin-Verbindungen der Formel (I) und deren pharmakologisch annehmbaren Salze:

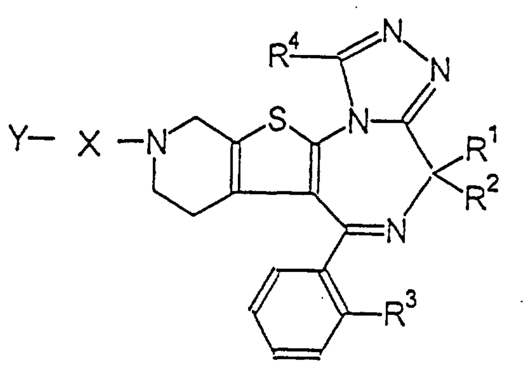

(I)

worin $R^1$ und $R^2$ unabhängig voneinander ein Wasserstoffatom oder eine $C_{1-6}$-Alkyl-Gruppe bedeuten, unter der Voraussetzung, daß Verbindungen worin $R^1$ Wasserstoff ist und $R^2$ Methyl ist ausgenommen sind, $R^3$ ein Wasserstoffatom oder ein Halogenatom bedeutet, $R^4$ ein Wasserstoffatom oder eine $C_{1-6}$-Alkyl-Gruppe bedeutet, X die folgenden Bedeutungen hat:

(a) eine Gruppe der Formel:

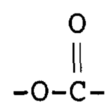

(b) eine Gruppe der Formel:

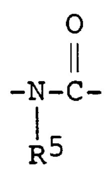

worin $R^5$ ein Wasserstoffatom oder eine $C_{1-6}$-Alkyl-Gruppe bedeutet,

(c) eine Gruppe der Formel:

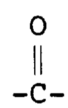

oder

(d) eine Gruppe der Formel:

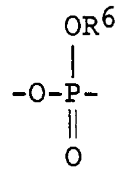

(worin $R^6$ eine $C_{1-6}$-Alkyl-Gruppe bedeutet) und Y die folgenden Bedeutungen hat:

(1) eine $C_{3-7}$-Cycloalkyl-Gruppe, die (eine) Methyl-Gruppe(n) als (einen) Substituenten aufweisen kann,

(2) ein $C_{3-7}$-Cycloalkyl-$C_{1-6}$-alkyl,

(3) eine Alkinyl-Gruppe mit bis zu 6 Kohlenstoffatomen,

(4) eine Gruppe der Formel:

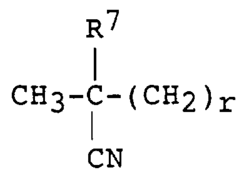

in der $R^7$ Wasserstoff oder Methyl ist und r 0, 1 oder 2 ist,

(5) eine Gruppe der Formel $NC-(CH_2)_p-$ (worin p eine ganze Zahl von 1 bis 6 ist),

(6) eine Gruppe der Formel $A-(CH_2)_q-$ (worin A eine Gruppe darstellt, die unter einer Pyridyl-Gruppe, einer Pyranyl-Gruppe und einer Morpholino-Gruppe ausgewählt wird, und q eine ganze Zahl von 0 bis 6 ist),

(7) eine Alkinyl-Gruppe mit bis zu 6 Kohlenstoffatomen, worin eine Phenyl-Gruppe oder ein $C_{3-7}$-Cycloalkyl-Gruppe mit irgendeinem Kohlenstoffatom verknüpft ist,

(8) eine Gruppe der Formel:

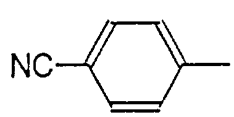

(9) eine Gruppe der Formel:

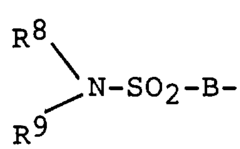

worin $R^8$ und $R^9$ gleich oder verschieden sind und ein Wasserstoffatom, eine $C_{1-6}$-Alkyl-Gruppe, eine Pyridylmethyl-Gruppe oder eine $C_{3-7}$-Cycloalkyl-Gruppe bedeuten oder $R^8$ und $R^9$ unter Bildung eines Morpholino-, Piperidino-, Pyrrolidino-, Azepano-, Imidazolyl- und Pyrrolyl-Rings miteinander verbunden sein können, und B eine Phenylen-Gruppe oder eine $C_{1-3}$-Alkylen-Gruppe darstellt,

(10) eine Gruppe der Formel:

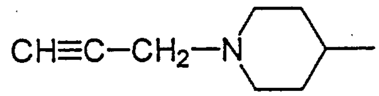

(11) eine Gruppe der Formel:

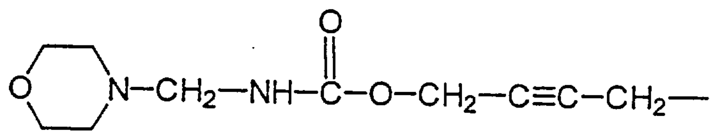

(12) eine Gruppe der Formel:

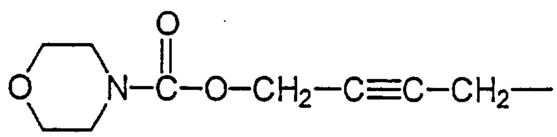

(13) eine Gruppe der Formel:

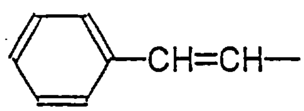

(14) eine Gruppe der Formel:

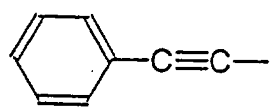

(15) eine $C_{1-6}$-Alkyl-Gruppe oder

(16) eine $C_{3-7}$-Cycloalkyl-alkenyl-Gruppe, worin die Alkenyl-Gruppe bis zu 6 Kohlenstoffatome hat, unter der folgenden Voraussetzung:
wenn X

(a)

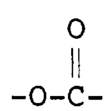

(b)

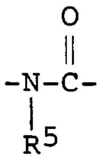

oder

(c)

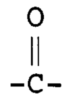

ist, wird Y unter (1) bis (14) oder (16) ausgewählt, und wenn X (d) ist

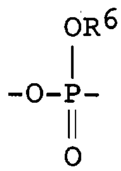

wird Y unter (15) ausgewählt.

**2.** Triazol-1,4-diazepin-Verbindung der nachstehend angegebenen Formel:

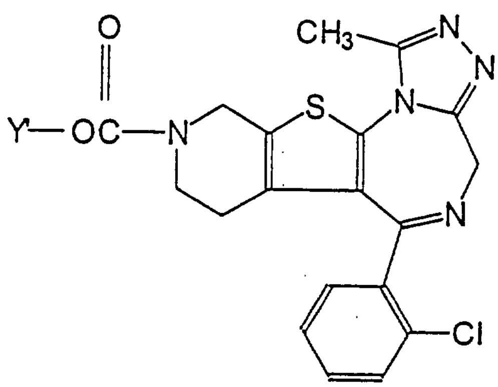

worin Y' ausgewählt unter:

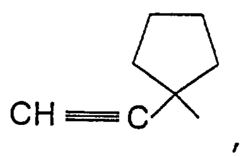

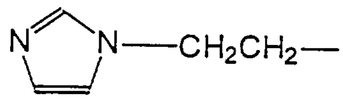

,

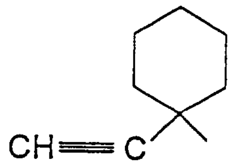

,

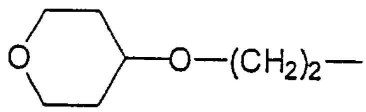

oder

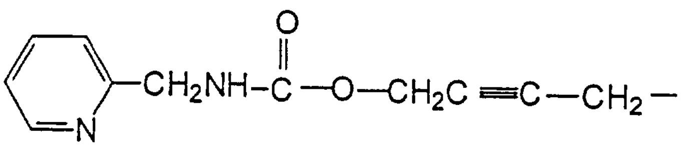

.

3. Triazol-1,4-diazepin-Verbindung der nachstehend angegebenen Formel:

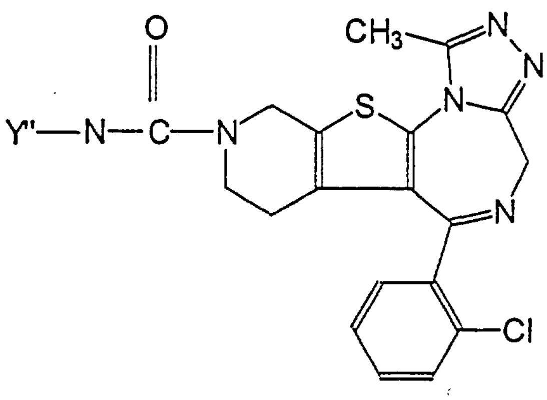

worin Y" ausgewählt wird unter:

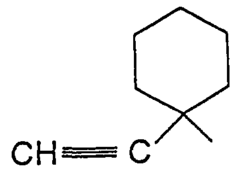
,

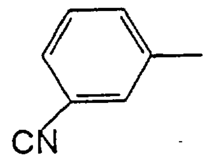

oder

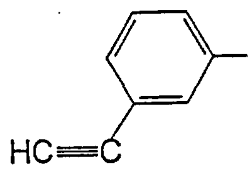
,

**4.** Triazol-1,4-diazepin-Verbindung der nachstehend angegebenen Formel:

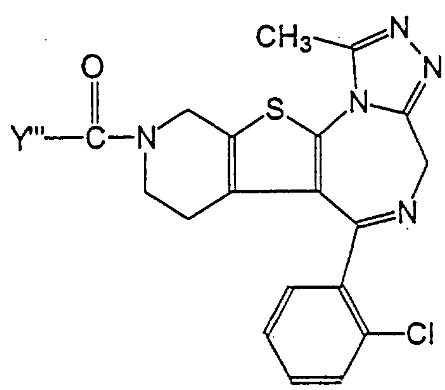

worin Y''' ausgewählt wird unter:

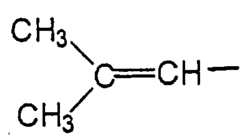
,

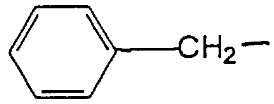

,

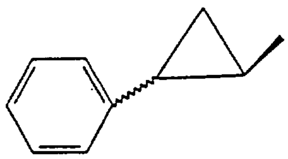

,

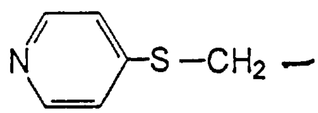

,

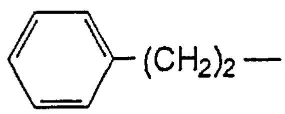

,

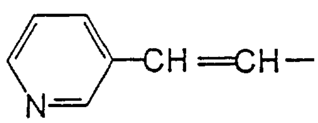

,

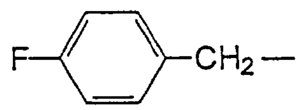

oder

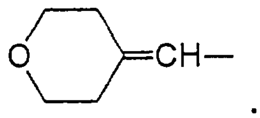

**5.** Die Verbindung und das Salz, wie sie in Anspruch 1 definiert sind, worin X (c) [-CO-] ist.

**6.** Die Verbindung und das Salz, wie sie in Anspruch 1 definiert sind, worin X (a) [-COO-] ist.

**7.** Die Verbindung und das Salz, wie sie in Anspruch 1 definiert sind, worin X (b) [-$NR^5$-CO-] ist.

**8.** Die Verbindung und das Salz, wie sie in einem der Ansprüche 1 oder 5 bis 7 definiert sind, worin Y ein $C_{3-7}$-Cycloalkyl ist.

**9.** Die Verbindung und das Salz, wie sie in einem der Ansprüche 1 oder 5 bis 7 definiert sind, worin Y Cyclopropyl ist.

**10.** Die Verbindung und das Salz, wie sie in Anspruch 1 definiert sind, worin Y eine der Bedeutungen (4), (3), (16) und (2) hat.

**11.** Die Verbindung und das Salz, wie sie in Anspruch 1 definiert sind, worin Y

$$HC{\equiv}C\text{-}C(CH_3)_2\text{-}$$

ist.

**12.** Die Verbindung und das Salz, wie sie in Anspruch 1 definiert sind, worin $R^3$ Chlor ist, $R^1$ und $R^2$ Wasserstoff sind, $R^4$ Methyl ist, und Y-X- wie folgt definiert ist:

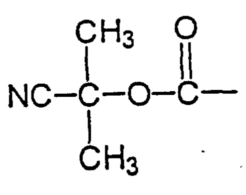

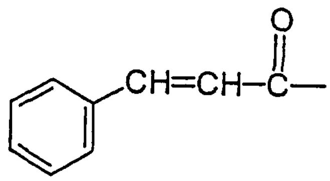

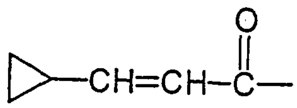

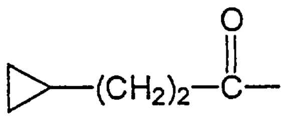

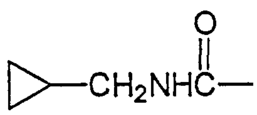

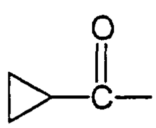

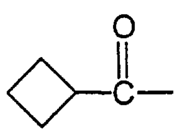

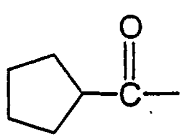

**13.** Triazol-1,4-diazepin-Verbindungen der Formel (II) und deren pharmakologisch annehmbaren Salze:

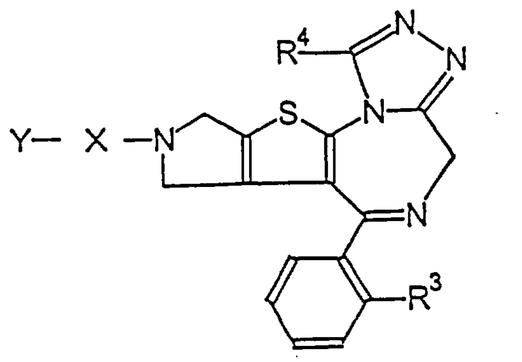

(II)

worin $R^3$ ein Halogenatom bedeutet, $R^4$ eine $C_{1-6}$-Alkyl-Gruppe bedeutet, X

(a) eine Gruppe der Formel:

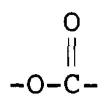

bedeutet und Y die folgenden Bedeutungen hat:

(2) ein $C_{3-7}$-Cycloalkyl-$C_{1-6}$-alkyl,

(3) eine Alkinyl-Gruppe mit bis zu 6 Kohlenstoffatomen,

(4) eine Gruppe der Formel:

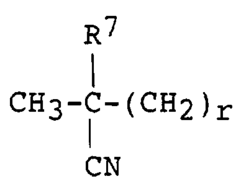

in der $R^7$ Wasserstoff oder Methyl ist und r 0, 1 oder 2 ist,

(5) eine Gruppe der Formel NC-$(CH_2)_p$- (worin p eine ganze Zahl von 1 bis 6 ist),

(6) eine Gruppe der Formel A-$(CH_2)_q$- (worin A eine Gruppe darstellt, die unter einer Pyridyl-Gruppe, einer Pyranyl-Gruppe und einer Morpholino-Gruppe ausgewählt wird, und q eine ganze Zahl von 0 bis 6 ist).

**14.** Eine Triazolo-1,4-diazepin-Verbindung gemäß Anspruch 13 mit der folgenden Formel:

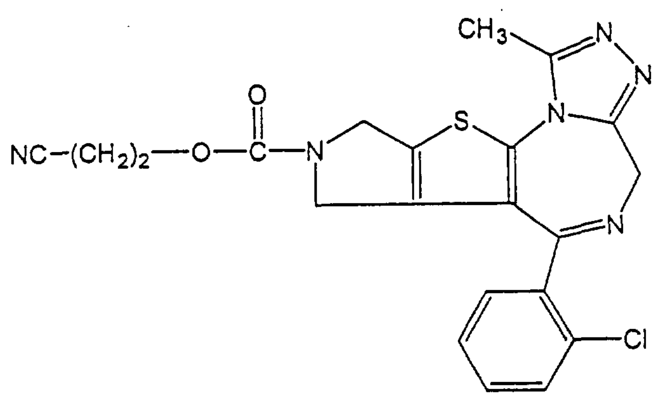

**15.** Eine Triazolo-1,4-diazepin-Verbindung gemäß Anspruch 13 mit der folgenden Formel:

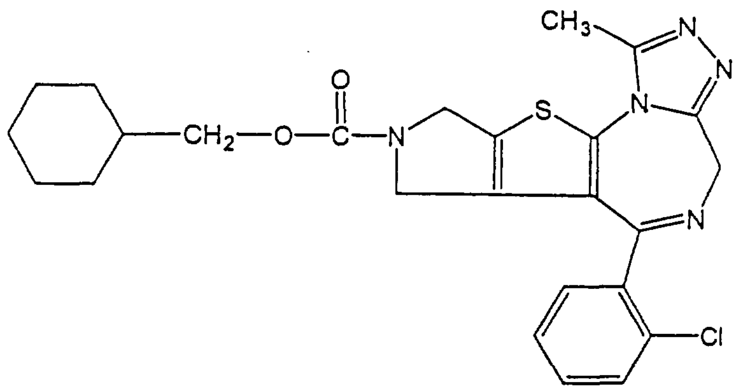

**16.** Eine Triazolo-1,4-diazepin-Verbindung gemäß Anspruch 13 mit der folgenden Formel:

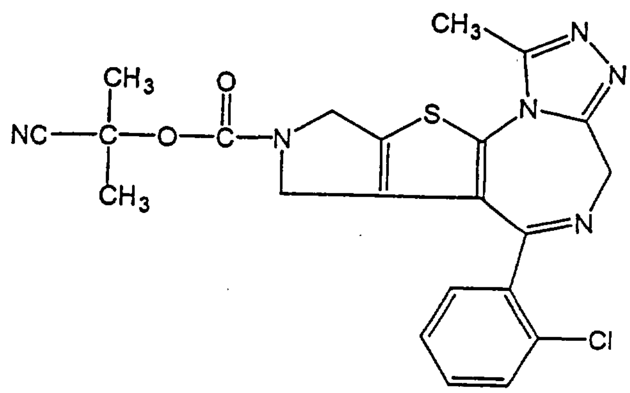

**17.** Triazol-1,4-diazepin-Verbindung der folgenden Formel:

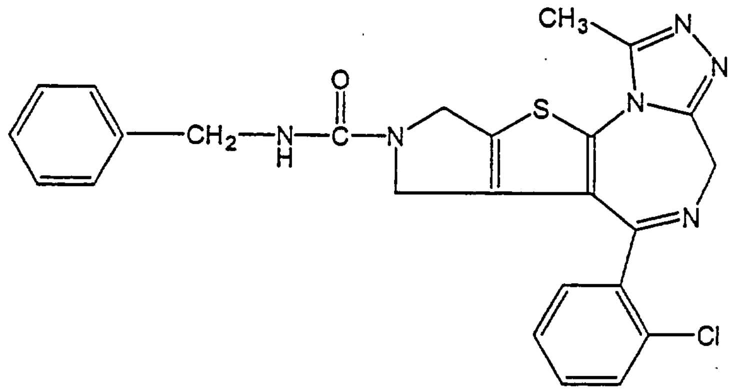

**18.** Triazol-1,4-diazepin-Verbindung der folgenden Formel:

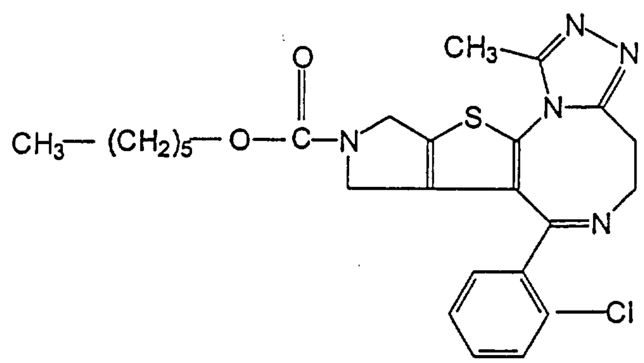

**19.** Triazol-1,4-diazepin-Verbindung der folgenden Formel:

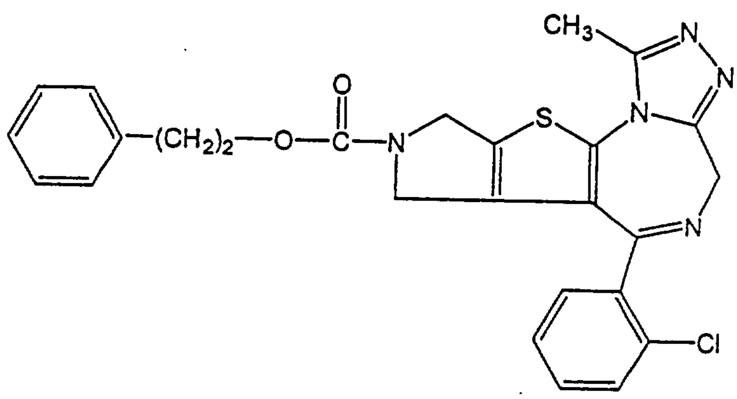

**20.** Triazol-1,4-diazepin-Verbindung der folgenden Formel:

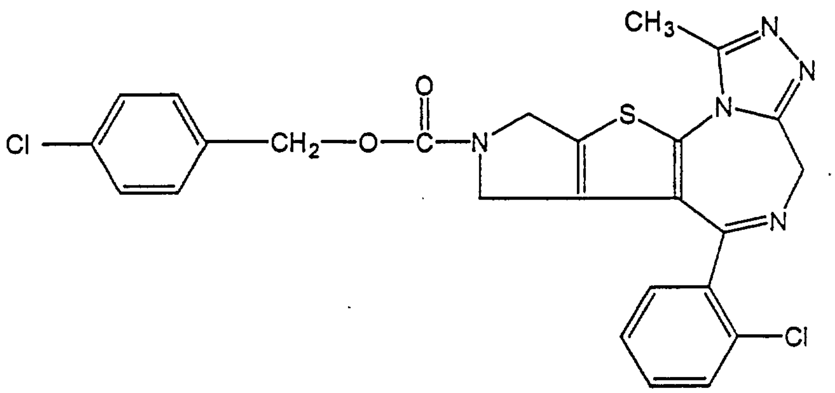

21. Triazol-1,4-diazepin-Verbindung der folgenden Formel:

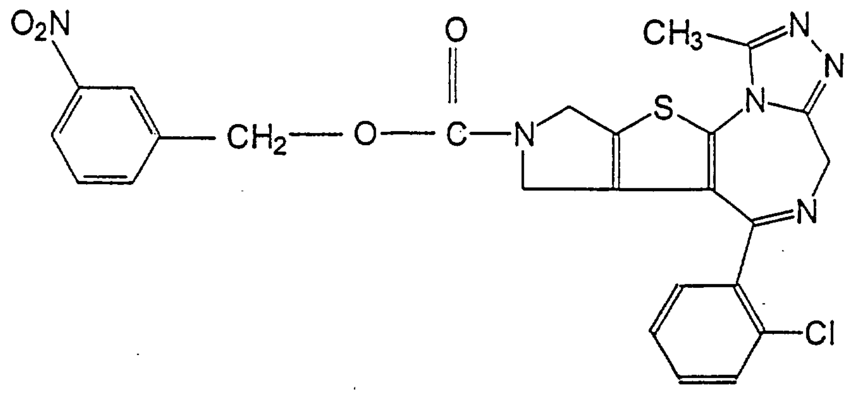

22. Triazol-1,4-diazepin-Verbindung der folgenden Formel:

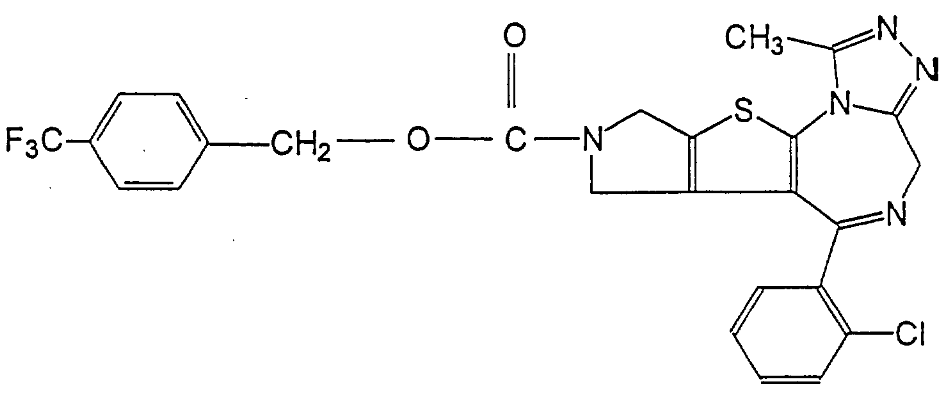

23. Eine pharmazeutische Zusammensetzung, die eine pharmakologisch wirksame Menge einer Verbindung, wie sie in einem der Ansprüche 1 bis 22 definiert ist oder ein Salz hiervon und einen pharmakologisch annehmbaren Träger umfaßt.

24. Die Verwendung einer Verbindung oder eines Salzes hiervon, wie sie in einem der vorangehenden Ansprüche 1 bis 22 definiert ist, zur Herstellung einer Medikaments zur Behandlung einer Krankheit, gegen welche Anti-PAF-Aktivität wirksam ist.

**25.** Verwendung gemäß Anspruch 24, worin die Krankheit eine allergische Krankheit ist.

**26.** Verwendung gemäß Anspruch 24, worin die Krankheit Asthma ist.

## Revendications

**1.** Dérivés triazolo-1,4-diazépines de formule (I) et sels pharmacologiquement acceptables de ceux-ci de formule :

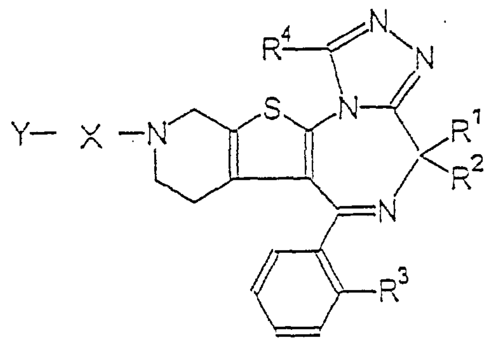

(I)

dans laquelle $R^1$ et $R^2$ représentent indépendamment un atome d'hydrogène ou un groupe alkyle en $C_1$-$C_6$, à condition que les composés dans lesquels $R^1$ est un atome d'hydrogène et $R^2$ est méthyle soient exclus, $R^3$ représente un atome d'hydrogène ou un atome d'halogène,$R^4$ représente un atome d'hydrogène ou un groupe alkyle en $C_1$-$C_6$, X représente :

- (a) un groupe de formule :

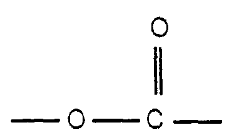

- (b) un groupe de formule :

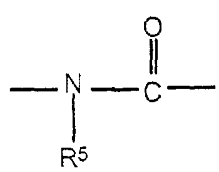

où $R^5$ représente un atome d'hydrogène ou un groupe alkyle en $C_1$-$C_6$ ;

- (c) un groupe de formule :

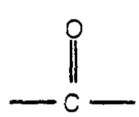

ou
- (d) un groupe de formule :

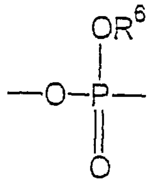

(dans laquelle $R^6$ représente un groupe alkyle en $C_1$-$C_6$) et Y représente :

(1) un groupe cycloalkyle en $C_3$-$C_7$ qui peut avoir un groupe méthyle comme substituant ;

(2) un groupe ($C_3$-$C_7$)cycloalkyl-($C_1$-$C_6$)alkyle ;

(3) un groupe alcynyle ayant jusqu'à 6 atomes de carbone ;

(4) un groupe de formule :

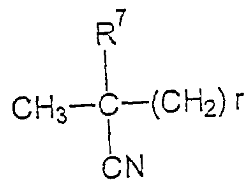

dans lequel $R^7$ est hydrogène ou méthyle et r est 0,1 ou 2 ;

(5) un groupe de formule NC-$(CH_2)_p$- (dans laquelle p est un entier de 1 à 6) ;

(6) un groupe de formule A-$(CH_2)$q- (dans laquelle A représente un groupe choisi parmi un groupe pyridyle, un groupe pyranyle et un groupe mopholino et q est un entier de 0 à 6),

(7) un groupe alcynyle ayant jusqu'à 6 atomes de carbone dans lequel un groupe phényle ou un groupe ($C_3$-$C_7$)cycloalkyle est attaché à l'un quelconque des atomes de carbone,

(8) un groupe de formule :

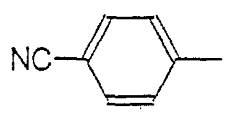

(9) un groupe de formule :

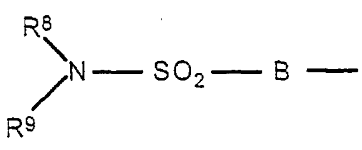

dans lequel $R^8$ et $R^9$ identiques ou différents représentent un atome d'hydrogène, un groupe ($C_1$-$C_6$) alkyle, un groupe pyridylméthyle ou un groupe ($C_3$-$C_7$)cycloalkyle ou $R^8$ et $R^9$ peuvent être rattachés ensemble pour former morpholino, pipéridino, pyrrolidino, azépano, imidazoyle ou pyrrolyle, et B représente un groupe phénylène ou un groupe ($C_1$-$C_3$)alkylène,

(10) un groupe de formule :

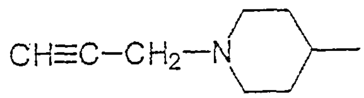

(11) un groupe de formule :

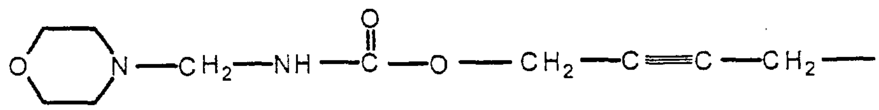

(12) un groupe de formule :

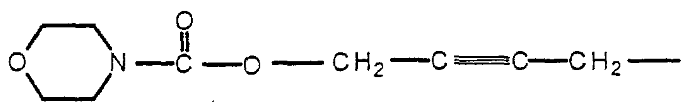

(13) un groupe de formule :

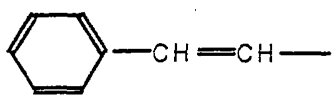

(14) un groupe de formule :

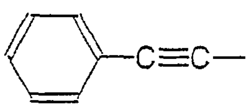

(15) un groupe alkyle en $C_1$-$C_6$

(16) un groupe ($C_3$-$C_7$)cycloalkyle-alcényle où alcényle a jusqu'à 6 atomes de carbone ;
à condition que
lorsque X est

(a)

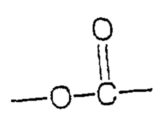

(b)

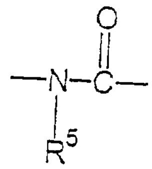

ou

(c)

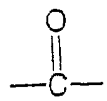

Y est choisi parmi (1) à (14) ou (15) et
lorsque X est (d)

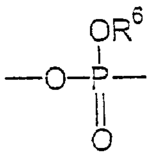

Y est choisi parmi (15)

**2.** Dérivé triazolo-1,4-diazépine de formule :

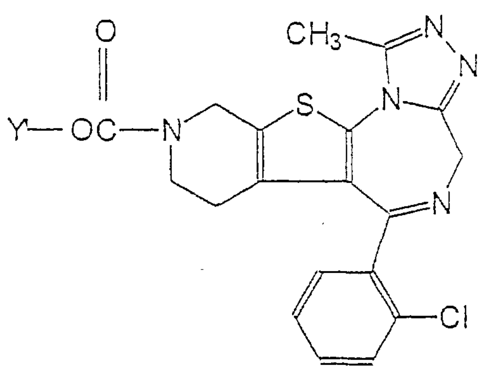

dans lequel Y' est choisi parmi :

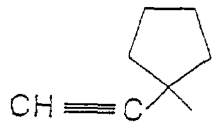

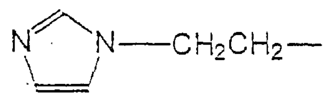

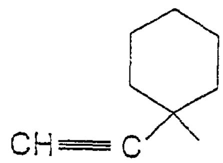

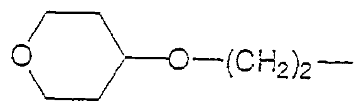

or

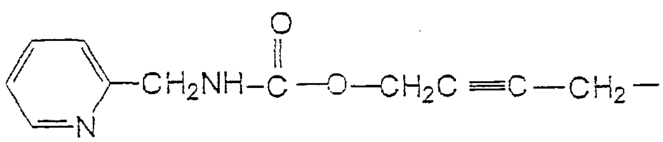

**3.** Dérivé triazolo-1,4-diazépine de formule :

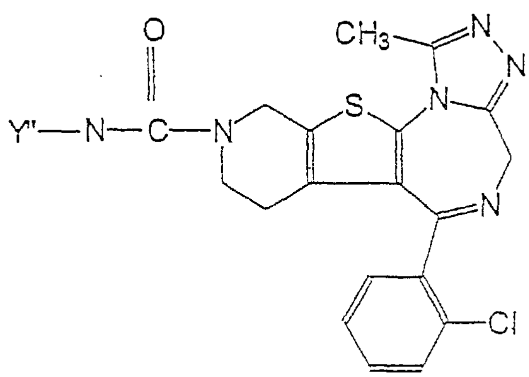

dans laquelle Y" est choisi parmi :

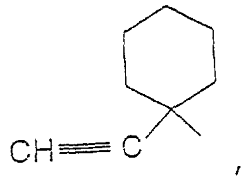

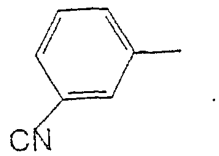

or

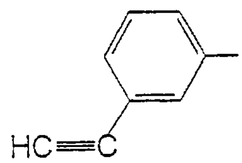

**4.** Dérivé triazolo-1,4-diazépine de formule :

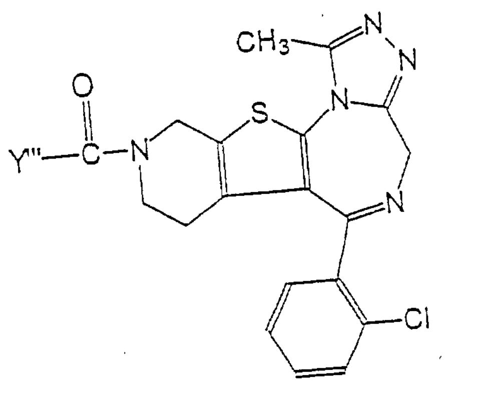

dans laquelle y''' est choisi parmi :

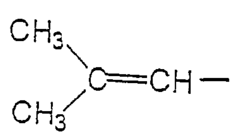

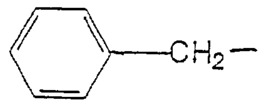

,

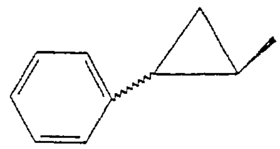

,

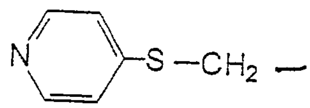

,

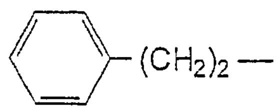

,

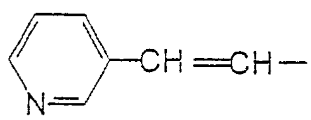

,

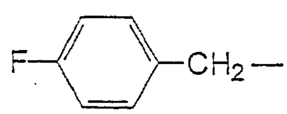

or

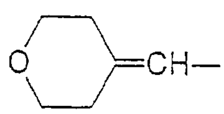

**5.** Composé et sel selon la revendication 1, dans lequel X est (c), [-CO-].

**6.** Composé et sel selon la revendication 1, dans lequel X est (a) [-COO-].

**7.** Composé et sel selon la revendication 1, dans lequel X est (b) [$-NR^5-CO-$]

**8.** Composé et sel selon l'une quelconque des revendications 1 ou 5 à 7 dans lequel Y est un groupe cycloalkyle en $C_3$-$C_7$.

**9.** Composé et sel selon l'une quelconque des revendications 1 ou 5 à 7 dans lequel Y est un groupe cyclopropyle.

**10.** Composé et sel selon la revendication 1 dans lequel Y est l'un quelconque de (4), (3), (16) et (2).

**11.** Composé et sel selon la revendication 1 dans lequel Y est

$$HC{\equiv}C\text{-}C(CH_3)_2\text{—}$$

**12.** Composé et sel selon la revendication 1 dans lequel $R^3$ est un atome de chlore, $R^1$ et $R^2$ sont un atome d'hydrogène, $R^4$ est un groupe méthyle et Y-X- est tel que défini ci-dessous :

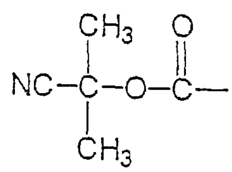

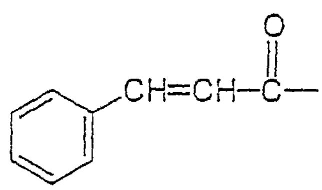

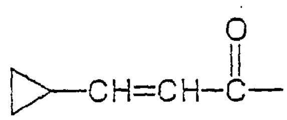

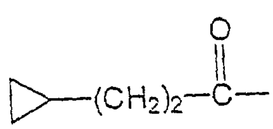

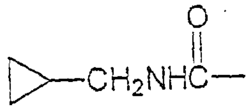

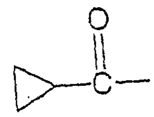

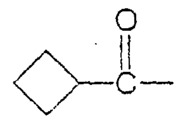

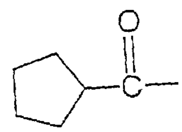

**13.** Dérivé triazolo-1,4-di-azépine de formule (II) et sel pharmacologiquement acceptable de celui-ci, de formule :

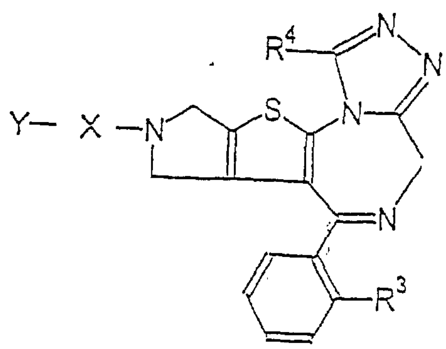

dans lequel $R^3$ représente un atome d'halogène, $R^4$ représente un groupe alkyle en $C_1$-$C_6$, X représente :

(a) un groupe de formule :

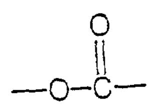

et Y représente

(2) un groupe ($C_3$-$C_7$) cycloalkyl-($C_1$-$C_6$) alkyle,

(3) un groupe alcynyle possédant jusqu'à 6 atomes de carbone,

(4) un groupe de formule :

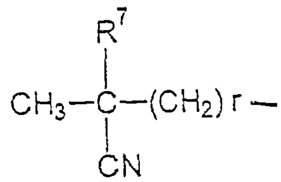

dans lequel $R^7$ est un atome d'hydrogène ou méthyle et r est 0, 1 ou 2,

(5) un groupe de formule $NC-(CH_2)_p-$ (dans lequel p est un entier de 1 à 6), ou

(6) un groupe de formule $A-(CH_2)_q-$ (dans lequel A représente un groupe choisi parmi un groupe pyridyle, un groupe pyranyle et un groupe morpholino et q est un entier de 0 à 6).

**14.** Dérivé triazolo-1,4-di-azépine selon la revendication 13 de formule :

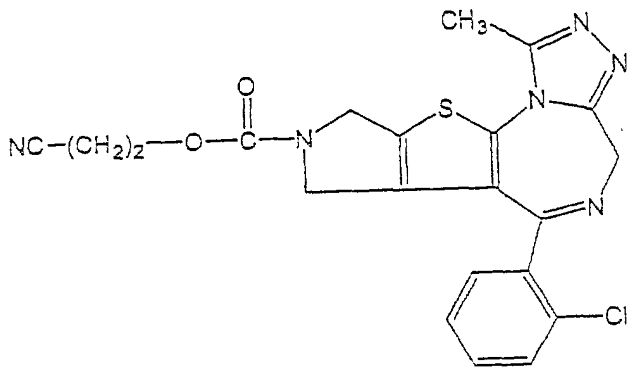

**15.** Dérivé triazolo-1,4-di-azépine selon la revendication 13 de formule :

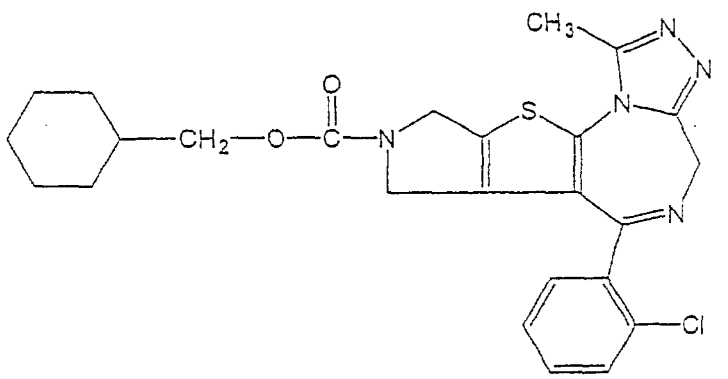

**16.** Dérivé triazolo-1,4-di-azépine selon la revendication 13 de formule :

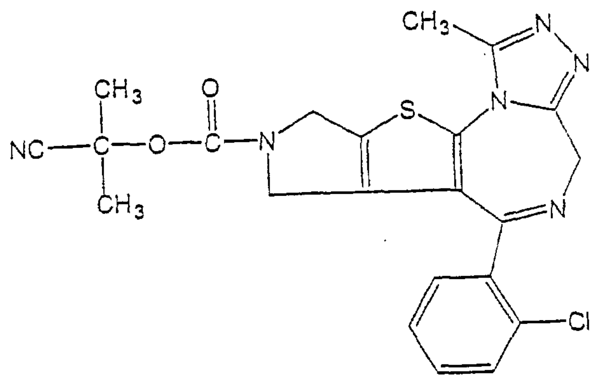

**17.** Dérivé triazolo-1,4-di-azépine de formule :

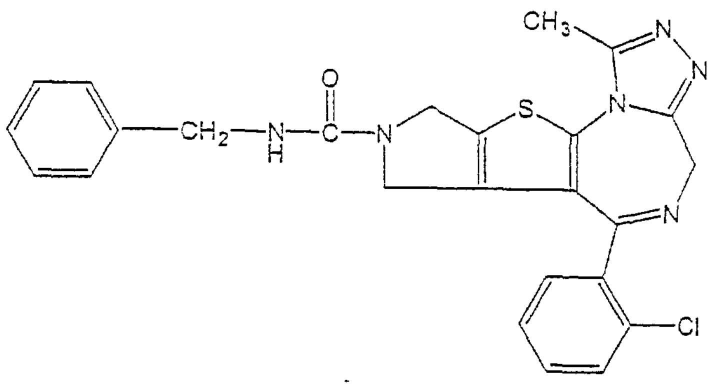

**18.** Dérivé triazolo-1,4-di-azépine de formule :

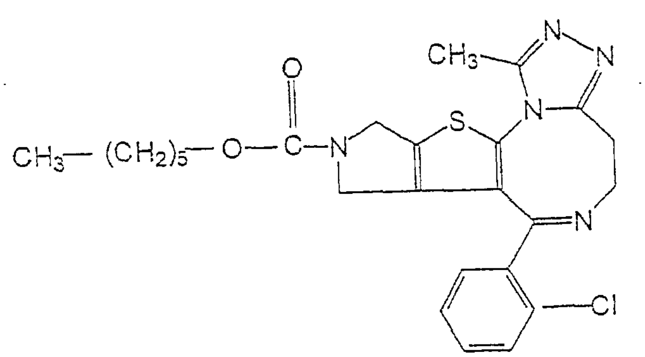

**19.** Dérivé triazolo-1,4-di-azépine de formule :

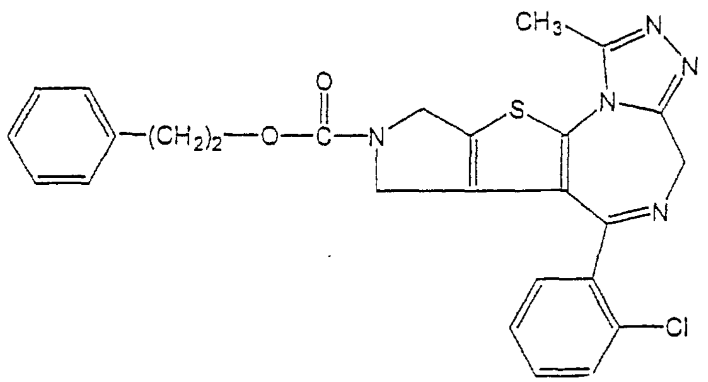

**20.** Dérivé triazolo-1,4-di-azépine de formule :

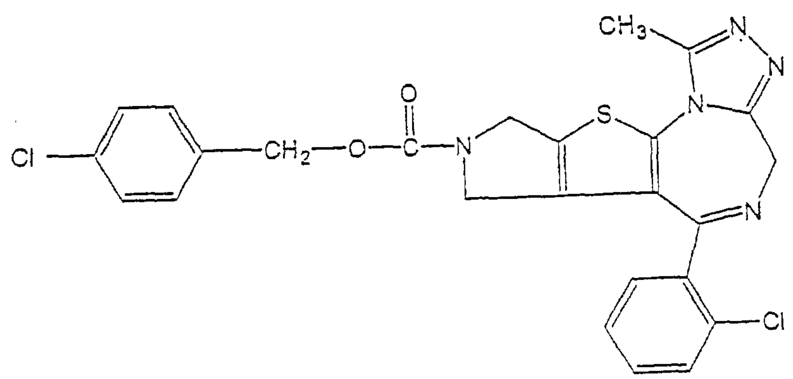

**21.** Dérivé triazolo-1,4-di-azépine de formule :

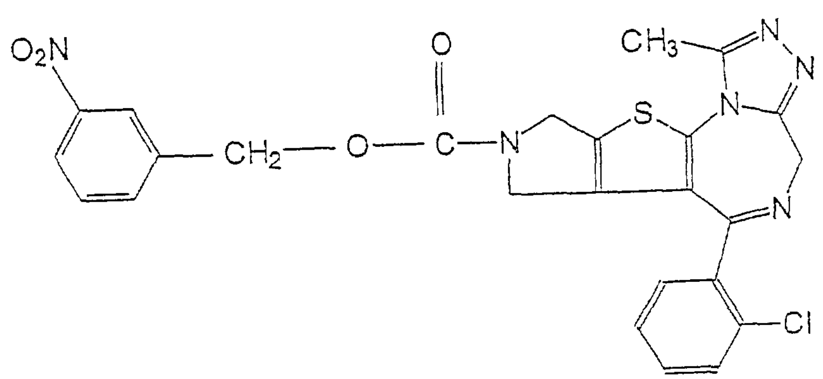

**22.** Dérivé triazolo-1,4-di-azépine de formule :

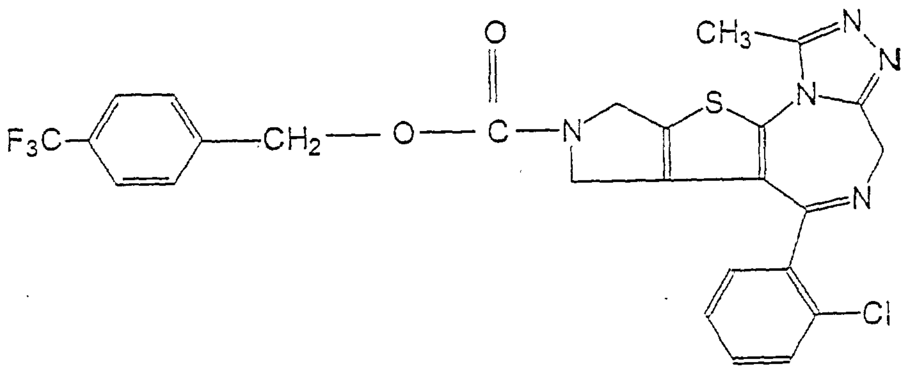

**23.** Composition pharmaceutique comprenant une quantité pharmacologiquement efficace d'un composé selon l'une quelconque des revendications 1 à 22 ou sel de celui-ci et un véhicule pharmacologiquement acceptable.

**24.** Utilisation d'un composé ou d'un sel de celui-ci tel que défini selon l'une quelconque des revendications précédentes 1 à 22 pour la fabrication d'un médicament pour le traitement d'une maladie pour laquelle une activité anti-PAF est utile.

**25.** Utilisation selon la revendication 24 dans laquelle la maladie est une maladie allergique.

**26.** Utilisation selon la revendication 24 dans laquelle la maladie est l'asthme.